# EUROPEAN PATENT SPECIFICATION

(11) **EP 1 059 279 B1**
(45) Date of publication and mention of the grant of the patent: **17.08.2005**
(21) Application number: 99106903.0
(22) Date of filing: 25.05.1993
(51) Int. Cl.: C07C 41/28, C07C 43/04, C07C 43/10, C07C 43/11, C07C 43/15, C08F 8/04, C08F 16/18, C08F 299/02, C10M 107/24, C07C 43/315

(54) **Polyvinyl ether compound and method of preperation**
Polyvinyletherverbindung und Herstellungsverfahren
Composé d'éther polyvinylique et procédé de préparation

(30) Priority: 04.06.1992 JP 14392292; 07.09.1992 JP 23784292
(43) Date of publication of application: 13.12.2000
(62) Divisional of application: 93910387.5
(73) Proprietor: IDEMITSU KOSAN CO., LTD., Tokyo 100-8321 (JP)
(72) Inventor: Egawa, Tatsuya c/o Idemitsu Kosan Co., Ltd., Sodegaura-shi, Chiba-ken (JP); Kawaguchi, Yasuhiro c/o Idemitsu Kosan Co., Ltd., Sodegaura-shi, Chiba-ken (JP); Mogami, Kenji, c/o Idemitsu Kosan Co.,Ltd., Sodegaura-shi, Chiba-ken (JP); Shimizu, Nobuaki, c/o Idemitsu Kosan Co.,Ltd., Sodegaura-shi, Chiba-ken (JP)
(74) Representative: Gille, Struck, Neidlein, Prop, Roos

(56) References cited:
- DE-B- 1 231 678
- GB-A- 1 500 020
- GB-A- 1 581 412
- US-A- 2 165 962
- US-A- 2 487 525
- US-A- 2 590 598
- US-A- 3 121 120
- US-A- 4 482 753
- W. L. HOWARD ET AL: "Hydrogenolysis of ketals" JOURNAL OF ORGANIC CHEMISTRY, vol. 26, pages 1026-1028, XP002108539 EASTON US

## Description

### BACKGROUND OF THE INVENTION

### 1. Field of the Invention

The present invention relates to a novel use of a polyvinylether compound as a lubricating oil for compression-type refrigerators, and a novel method of production of ether compounds. More particularly, the present invention relates to (1) a use of a polyvinylether compound as a lubricating oil for compression-type refrigerators, the polyvinyl ether compound having excellent compatibility with hydrogen-containing Flon compounds ["a Flon compound" means a chlorofluorocarbon (CFC), a hydrofluorocarbon (HFC) and a hydrochlorofluorocarbon (HCFC) in general], such as 1,1,1,2-tetrafluoroethane (referred to as Flon 134a, hereinafter) and the like, which can be used as the refrigerant to replace compounds causing environmental pollution, such as dichlorofluoroethane (referred to as Flon 12, hereinafter) and the like, and having an excellent lubricating property; (2) a method of efficiently and industrially advantageously producing a useful ether compound having a wide range of applications as solvent, lubricating oil and the like by hydrogenation of an acetal compound or a ketal compound.

### 2. Description of the Related Arts

Compression-type refrigerators are generally constituted of a compressor, a condenser, an expansion valve and an evaporator and has a structure that mixed fluid of a refrigerant and a lubricating oil is circulated in the closed system. Depending on the type of machinery, in the compression-type refrigerator generally the temperature is high in the compressor and low in the refrigerating chamber and it is generally required that the refrigerant and the lubricating oil be circulated in the system without causing phase separation in the wide range of temperature. When the phase separation occurs during the operation of the refrigerator, the life and the efficiency of the apparatus are adversely affected to a great extent. For example, when the phase separation of the refrigerant and the lubricating oil occurs in the part of the compressor, the lubrication of the moving parts is deteriorated and the seizure occurs to cause decrease in the life of the apparatus to a great extent. When the phase separation occurs in the evaporator, the efficiency of heat exchange is decreased because of the presence of the lubricating oil of high viscosity.

Because the lubricating oil for refrigerators is used for the purpose of lubricating moving parts in refrigerators, the lubricating property is naturally important. Particularly, because the temperature in the compressor is high, the viscosity which can hold the oil film necessary for the lubrication is important. The required viscosity is different depending on the kind of the compressor used and the working conditions and it is generally preferable that the viscosity (kinematic viscosity) of the lubricating oil before mixing with the refrigerant be 5 to 1000 cSt at 40°C. When the viscosity is lower than this range, the oil film becomes thin to cause insufficient lubrication, and, when the viscosity is higher than this range, the efficiency of the heat exchange is decreased.

Electric refrigerators have the motor and the compressor built into a single body and the lubricating oil for them is required to have a high degree of electric insulating property. In general, a volume intrinsic resistance of 10¹² Ω·cm or more at 80°C is required. When the resistance is lower than this value, the possibility of the leak of electricity arises.

As the refrigerant for compressor-type refrigerators, mainly Flon 12 has heretofore been used and, as the lubricating oil, various kinds of mineral oils and synthetic oils satisfying the required properties described above have been used. However, chlorofluorocarbons (CFC) including Flon 12 are being more rigorously restricted world-wide because they bring environmental pollution such as the destruction of the ozone layer. By this reason, hydrogen-containing Flon compounds such as hydrofluorocarbons (HFC) and hydrochlorofluorocarbons (HCFC) are attracting attention as the novel kinds of the refrigerant. The hydrogen-containing fluorocarbons, particularly hydrofluorocarbons (HFC), typified by Flon 134a, are preferred as the refrigerant for compression-type refrigerators because they have little possibility of causing the destruction of the ozone layer and can replace Flon 12 with little change in the structure of refrigerators which have heretofore been used.

When a hydrogen-containing Flon compound described above, such as Flon 134a, Flon 32, Flon 125, Flon 22 and the like, is adopted as the refrigerant for compression-type refrigerators to replace Flon 12, a lubricating oil having good compatibility with the hydrogen-containing Flon compound, such as Flon 134a, Flon 32, Flon 125, Flon 22 and the like, and good lubricating property satisfying the requirements described above is naturally required. However, because the lubricating oils which have heretofore been used in combination with Flon 12 do not have good compatibility with the hydrogen-containing Flon, such as Flon 134a, Flon 32, Flon 125 and the like, a new lubricating oil suited for these compounds is required. When a new lubricating oil is adopted, it is desired that major change in the structure of the apparatus be not necessary. It is not desirable that the structure of the currently used apparatus must have major changes because of the new lubricating oil.

As the lubricating oil having compatibility with Flon 134a, for example, polyoxyalkylene glycols have been known. Such lubricating oils are disclosed, for example, in Research Disclosure No. 17463 (October, 1978), the specification of the United States Patent No. 4755316, Japanese Patent Application Laid-Open No. 1989-256594, Japanese Patent Application Laid-Open No. 1989-259093, Japanese Patent Application Laid-Open No. 1989-259094, Japanese Patent Application Laid-Open No. 1989-271491, Japanese Patent Application Laid-Open No. 1990-43290, Japanese Patent Application Laid-Open No. 1990-84491, Japanese Patent Applications Laid-Open No. 1990-132176 to 132178, Japanese Patent Application Laid-Open No. 1990-132179, Japanese Patent Application Laid-Open No. 1990-173195, Japanese Patent Applications Laid-Open No. 1990-180986 to 180987, Japanese Patent Applications Laid-Open No. 1990-182780 to 182781, Japanese Patent Application Laid-Open No. 1990-242888, Japanese Patent Application Laid-Open No. 1990-258895, Japanese Patent Application Laid-Open No. 1990-269195, Japanese Patent Application Laid-Open No. 1990-272097, Japanese Patent Application Laid-Open No. 1990-305893, Japanese Patent Application Laid-Open No. 1991-28296, Japanese Patent Application Laid-Open No. 1991-33193, Japanese Patent Applications Laid-Open No. 1991-103496 to 103497, Japanese Patent Application Laid-Open No. 1991-50297, Japanese Patent Application Laid-Open No. 1991-52995, Japanese Patent Applications Laid-Open No. 1991-70794 to 70795, Japanese Patent Application Laid-Open No. 1991-79696, Japanese Patent Application Laid-Open No. 1991=106992, Japanese Patent Application Laid-Open No. 1991-109492, Japanese Patent Application Laid-Open No. 1991-121195, Japanese Patent Application Laid-Open No. 1991-205492, Japanese Patent Application Laid-Open No. 1991-231992, Japanese Patent Application Laid-Open No. 1991-231994, Japanese Patent Application Laid-Open No. 1992-15295, Japanese Patent Application Laid-Open No. 1992-39394 and Japanese Patent Applications Laid-Open No. 1992-41591 to 41592. However, the lubricating oils from the polyoxyalkylene glycols generally have low volume intrinsic resistances and no example satisfying the value of 10¹² Ω·cm or more at 80°C has been disclosed yet.

As the compound having compatibility with Flon 134a in addition to the lubricating oils from polyoxyalkylene glycols, lubricating oils of esters are disclosed in British Patent Laid-Open No. 2216541, WO No. 6979 (1990), Japanese Patent Applications Laid-Open No. 1990-276894, Japanese Patent Applications Laid-Open No. 1991-128992, Japanese Patent Applications Laid-Open No. 1991-88892, Japanese Patent Applications Laid-Open No. 1991-179091, Japanese Patent Applications Laid-Open No. 1991-252497, Japanese Patent Applications Laid-Open No. 1991-275799, Japanese Patent Applications Laid-Open No. 1992-4294, Japanese Patent Applications Laid-Open No. 1992-20597 and the specification of the United States Patent No. 5021179. However, the lubricating oils from the esters do not have sufficient compatibility because the phase separation occurs when viscosity of the lubricating oils is high at lower temperatures even though they show good compatibility at higher temperatures.

Thus, it is the real situation at present that a lubricating oil for the compression-type refrigerators having excellent compatibility with Flon 134a, excellent stability and lubricating property and a volume intrinsic resistance at 80°C of 10¹² Ω·cm or more has not been discovered yet. The development of such a lubricant is strongly desired.

Polyvinyl ether compounds are important compounds having a wide range of applications, such as solvents, lubricating oils, adhesives, resins and the like and, as the methods of production of polyvinyl ether compounds, the methods of polymerizing vinyl ether monomers with a radical, a cation or an irradiation are known ("Gosei Kobunshi III", edited by Shunsuke Murahashi, Minoru Imoto and Hisashi Tani, published by Asakura Shoten). However, these conventional methods are not always satisfactory because of the problems that a compound having the desired degree of polymerization is not easily obtained, that the process is complicated, that control of the reaction is difficult, that a large amount of solvent is required and the like problems. Thus, development of the method of production of polyvinyl ether compound free from the aforesaid problems is strongly desired.

As a method of producing an ether compound having a wide range of applications as solvents and lubricants from acetal compounds or ketal compounds, for example, a method of using a combination of an acid and an alkali metal hydride, a method of using a silicon reagent and a method of using diborane or the like are known ("Jikken Kagaku Koza", Volume 20, the 4th edition, published by Maruzen).

However, these reactions use stoichiometric amounts of very expensive materials such as the alkali metal hydride, diborane and the silicon reagent as the hydrogenating reagent and are not preferable as the method of industrial production.

A method of a combination of an acid catalyst and catalytic hydrogenation is known. W. L. Howard [J. Org. Chem. Volume 26, Page 1026 (1961)] reported the formation of an ether by catalytic hydrocracking of a ketal by using a catalyst in which rhodium is supported on alumina in the presence of hydrochloric acid. In the specification of the United States Patent No. 4088700, a method of production of an ether compound by catalytic hydrocracking of 1,3-dioxoranes which are cyclic acetals by using a platinum catalyst or a rhodium catalyst in the presence of a Lewis acid such as boron trifluoride, aluminum trichloride and the like is shown. However, because these methods of production use hydrochloric acid, boron trifluoride, aluminum trichloride or the like, corrosion of the apparatus becomes the problem when the ordinary apparatus is used and a special treatment is necessary, making the methods unfavorable.

As a method without using acids, for example, a method of producing an ether compound by hydrocracking of acetals by using a palladium catalyst supported on carbon is proposed in Japanese Patent Application Laid-Open No. 1983-4739 and Japanese Patent Application Laid-Open No. 1983-177929. Though the method is free from the corrosion problem because an acid is not used, conversion of the raw material acetal is not satisfactory.

Thus, a method of production of the ether compound from an acetal compound or a ketal compound which has sufficient reaction activity, shows good selectivity and does not cause corrosion of the apparatus is not discovered yet and the development of such a method is strongly desired.

On the other hand, for the applications as lubricating oils, electric insulating oils and solvents, fluidity is necessary and a polymer having a lower degree of polymerization is desired. Concerning generally known polyalkyl vinyl ethers, examples of synthesis of various kinds of alkyl polyvinyl ethers are described in "Jikken Kagaku Koza", Volume 18, "Reaction of organic compounds II(A)", edited by Chemical Society of Japan (published by Maruzen). In the cases of alkyl vinyl ethers having a low molecular weight and an alkyl group of 3 or less carbon atoms among these examples, examples of the lowest molecular weight of a polymer are 2545 for the polymer of methyl vinyl ether, 4000 for the polymer of ethyl vinyl ether, 4830 for the polymer of n-propyl vinyl ether and 4580 for the polymer of isopropyl vinyl ether. A polymer of methyl vinyl ether having a molecular weight of 3000 is described in Macromolecules, Volume 17, Page 2228 (1984) and a polymer of ethyl vinyl ether having a molecular weight of 2600 is described in Macromolecules, Volume 18, Page 2 (1985). However, the values of molecular weight suggest that these polymers have very low fluidity and are in the form of semi-solid at room temperature.

In the cases of the compounds having an alkyl group of 4 or more carbon atoms, an example in which a dimer is isolated and an example in which a polymer having a molecular weight of 600 is obtained are found. However, polymers of alkyl vinyl ethers having an alkyl group of 4 or more carbon atoms do not satisfy the required property because they are not compatible with the hydrofluorocarbons such as Flon 134a.

As described in the above, a polymer of alkyl vinyl ether having an alkyl group of 3 or less carbon atoms which has a molecular weight of 1200 or less has not been known heretofore.

Ends of these polymers are olefins in the case of the acid catalysts and acetals when an alcohol is present and, when water is present, ends of acetal and ends of aldehyde are also formed. The end of olefin occasionally causes coloring and increase in viscosity in the presence of an acid and the end of aldehyde also occasionally causes coloring. Acetals are decomposed to olefins and alcohols in the presence of an acid. The olefins react with each other to cause coloring and increase in viscosity and, when water is present additionally, aldehydes are formed, also occasionally causing coloring. However, a polyvinyl ether compound free from the structures of acetals, aldehydes and olefins causing degradation at the ends of the molecule and has an excellent stability has not been reported yet.

### SUMMARY OF THE INVENTION

The present invention was undertaken under the situation described above with the objects of providing (1) a lubricating oil for compression-type refrigerators having excellent compatibility with hydrogen-containing Flon compounds, such as Flon 134a, Flon 32, Flon 125 and the like, which can replace the refrigerant of Flon 12 or other Flon compounds that are hardly decomposed and, particularly, cause the problem on environmental pollution, in the whole range of the working temperatures, has excellent stability and lubricating property and has a volume intrinsic resistance at 80°C of 10¹² Ω·cm or more; and (2) a method of efficiently producing an ether compound by hydrogenation of an acetal compound or a ketal compound by using a catalyst having a sufficient reaction activity and excellent selectivity and causing no corrosion of the apparatus.

As the result of the intensive studies by the present inventors for achieving the objects described above, it was discovered (1) that the first object described above can be achieved by a use of a polyvinyl ether compound having a specific structure as a lubricating oil and (2) that the second object described above can be achieved by using a solid catalyst having acidic property and hydrogenating ability as the catalyst. The present invention was completed on the basis of the aforesaid discovery.

The method of production of an ether compound expressed by the general formula (XV): 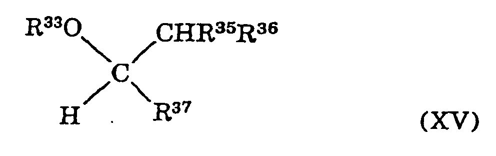 or by the general formula (XVI): 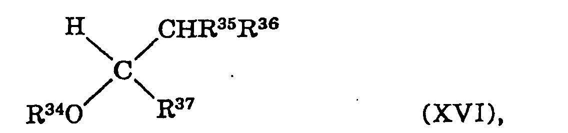 wherein R³³ and R³⁴ are each a hydrocarbon group or a hydrocarbon group containing ether oxygens in the main chain, in the side chain or in the both of them, and may be the same or different from each other and R³⁵, R³⁶ and R³⁷ are each a hydrogen atom, a hydrocarbon group or a hydrocarbon group containing ethereal oxygens in the main chain, in the side chain or in the both of them, and may be the same or different from each other, which is provided according to the second object of the invention comprises bringing an acetal compound or a ketal compound expressed by the general formula (XIV): 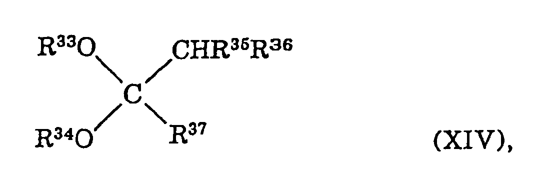 wherein R³³, R³⁴, R³⁵, R³⁵ and R³⁷ are as previously defined, into the reaction with hydrogen in the presence of a solid catalyst having acidic property and hydrogenating ability, wherein the catalysts are defined as in claim 5.

The polyvinyl ether compound which is used according to the first object of the invention comprises a homopolymer or a copolymer of an alkyl vinyl ether comprising the constituting units expressed by the general formula (I) or (I'): 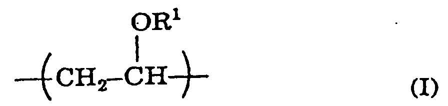 or 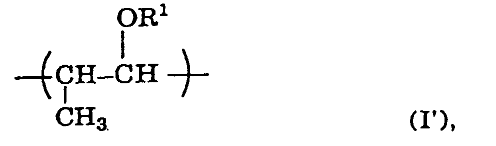 wherein R¹ is an alkyl group having 1 to 3 carbon atoms, and having a weight-average molecular weight of 300 to 1200 and one end having the structure expressed by the general formula (II): 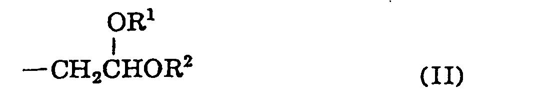 or the general formula (III):

-CH=CHOR^{a} (III)

wherein R¹ is as previously defined, R² is a hydrocarbon group having 1 to 8 carbon atoms and R^{a} is either one of R¹ and R²;

### BRIEF DESCRIPTION OF THE DRAWINGS

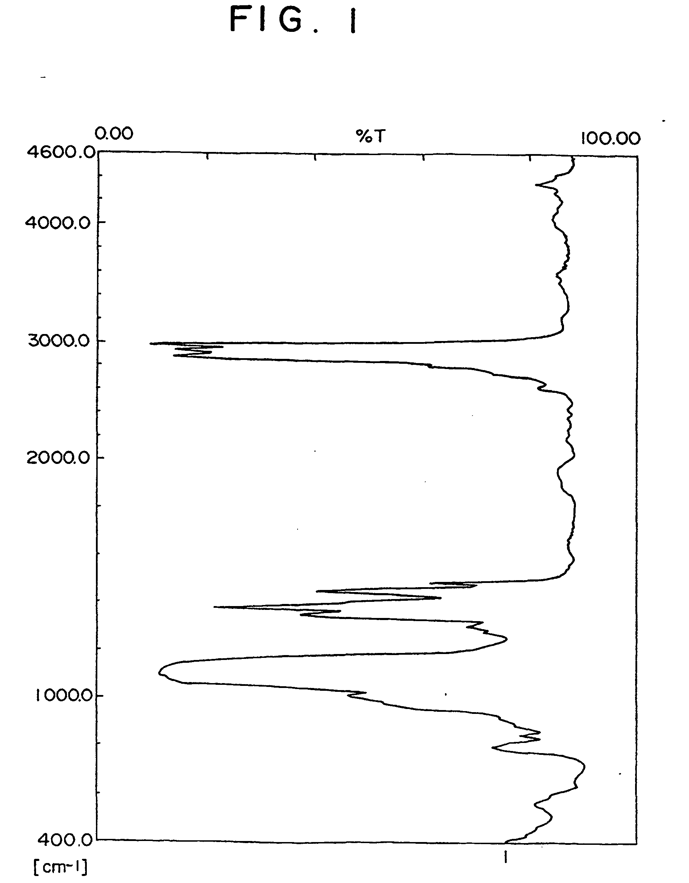

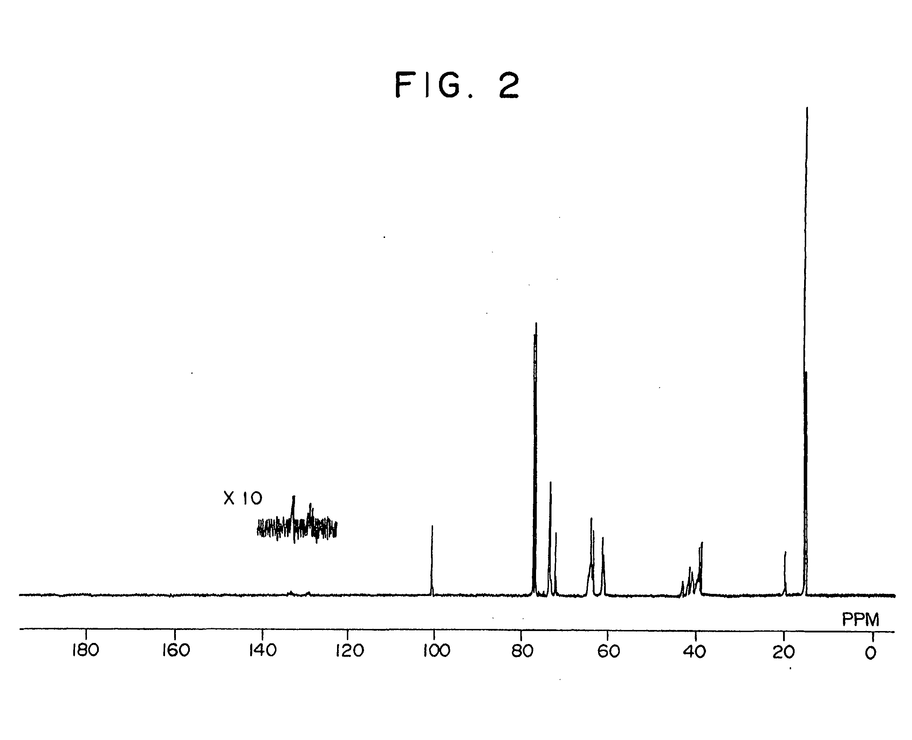

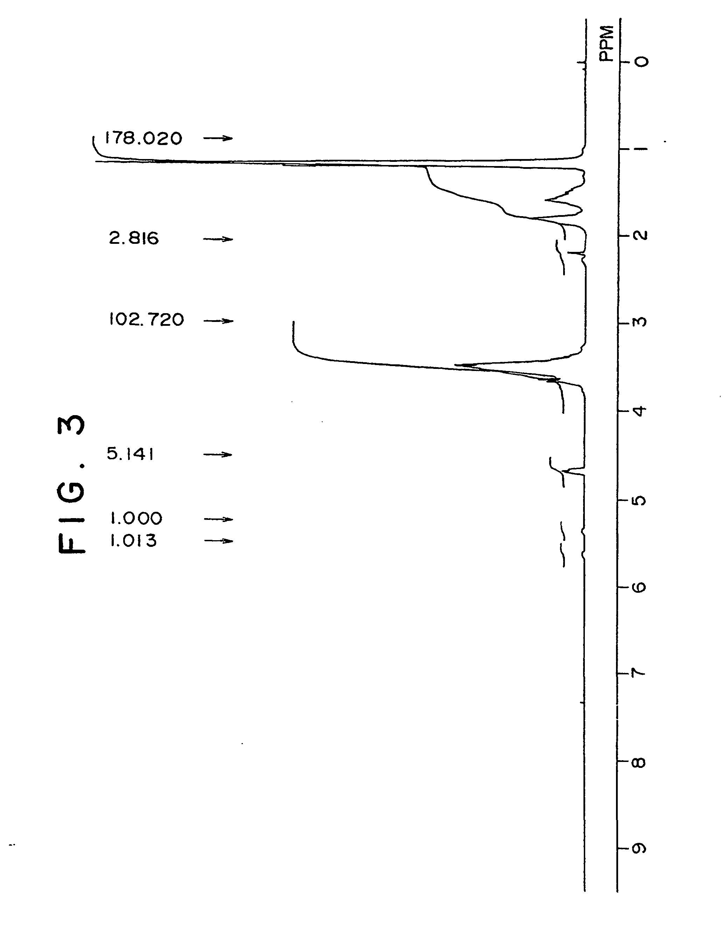

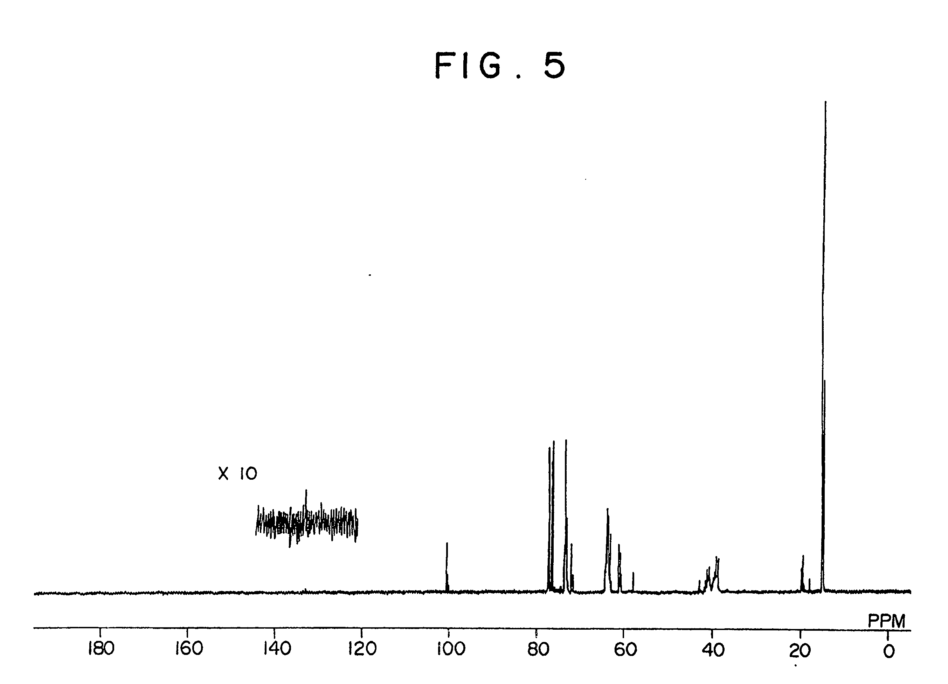

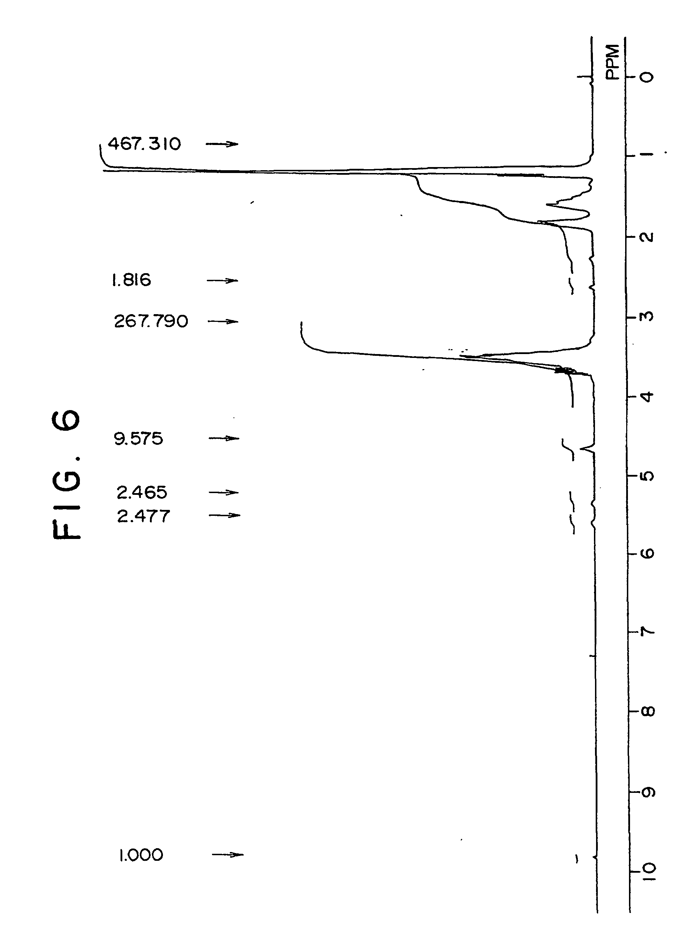

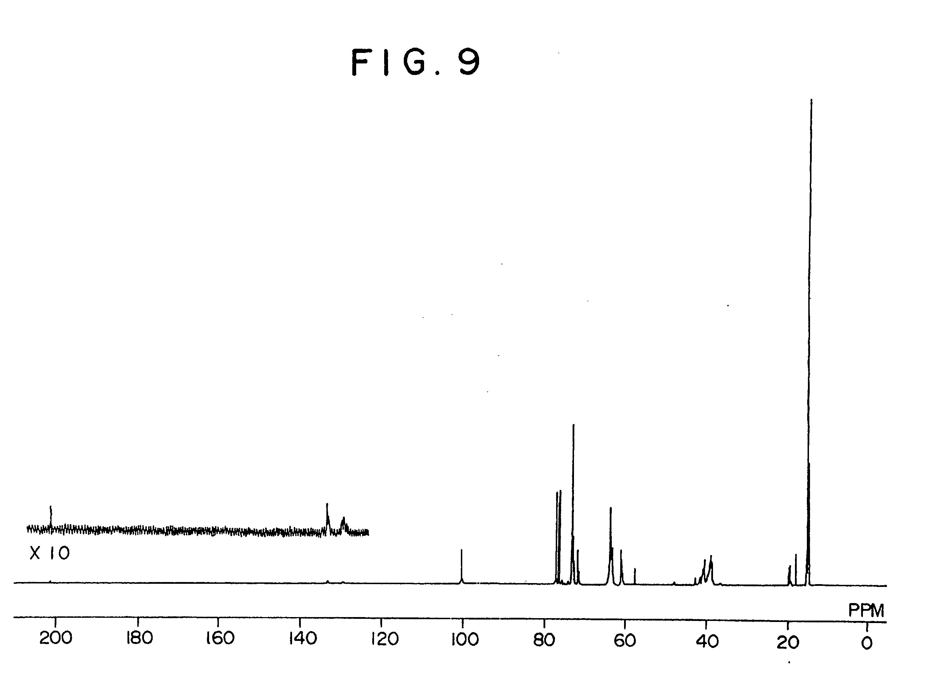

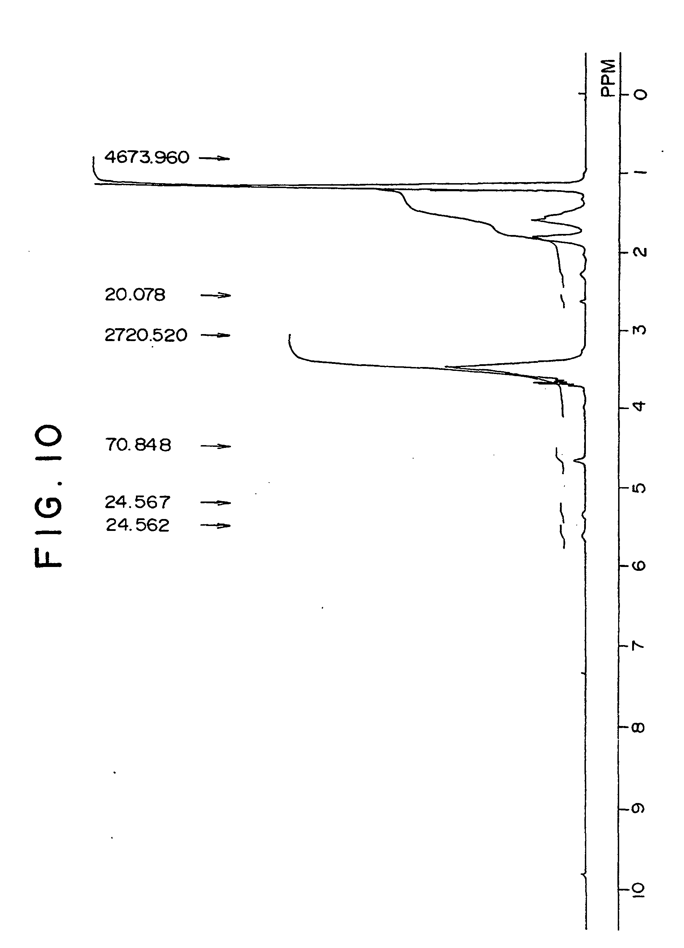

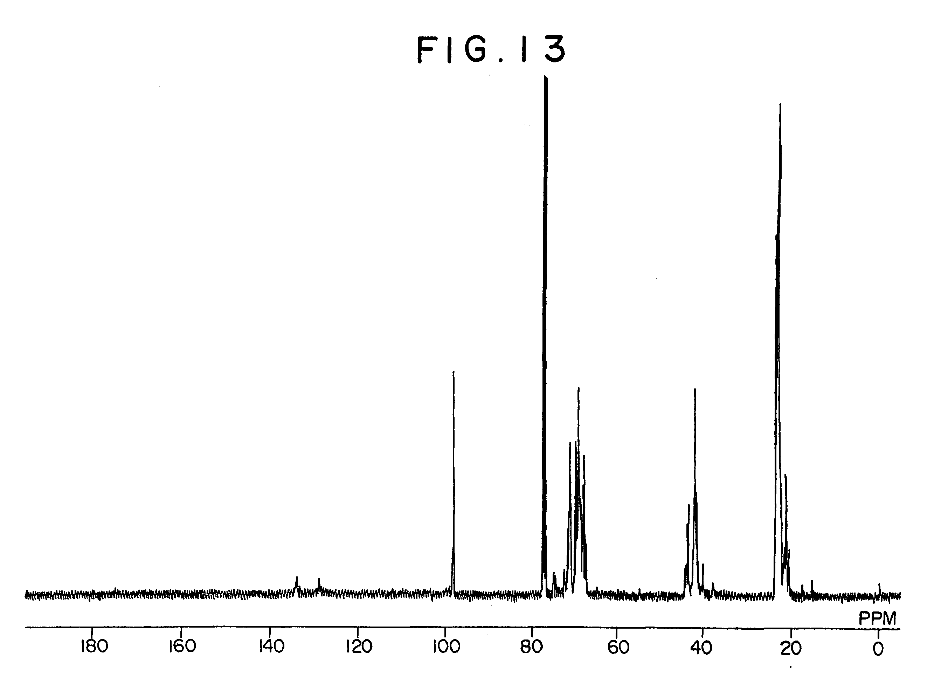

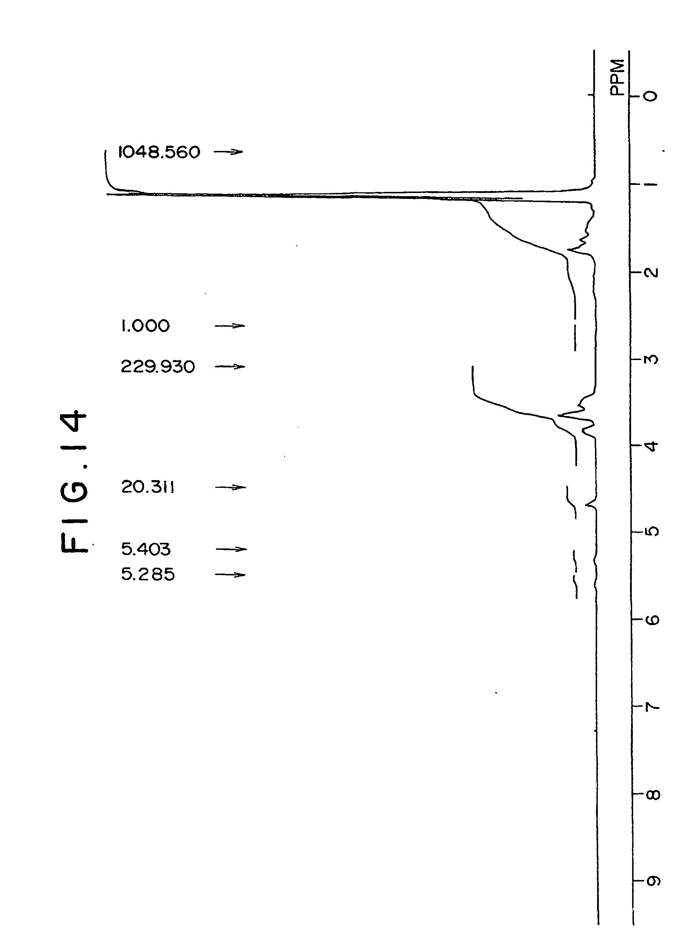

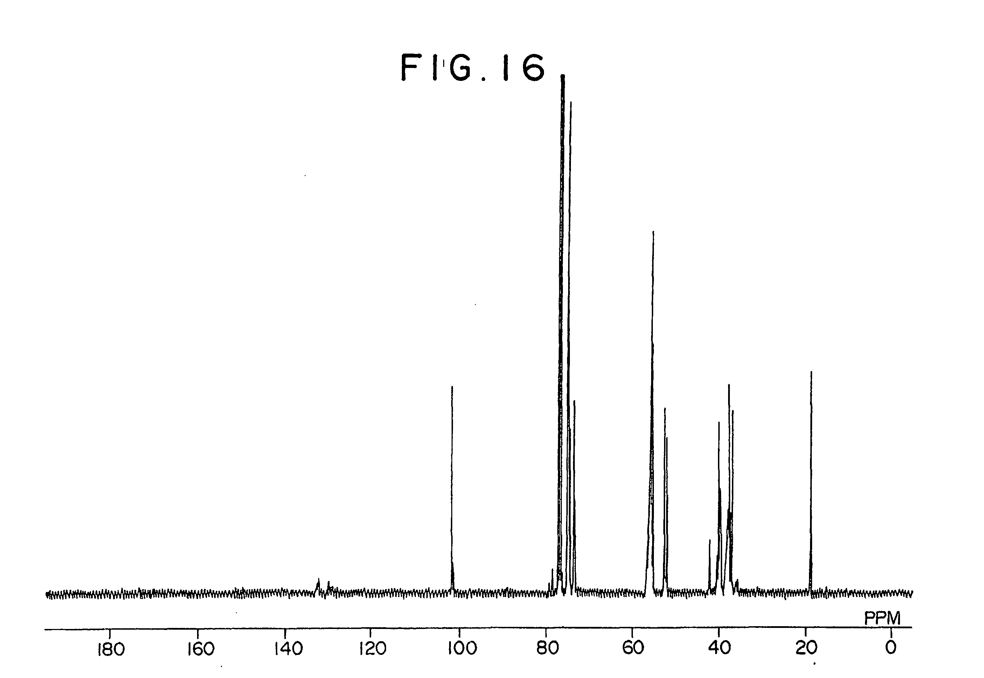

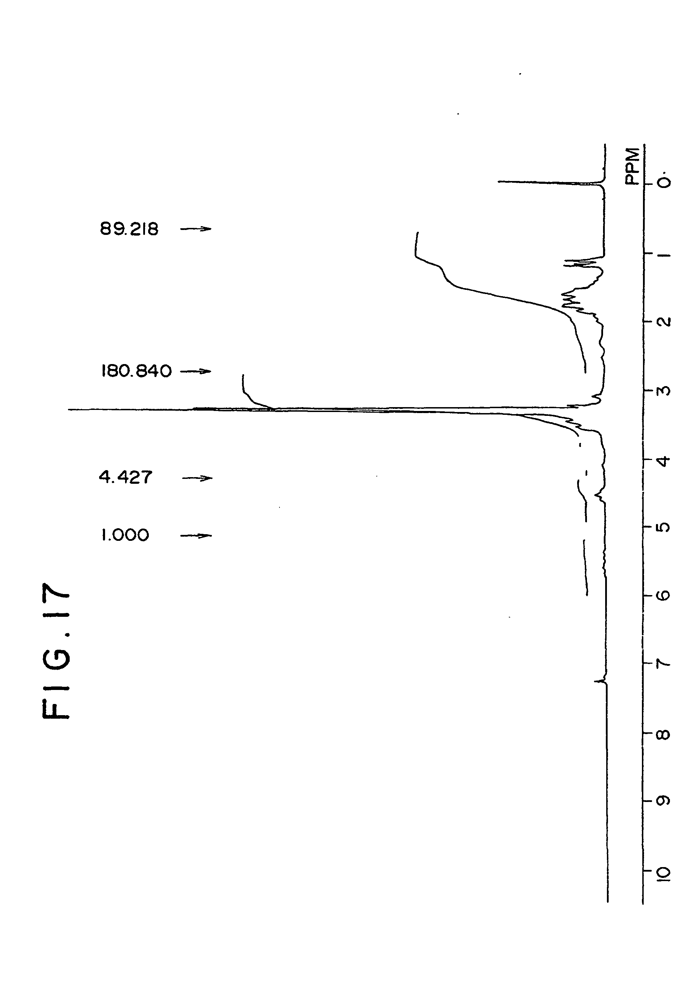

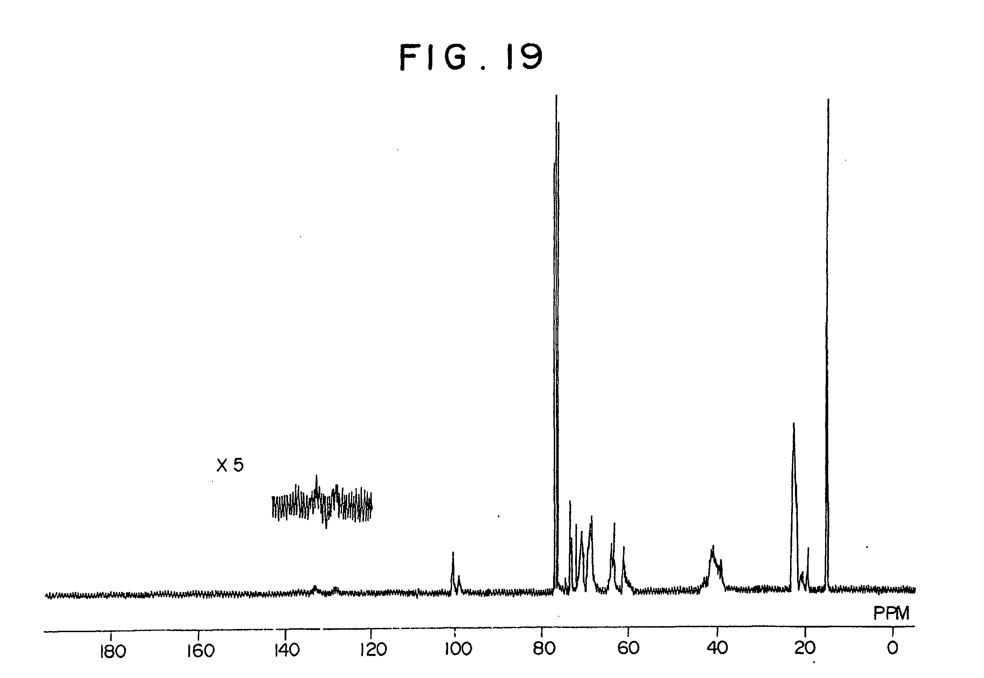

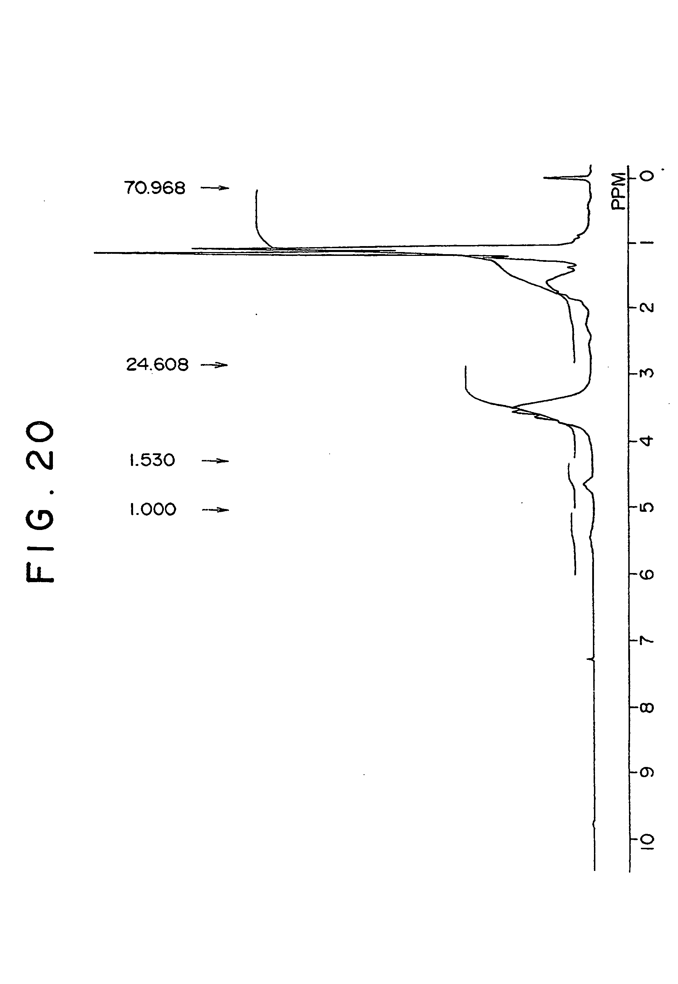

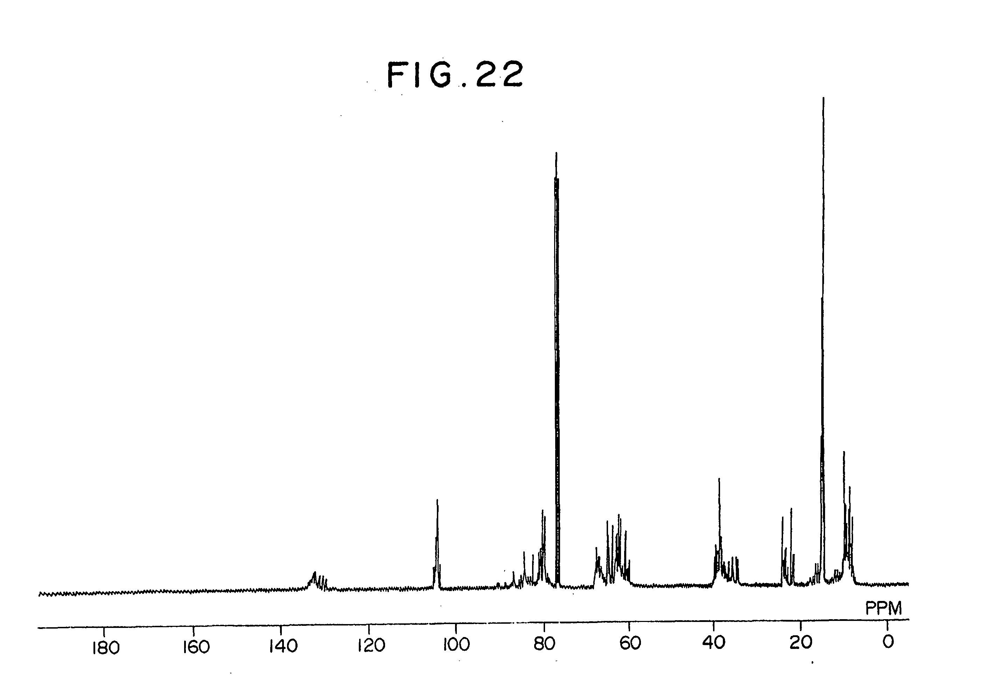

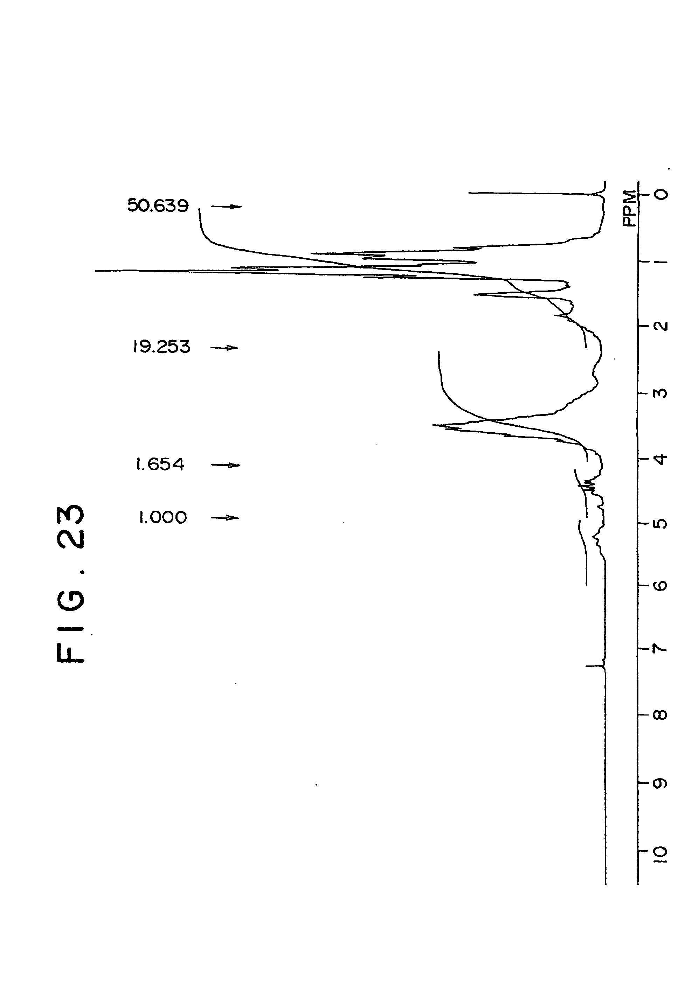

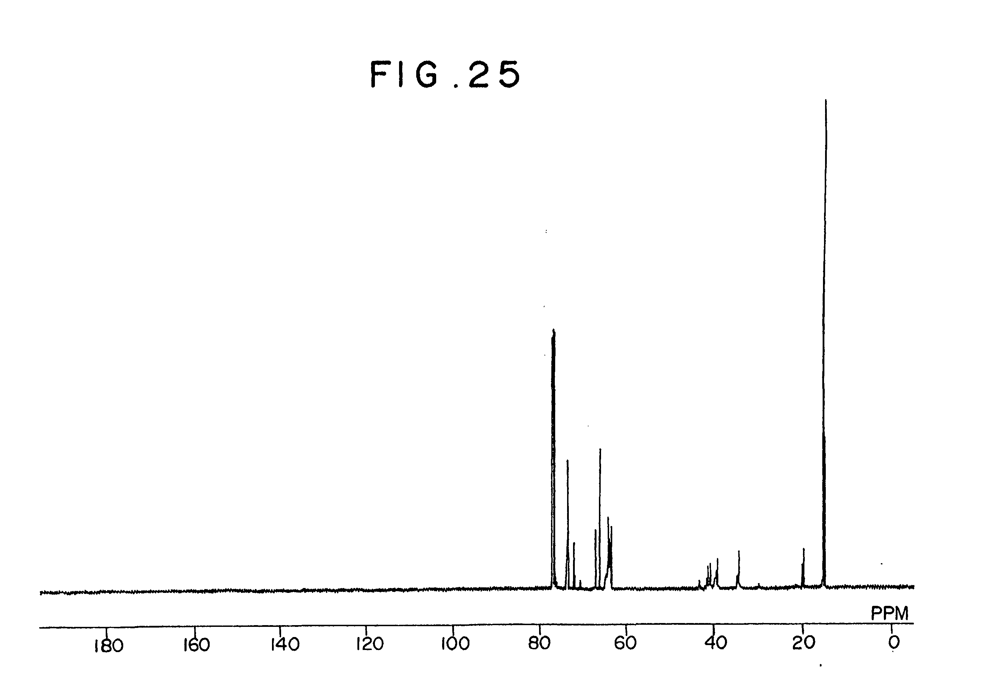

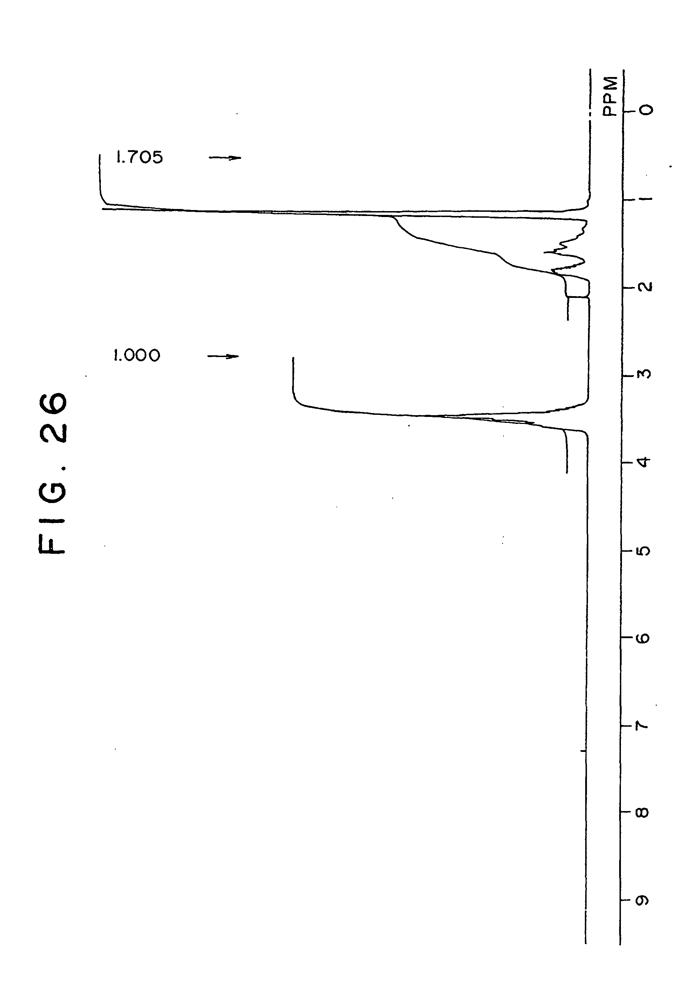

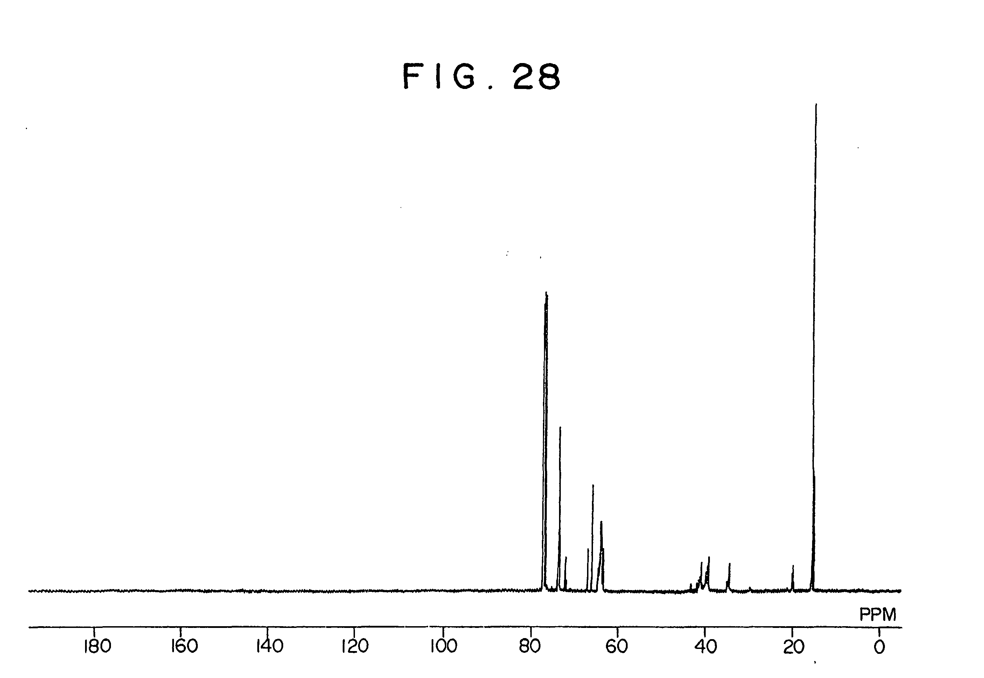

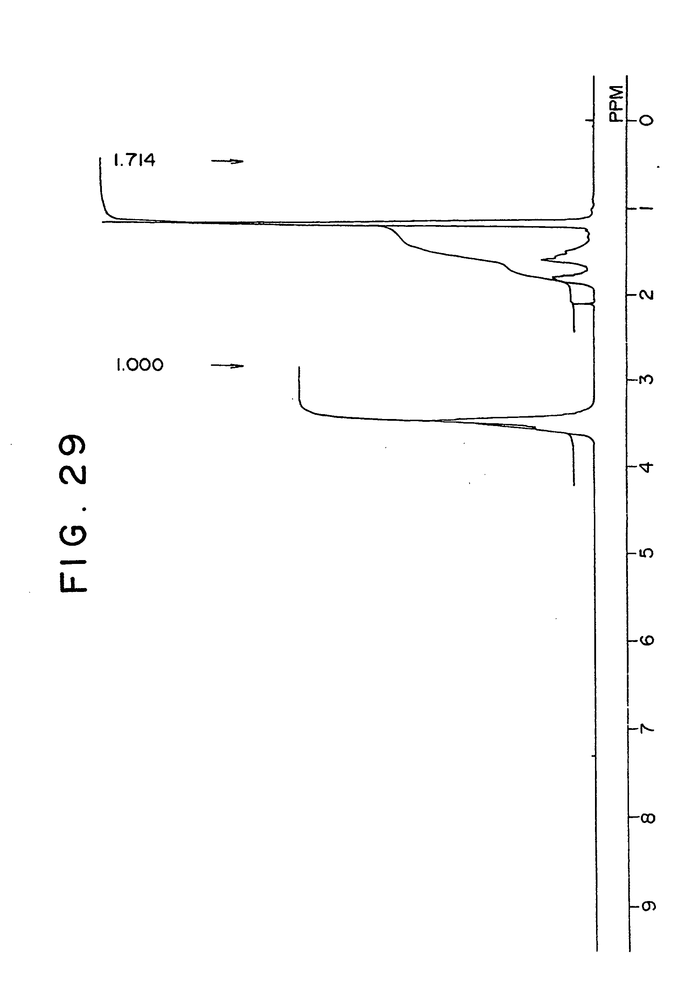

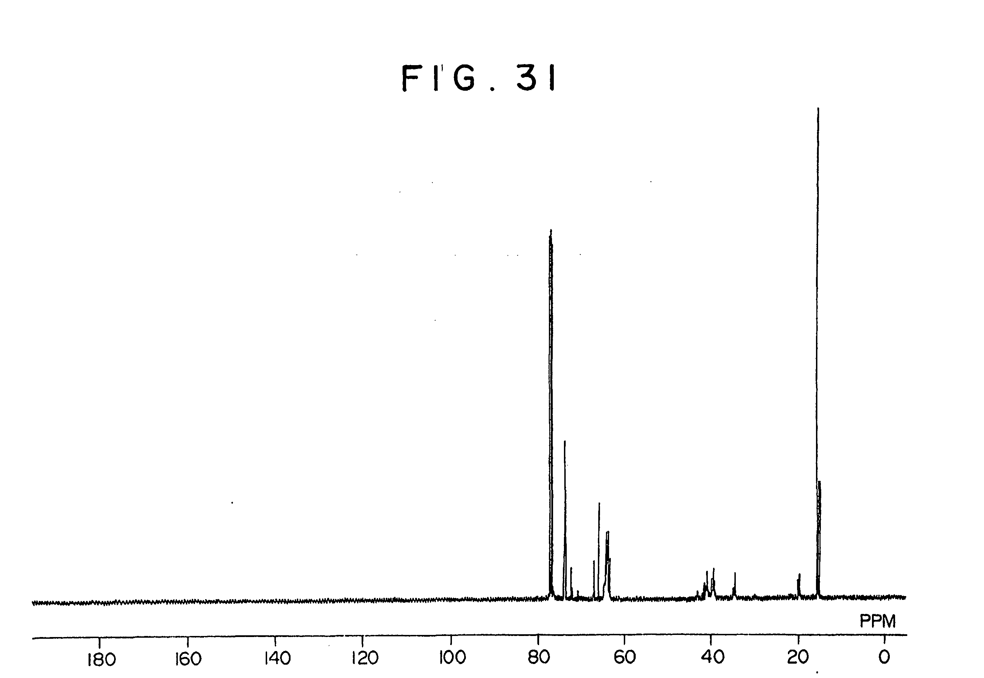

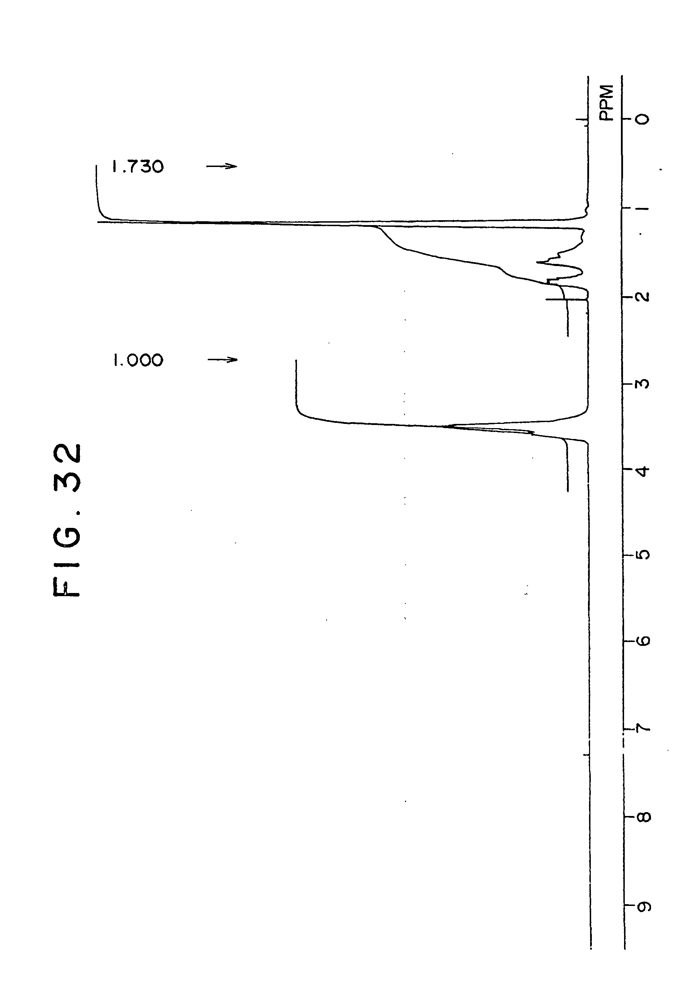

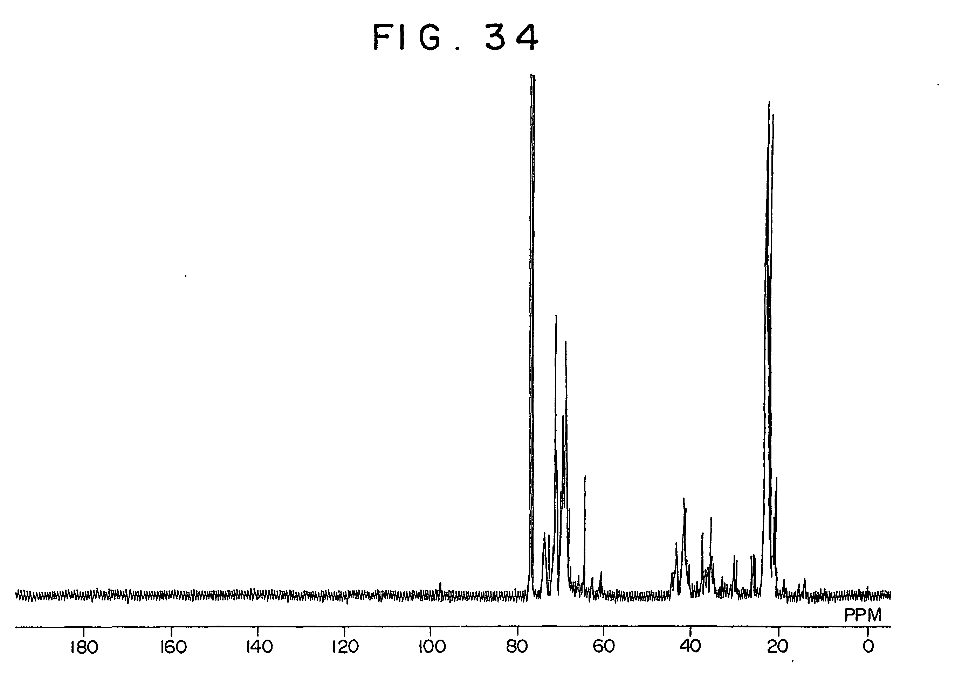

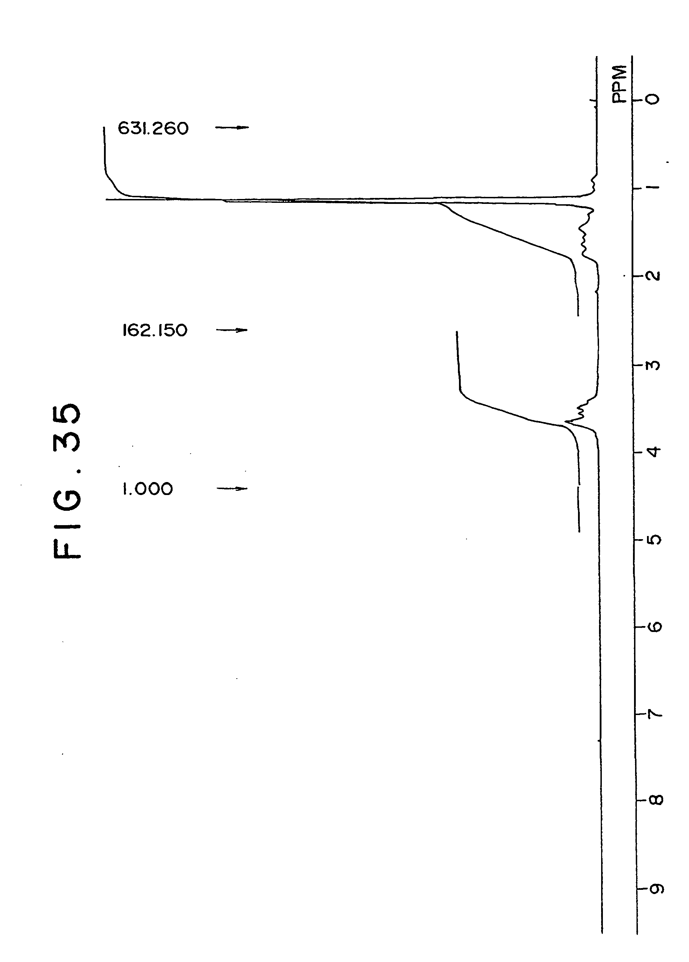

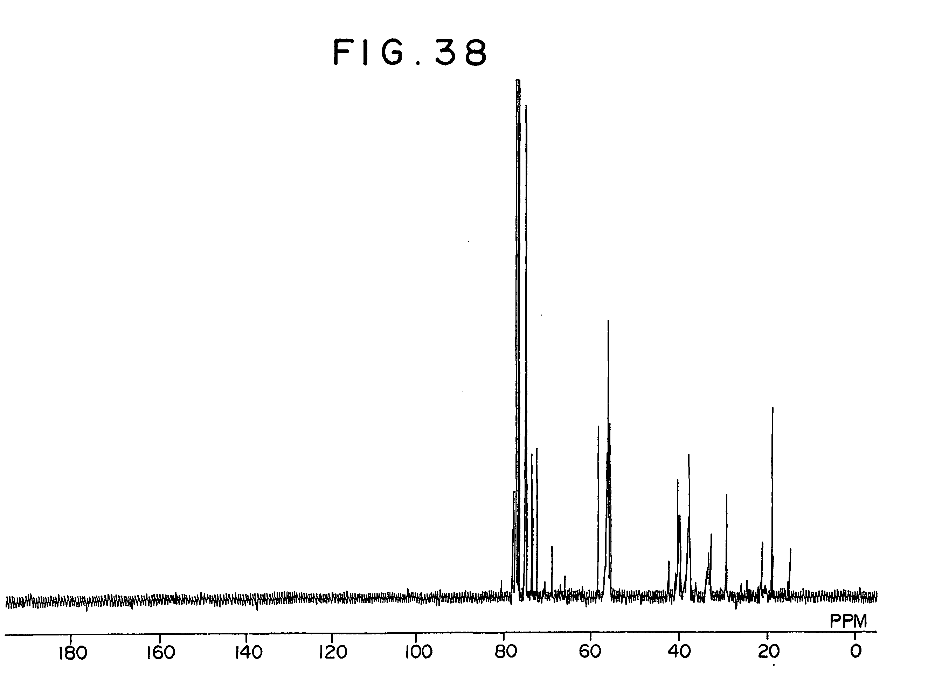

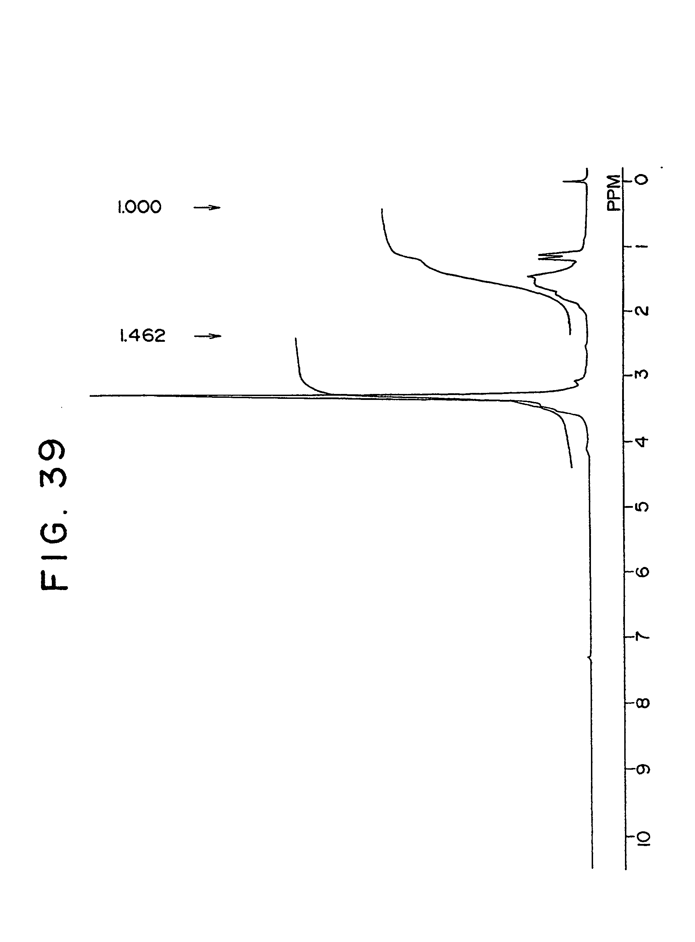

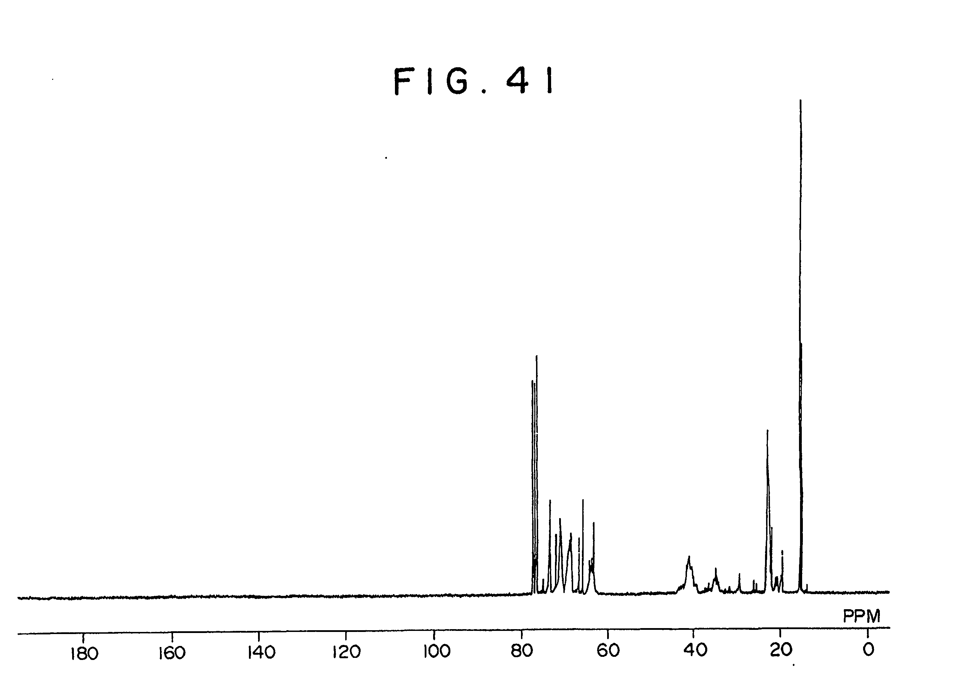

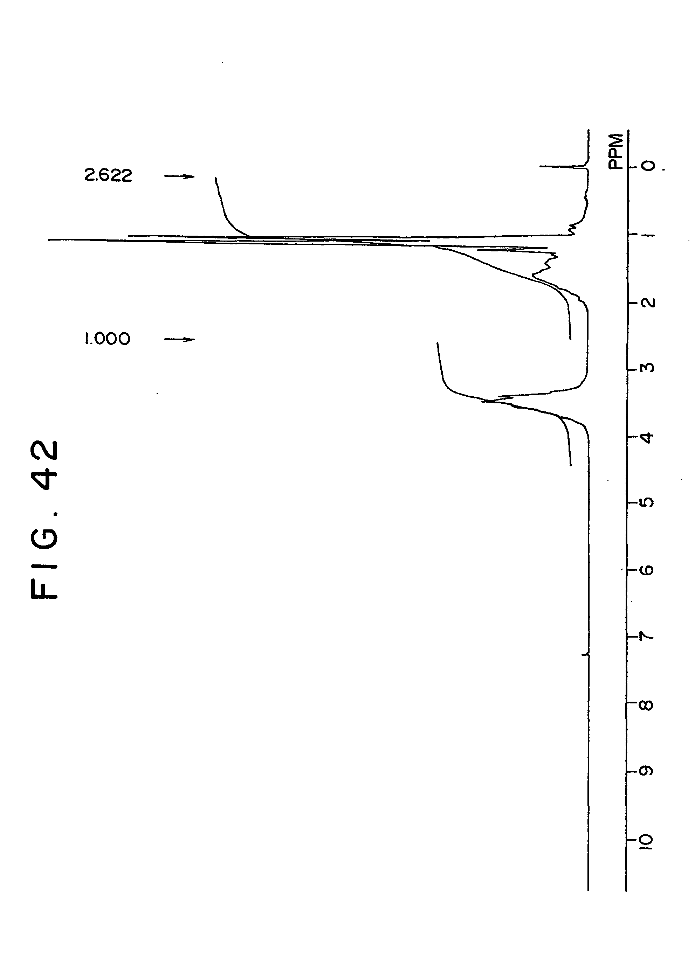

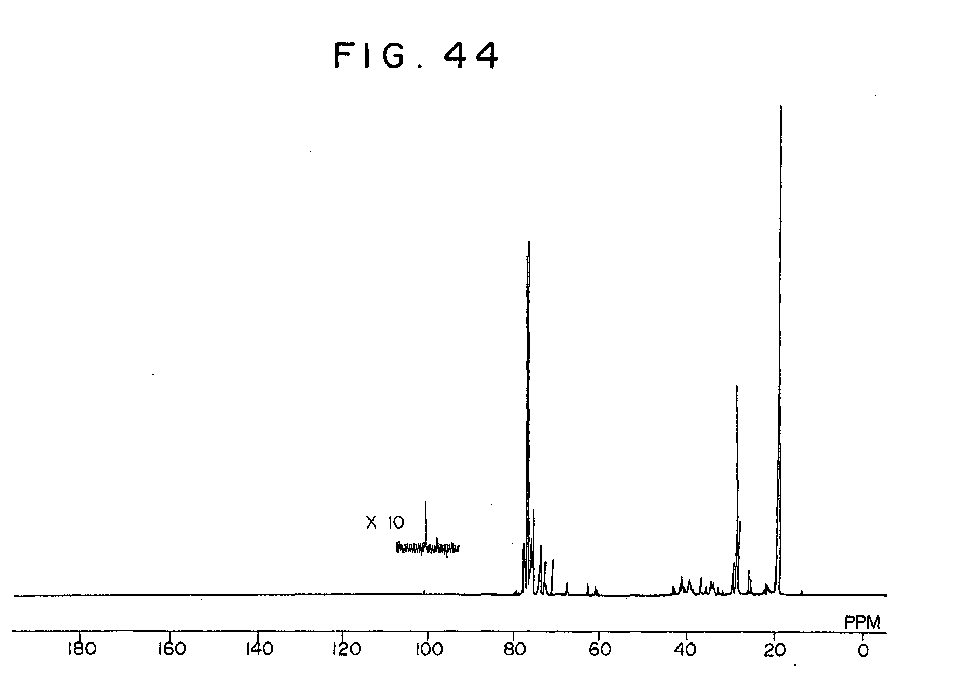

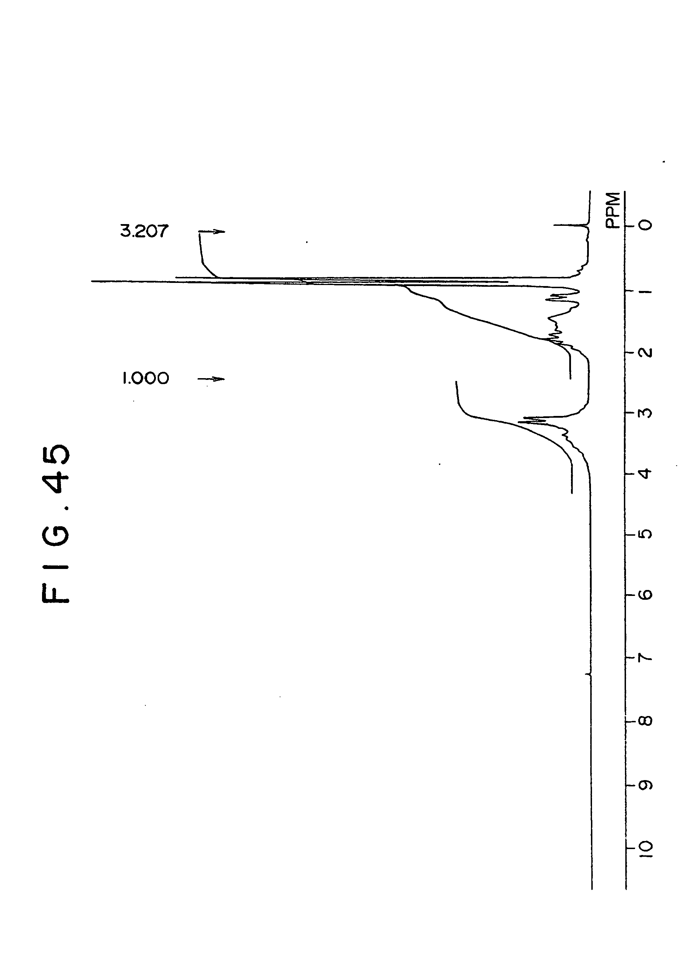

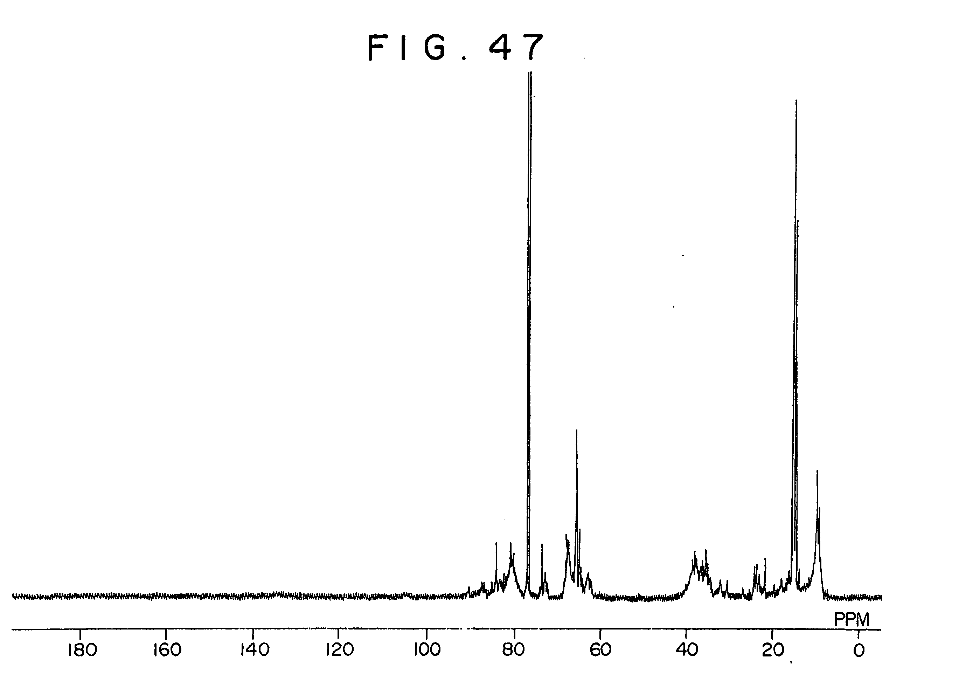

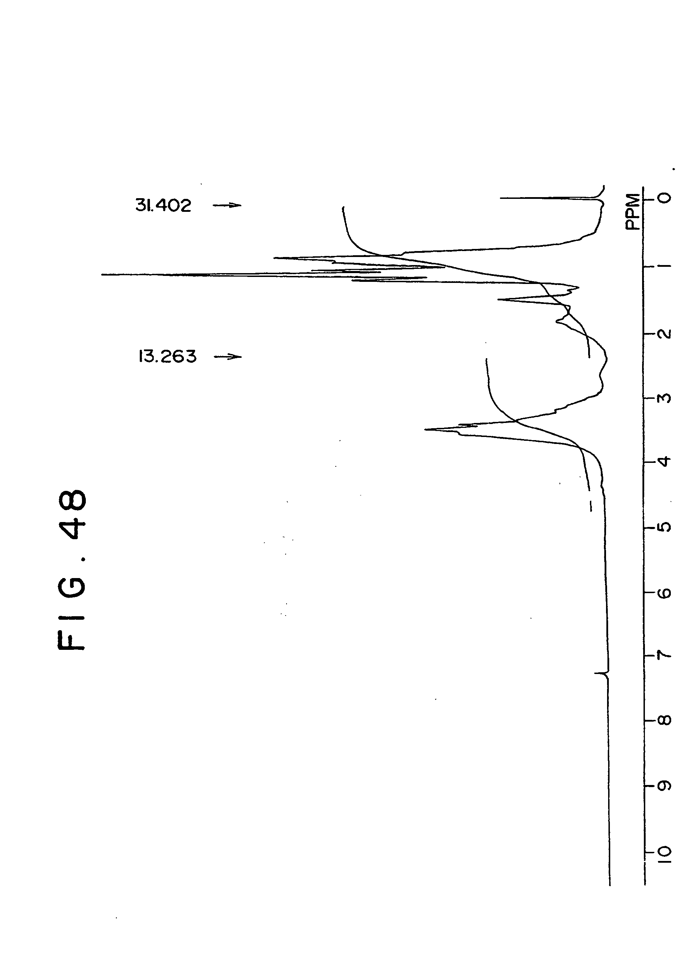

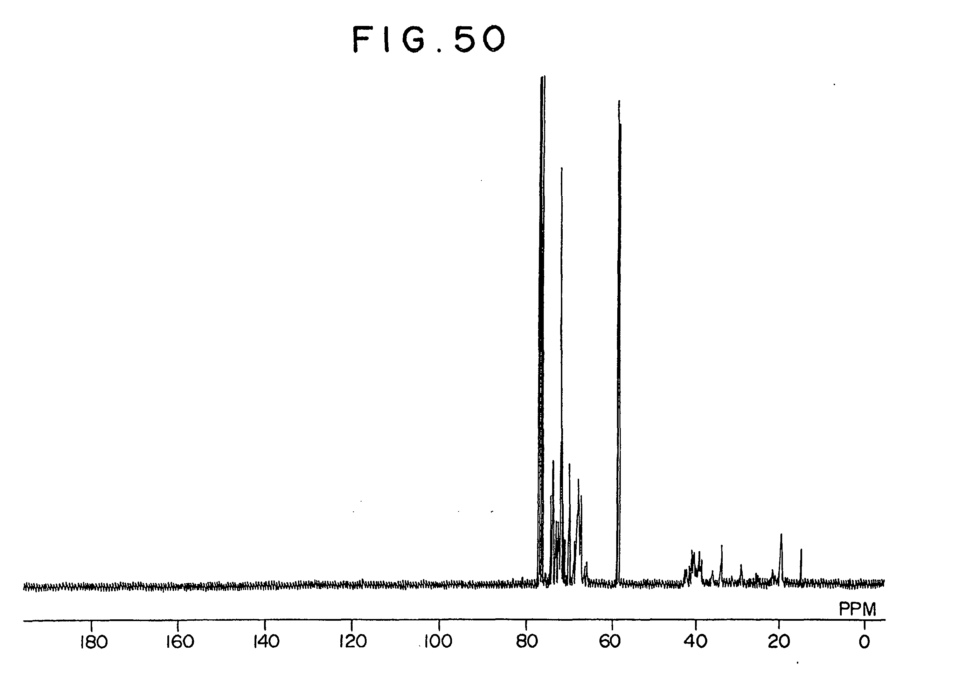

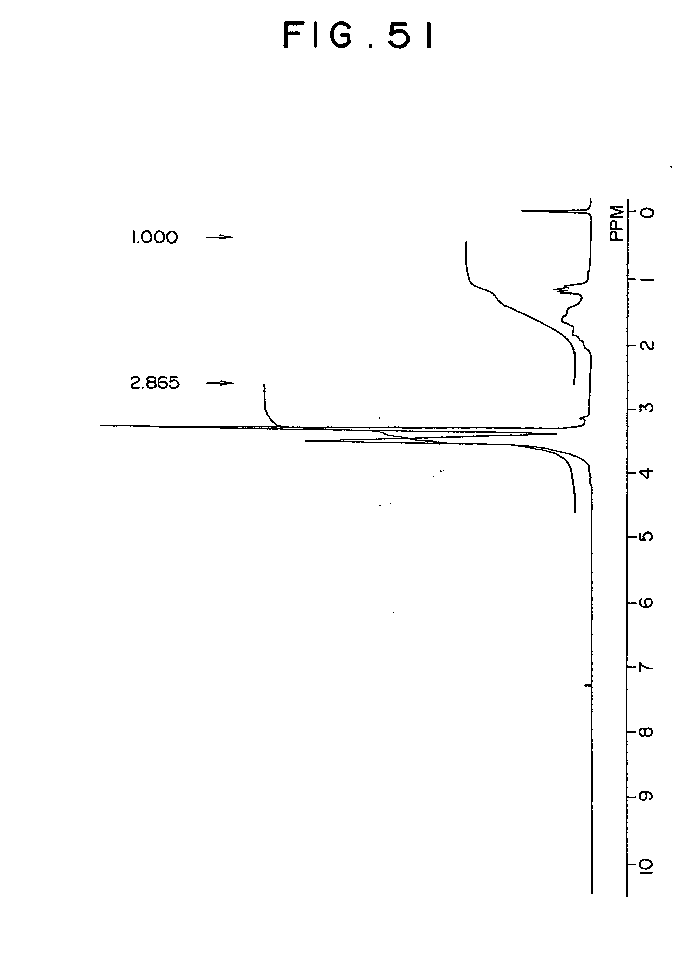

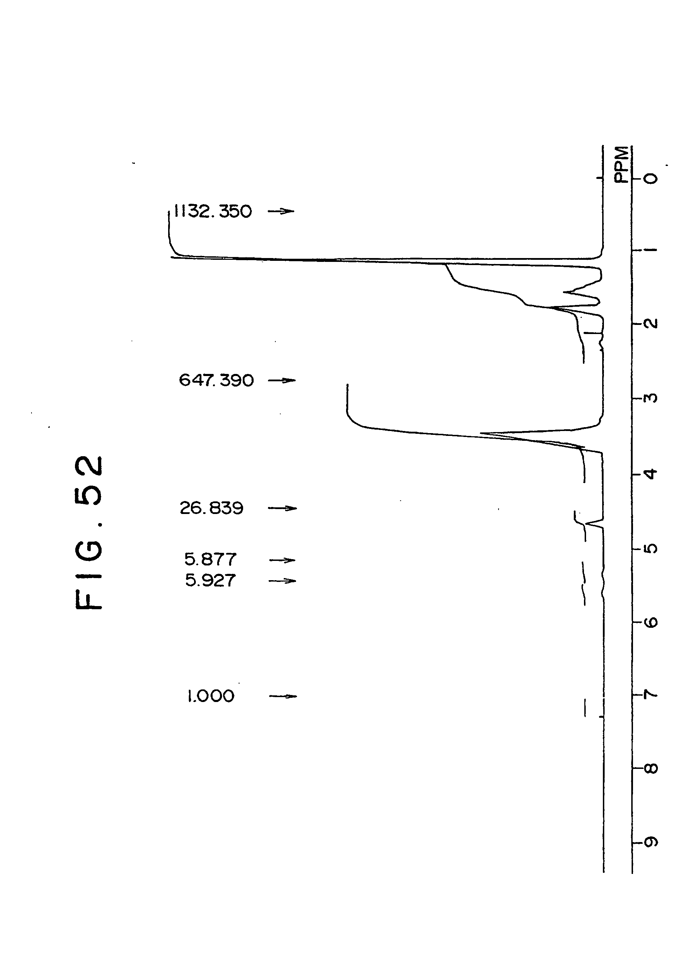

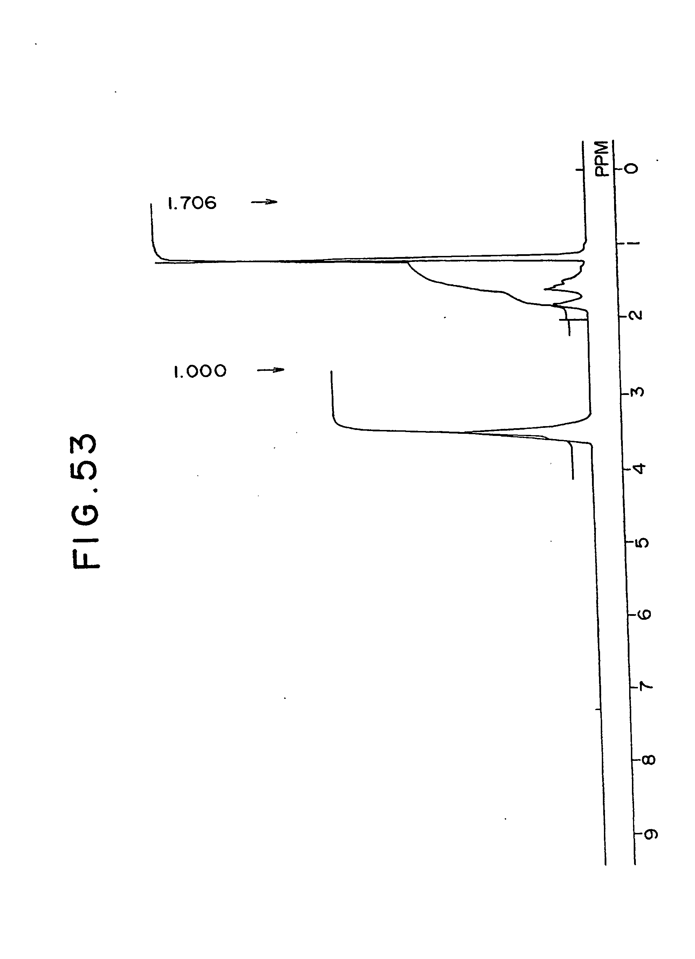

Figure 1, Figure 4, Figure 7, Figure 8, Figure 11, Figure 12, Figure 15, Figure 18, Figure 21, Figure 24, Figure 27, Figure 30, Figure 33, Figure 36, Figure 37, Figure 40, Figure 43, Figure 46 and Figure 49 are each the infrared absorption spectra of the polyvinyl ether compounds obtained in Reference Examples 8, 9, 10, 11, 12, 13, 14, 15, 16, 17, 18, 19, 20, 21, 22, 23, 24, 25 and 25A, respectively; Figure 2, Figure 5, Figure 9, Figure 13, Figure 16, Figure 19, Figure 22, Figure 25, Figure 28, Figure 31, Figure 34, Figure 38, Figure 41, Figure 44, Figure 47 and Figure 50 are each the 13C-NMR charts of the polyvinyl ether compounds obtained in Reference Examples 8, 9, 11, 13, 14, 15, 16, 17, 18, 19, 20, 22, 23, 24, 25 and 25A, respectively; Figure 3, Figure 6, Figure 10, Figure 14, Figure 17, Figure 20, Figure 23, Figure 26, Figure 29, Figure 32, Figure 35, Figure 39, Figure 42, Figure 45, Figure 48 and Figure 51 are each the ¹H-NMR charts of the polyvinyl ether compounds obtained in Reference Examples 8, 9, 11, 13, 14, 15, 16, 17, 18, 19, 20, 22, 23, 24, 25 and 25A, respectively; and Figure 52 and Figure 53 are each ¹H-NMR charts of the acetal oligomer prepared in Example 37 (1) and the ether compound prepared in Example 37 (2), respectively.

### DESCRIPTION OF PREFERRED EMBODIMENTS

The lubricating oil for compression-type refrigerators by the use of the polyvinylether compound of the first object of the invention is described first.

The lubricating oil for compression-type refrigerators comprises as the main component thereof a polyvinyl ether compound having the constituting units expressed by the general formula (XXII): 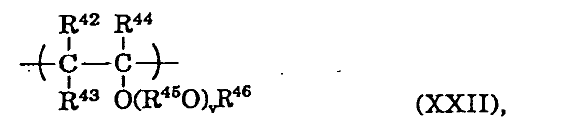 wherein R⁴², R⁴³ and R⁴⁴ are each a hydrogen atom or a hydrocarbon group having 1 to 8 carbon atoms, and may be the same or different from each other, R⁴⁵ is a bivalent hydrocarbon group having 2 to 10 carbon atoms, R⁴⁶ is a hydrocarbon group having 1 to 10 carbon atoms, v is a number the average of which is in the range of 0 to 10, R⁴² to R⁴⁶ may be the same or different from each other among the constituting units and R⁴⁵O may be the same or different from each other when a plural R⁴⁵O are present.

In the formula (XXII), R⁴², R⁴³ and R⁴⁴ are each a hydrogen atom or a hydrocarbon group having 1 to 8 carbon atoms, and may be the same or different from each other. Specific examples of the hydrocarbon group described above include an alkyl group, such as methyl group, ethyl group, n-propyl group, isopropyl group, n-butyl group, isobutyl group, sec-butyl group, tert-butyl group, various kinds of pentyl group, various kinds of hexyl group, various kinds of heptyl group and various kinds of octyl group; a cycloalkyl group, such as cyclopentyl group, cyclohexyl group, various kinds of methylcyclohexyl group, various kinds of ethylcyclohexyl group, various kinds of dimethylcyclohexyl group and the like; an aryl group, such as phenyl group, various kinds of methylphenyl group, various kinds of ethylphenyl group and various kinds of dimethylphenyl group; or an arylalkyl group, such as benzyl group, various kinds of phenylethyl group and various kinds of methylbenzyl group. R⁴², R⁴³ and R⁴⁴ are preferably a hydrogen atom or an aliphatic hydrocarbon group having 5 or less carbon atoms, respectively, and more preferably a hydrogen atom or a hydrocarbon group having 3 or less carbon atoms, respectively.

R⁴⁵ in the formula (XXII) is a bivalent hydrocarbon group having 2 to 10 carbon atoms, which is exemplified by a bivalent aliphatic group, such as ethylene group, phenylethylene group, 1,2-propylene group, 2-phenyl-1,2-propylene group, 1,3-propylene group, various kinds of butylene group, various kinds of pentylene group, various kinds of hexylene group, various kinds of heptylene group, various kinds of octylene group, various kinds of nonylene group and various kinds of decylene group; an alicyclic group having two bonding positions on an alicyclic hydrocarbon, such as cyclohexane, methylcyclohexane, ethylcyclohexane, dimethylcyclohexane, propylcyclohexane and the like; a bivalent aromatic hydrocarbon, such as various kinds of phenylene group, various kinds of methylphenylene group, various kinds of ethylphenylene group, various kinds of dimethylphenylene group, various kinds of naphthylene group and the like; an alkylaromatic group having one univalent bonding position on each of the alkyl group part and the aromatic part of an alkylaromatic hydrocarbon, such as toluene, xylene, ethylbenzene and the like; or an alkylaromatic group having bonding positions on the parts of alkyl groups of a polyalkylaromatic hydrocarbon, such as xylene, diethylbenzene and the like. The aliphatic group having 2 to 4 carbon atoms is particularly preferable among them. A plurality of R⁴⁵O may be the same or different from each other.

In the formula (XXII), k shows the number of repeating and the average of k is in the range of 0 to 10 and preferably in the range of 0 to 5.

In the formula (XXII), R⁴⁶ is a hydrocarbon group having 1 to 10 carbon atoms, which group is exemplified by an alkyl group, such as methyl group, ethyl group, n-propyl group, isopropyl group, n-butyl group, isobutyl group, sec-butyl group, tert-butyl group, various kinds of pentyl group, various kinds of hexyl group, various kinds of heptyl group, various kinds of octyl group, various kinds of nonyl group and various kinds of decyl group; a cycloalkyl group, such as cyclopentyl group, cyclohexyl group, various kinds of methylcyclohexyl group, various kinds of ethylcyclohexyl group, various kinds of propylcyclohexyl group, various kinds of dimethylcyclohexyl group and the like; an aryl group, such as phenyl group, various kinds of methylphenyl group, various kinds of ethylphenyl group, various kinds of dimethylphenyl group, various kinds of propylphenyl group, various kinds of trimethylphenyl group, various kinds of butylphenyl group, various kinds of naphthyl group and the like; or an arylalkyl group, such as benzyl group, various kinds of phenylethyl group, various kinds of methylbenzyl group, various kinds of phenylpropyl group and various kinds of butylphenyl group. The hydrocarbons having 8 or less carbon atoms are preferable among them. When v is 0, an alkyl group having 1 to 6 carbon atoms is particularly preferable and, when v is 1 or more, an alkyl group having 1 to 4 carbon atoms is particularly preferable.

The number of repeating of the polyvinyl ether compound expressed by the formula (XXII) which corresponds to the degree of polymerization can be selected suitably according to the desired kinematic viscosity and is generally in the range of 5 to 1000 cSt at 40°C and preferably in the range of 5 to 800 cSt at 40°C.

The polyvinyl ether compound can be produced by the polymerization of the corresponding vinyl ether monomer and the method of the production of a polyvinyl ether compound described below which is the second object of the invention can be adopted as the method of polymerization.

The ends of the polyvinyl ether compound obtained can be converted to the desired structure by the method disclosed in the examples of the invention or by the conventional methods. The group to be converted to is a saturated hydrocarbon, an ether, an alcohol, a ketone, an amide, an imide, a nitrile or the like.

As the polyvinyl ether compound which is the main component of the lubricating oil, the compounds having the end structures shown in the following are preferably utilized:
(1) The structure in which one end is expressed by the formula (XXIII): 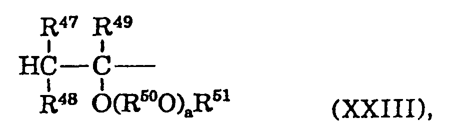 wherein R⁴⁷, R⁴⁸ and R⁴⁹ are each a hydrogen atom or a hydrocarbon group having 1 to 8 carbon atoms, and may be the same or different from each other, R⁵⁰ is a bivalent hydrocarbon group having 2 to 20 carbon atoms, R⁵¹ is a hydrocarbon group having 1 to 10 carbon atoms, a is a number the average of which is in the range of 0 to 10 and R⁵⁰O may be the same or different from each other when a plural R⁵⁰O are present, and the other end is expressed by (XXIV): 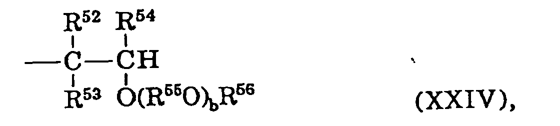 wherein R⁵², R⁵³ and R⁵⁴ are each a hydrogen atom or a hydrocarbon group having 1 to 8 carbon atoms, and may be the same or different from each other, R⁵⁵ is a bivalent hydrocarbon group having 2 to 10 carbon atoms, R⁵⁶ is a hydrocarbon group having 1 to 10 carbon atoms, b is a number the average of which is in the range of 0 to 10 and R⁵⁵O may be the same or different from each other when a plurality of R⁵⁵O are present;
(2) The structure in which one end is expressed by the formula (XXIII) and the other end is expressed by the formula (XXV): 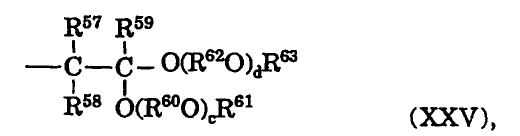 wherein R⁵⁷, R⁵⁸ and R⁵⁹ are each a hydrogen atom or a hydrocarbon group having 1 to 8 carbon atoms, and may be the same or different from each other, R⁶⁰ and R⁶² are a bivalent hydrocarbon group having 2 to 10 carbon atoms, respectively, and may be the same or different from each other, R⁶¹ and R⁶³ are each a hydrocarbon group having 1 to 10 carbon atoms, and may be the same or different from each other, c and d are each a number the average of which is in the range of 0 to 10, and may be the same or different from each other, R⁶⁰O may be the same or different from each other when a plurality of R⁶⁰O are present and R⁶²O may be the same or different from each other when a plurality of R⁶²O are present;
(3) The structure in which one end is expressed by the formula (XXIII) and the other end comprises an olefinically unsaturated bond; and
(4) The structure in which one end is expressed by the formula (XXIII) and the other end is expressed by the formula (XXVI): 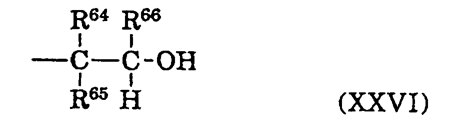
wherein R⁶⁴, R⁶⁵ and R⁶⁶ are each a hydrogen atom or a hydrocarbon group having 1 to 8 carbon atoms, and may be the same or different from each other.

The polyvinyl ether compound may be a mixture of two or more compounds having the end structures selected from the group consisting of the structures (1) to (4). The preferable examples of such mixture are a mixture of the compound having the structure (1) and the compound having the structure (4) and a mixture of the compound having the structure (2) and the compound having the structure (3).

The lubricating oil comprises the polyvinyl ether compound described above as the main component thereof. Because the kinematic viscosity of the lubricating oil before mixing with the refrigerant is preferably in the range of 5 to 1000 cSt at 40°C, the starting materials described above, the initiator and the conditions of the reaction are preferably selected in such a manner that the polyvinyl ether compound having a kinematic viscosity in the aforesaid range is formed. The average molecular weight of this polymer is generally in the range of 150 to 2000 and preferably in the range of 150 to 4000. When kinematic viscosity of a polymer is outside of the specified range, the kinematic viscosity can be adjusted to the specified range by mixing with another polymer having a suitable kinematic viscosity.

In the lubricating oil for refrigerators, the polyvinyl ether compound expressed by the formula (XXII) described above may be utilized singly or as a combination of two or more kinds. It may be utilized by mixing with other kinds of lubricating oils as well.

In the lubricating oil for refrigerators, various kinds of additives utilized in conventional lubricating oils, such as load resistant additives, chlorine scavengers, antioxidants, metal deactivators, defoaming agents, detergent-dispersants, viscosity-index improvers, oiliness agents, anti-wear additives, extreme pressure agents, rust preventives, corrosion inhibitors, pour point depressants and the like, may be added according to desire.

Examples of the load resistant additives described above are: organosulfur compound additives, such as monosulfides, polysulfides, sulfoxides, sulfones, thiosulfinates, sulfurized oils and fats, thiocarbonates, thiophenes, thiazoles, methanesulfonic acid esters and the like; phosphoric ester additives, such as phosphoric monoesters, phosphoric diesters, phosphoric triesters (tricresyl phosphate) and the like; phosphorous ester additives, such as phosphorous monoesters, phosphorous diesters, phosphorous triesters and the like; thiophosphoric ester additives, such as thiophosphoric triesters; fatty acid ester additives, such as higher fatty acids, hydroxylaryl fatty acids, esters of polyhydric alcohols containing carboxylic acids, acrylic esters and the like; organic chlorine additives, such as chlorinated hydrocarbons, chlorinated carboxylic acid derivatives and the like; organic fluorine additives, such as fluorinated aliphatic carboxylic acids, fluoroethylene resins, fluoroalkyl polysiloxanes, fluorinated graphite and the like; alcohol additives, such as higher alcohols and the like; and metallic compound additives, such as salts of naphthenic acid (lead naphthenate), salts of fatty acids (lead salts of fatty acids), salts of thiophosphates (zinc dialkyl dithiophosphates), salts of thiocarbamic acid, organomolybdenum compounds, organotin compounds, organogermanium compounds, boric acid esters and the like.

Examples of the chlorine scavenger are compounds having glycidyl ether group, epoxyfatty acid monoesters, epoxidized fats and oils, compounds having epoxycycloalkyl group and the like. Examples of the antioxidants are phenols (2,6-di-tert-butyl-p-cresol), aromatic amines (α-naphthylamine) and the like. Examples of the metal deactivators are benzotriazole derivatives and the like. Examples of the defoaming agents are silicone oil (dimethylpolysiloxane), polymethacrylates and the like. Examples of the detergent dispersants are sulfonates, phenates, succinimides and the like. Examples of the viscosity index improvers are polymethacrylates, polyisobutylene, ethylenepropylene copolymers, hydrogenated styrene-diene copolymers and the like.

The lubricating oil is utilized as the lubricating oil for compression-type refrigerators because of the excellent compatibility with the refrigerants and the excellent lubricating property. Unlike the conventional lubricating oils, the lubricating oil of the invention has excellent compatibility with hydrogen-containing Flon compounds, such as Flon 134a and the like, which are more specifically 1,1-dichloro-2,2,2-trifluoroethane (Flon 123), 1-chloro-1,1-difluoroethane (Flon 142b), 1,1-difluoroethane (Flon 152a), chlorodifluoromethane (Flon 22), trifluoromethane (Flon 23), difluoromethane (Flon 32), pentafluoroethane (Flon 125) and the like in addition to Flon 134a described above.

The lubricating oil of can be utilized by mixing with another lubricating oil for compression-type refrigerators for the purpose of improving the compatibility with the refrigerant.

The method of production of a polyvinyl ether compound used according to the invention is described next.

In the method of production of a polyvinyl ether compound, the compound expressed by the formula (IX): 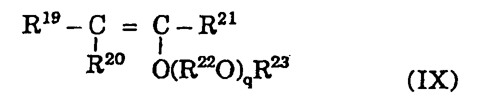 is utilized as the raw material vinyl ether monomer.

In the formula described above, R¹⁹, R²⁰ and R²¹ are each a hydrogen atom or a hydrocarbon having 1 to 8 carbon atoms, and may be the same or different from each other. Examples of the hydrocarbon group are the same as those shown as the examples in the description of R⁴² to R⁴⁴ in the formula (XXII). R¹⁹, R²⁰ and R²¹ are each preferably a hydrogen atom or an aliphatic hydrocarbon group having 5 or less carbon atoms, and more preferably a hydrogen atom or a hydrocarbon group having 3 or less carbon atoms.

In the formula (IX), R²² is a bivalent hydrocarbon group having 2 to 10 carbon atoms and examples of the bivalent hydrocarbon group are the same as those shown as the examples in the description of R⁴⁵ in the formula (XXII). R²² is preferably an aliphatic group having 2 to 4 carbon atoms and R²²O may be the same or different from each other.

In the formula (IX), q shows the number of repeating and the average of q is in the range of 0 to 10 and preferably in the range of 0 to 5.

R²³ in the formula (IX) is a hydrocarbon group having 1 to 10 carbon atoms and examples of the hydrocarbon group are the same as those shown as the examples in the description of R⁴⁶ in the formula (XXII). R²³ is preferably a hydrocarbon group having 8 or less carbon atoms. When q is 0, an alkyl group having 1 to 6 carbon atoms is particularly preferable and, when q is 1 or more, an alkyl group having 1 to 4 carbon atoms is particularly preferable.

Examples of the vinyl ether monomer expressed by the formula (IX) are: vinyl methyl ether, vinyl ethyl ether, vinyl n-propyl ether, vinyl isopropyl ether, vinyl n-butyl ether, vinyl isobutyl ether, vinyl sec-butyl ether, vinyl tert-butyl ether, vinyl n-pentyl ether, vinyl n-hexyl ether, vinyl 2-methoxyethyl ether, vinyl 2-ethoxyethyl ether, vinyl 2-methoxy-1-methylethyl ether, vinyl 2-methoxypropyl ether, vinyl 3,6-dioxaheptyl ether, vinyl 3,6,9-trioxadecyl ether, vinyl 1,4-dimethyl-3,6-dioxaheptyl ether, vinyl 1,4,7-trimethyl-3,6,9-trioxadecyl ether, 1-methoxypropene, 1-ethoxypropene, 1-n-propoxypropene, 1-isopropoxypropene, 1-n-butoxypropene, 1-isobutoxypropene, 1-sec-butoxypropene, 1-tert-butoxypropene, 2-methoxypropene, 2-ethoxypropene, 2-n-propoxypropene, 2-isopropoxypropene, 2-n-butoxypropene, 2-isobutoxypropene, 2-sec-butoxypropene, 2-tert-butoxypropene, 1-methoxy-1-butene, 1-ethoxy-1-butene, 1-n-propoxy-1-butene, 1-isopropoxy-1-butene, 1-n-butoxy-1-butene, 1-isobutoxy-1-butene, 1-sec-butoxy-1-butene, 1-tert-butyoxy-1-butene, 2-methoxy-1-butene, 2-ethoxy-1-butene, 2-n-propoxy-1-butene, 2-isopropoxy-1-butene, 2-n-butoxy-1-butene, 2-isobutoxy-1-butene, 2-sec-butoxy-1-butene, 2-tert-butoxy-1-butene, 2-methoxy-2-butene, 2-ethoxy-2-butene, 2-n-propoxy-2-butene, 2-isoporpoxy-2-butene, 2-n-butoxy-2-butene, 2-isobutoxy-2-butene, 2-sec-butoxy-2-butene, 2-tert-butoxy-2-butene and the like. These vinyl ethers can be produced by any of the conventional methods.

In the method of the invention, a combination of a Brønsted acid, a Lewis acid or an organometallic compound and water, an alcohol, a phenol, an acetal or an addition product of a vinyl ether and a carboxylic acid can be used as the initiator.

Examples of the Brønsted acid are hydrofluoric acid, hydrochloric acid, hydrobromic acid, nitric acid, sulfuric acid, trichloroacetic acid, trifluoroacetic acid and the like. Examples of the Lewis acid are boron trifluoride, aluminum trichloride, aluminum tribromide, tin tetrachloride, zinc dichloride, ferric chloride and the like. Among these Lewis acids, boron trifluoride and complexes thereof are particularly preferable. Examples of the organometallic compound are diethyl aluminum chloride, ethyl aluminum chloride, diethylzinc and the like.

A suitable compound may be selected from water, an alcohol, a phenol, an acetal and an adduct of a vinyl ether with a carboxylic acid and utilized in combination with a Brønsted acid, a Lewis acid or an organometallic compound.

Examples of the alcohol described above are: saturated aliphatic alcohols having 1 to 10 carbon atoms, such as methanol, ethanol, propanol, isopropanol, butanol, isobutanol, sec-butanol, tert-butanol, various kinds of pentanol, various kinds of hexanol, various kinds of octanol and the like; alcohols containing oxygen bonded as the ether bond, such as ethylene glycol monoalkyl ethers, ethylene glycol monoaryl ethers, propylene glycol monoalkyl ethers, propylene glycol monoaryl ethers, polyethylene glycol monoalkyl ethers, polyethylene glycol monoaryl ethers, polypropylene glycol monoalkyl ethers, polypropylene glycol monoaryl ethers, 1,3-dimethoxy-2-propanol, 1-ethoxy-3-methoxy-2-propanol, 1,3-diethoxy-2-propanol and the like; and the like compounds. Among these compounds, aliphatic alcohols having 3 or less carbon atoms are preferable as the aliphatic alcohols, and methanol and ethanol are particularly preferable. As the alcohol containing oxygen bonded as the ether bond, compounds of this structure having 14 or less carbon atoms are preferable.

Examples of the phenol are phenol, various kinds of cresol and the like compounds.

Examples of the acetal are acetaldehyde dimethyl acetal, acetaldehyde diethyl acetal, acetaldehyde methyl ethyl acetal, acetaldehyde di-n-propyl acetal, acetaldehyde methyl n-propyl acetal, acetaldehyde ethyl n-propyl acetal, acetaldehyde diisopropyl acetal, acetaldehyde methyl isopropyl acetal, acetaldehyde ethyl isopropyl acetal, acetaldehyde n-propyl isopropyl acetal, acetaldehyde di-n-butyl acetal, acetaldehyde methyl n-butyl acetal, acetaldehyde ethyl n-butyl acetal, acetaldehyde n-propyl n-butyl acetal, acetaldehyde isopropyl n-butyl acetal, acetaldehyde diisobutyl acetal, acetaldehyde methyl isobutyl acetal, acetaldehyde ethyl isobutyl acetal, acetaldehyde n-propyl isobutyl acetal, acetaldehyde isopropyl isobutyl acetal, acetaldehyde n-butyl isobutyl acetal, acetaldehyde di-sec-butyl acetal, acetaldehyde methyl sec-butyl acetal, acetaldehyde ethyl sec-butyl acetal, acetaldehyde n-propyl sec-butyl acetal, acetaldehyde isopropyl sec-butyl acetal, acetaldehyde n-butyl sec-butyl acetal, acetaldehyde isobutyl sec-butyl acetal, acetaldehyde di-tert-butyl acetal, acetaldehyde methyl tert-butyl acetal, acetaldehyde ethyl tert-butyl acetal, acetaldehyde n-propyl tert-butyl acetal, acetaldehyde isopropyl tert-butyl acetal, acetaldehyde n-butyl tert-butyl acetal, acetaldehyde isobutyl tert-butyl acetal, acetaldehyde sec-butyl tert-butyl acetal, acetaldehyde di(β-methoxyethyl) acetal, acetaldehyde (β-methoxyisopropyl) acetal, propionaldehyde dimethyl acetal, propionaldehyde diethyl acetal, propionaldehyde methyl ethyl acetal, propionaldehyde di-n-propyl acetal, propionaldehyde methyl n-propyl acetal, propionaldehyde ethyl n-propyl acetal, propionaldehyde diisopropyl acetal, propionaldehyde methyl isopropyl acetal, propionaldehyde ethyl isopropyl acetal, propionaldehyde n-propyl isopropyl acetal, propionaldehyde di-n-butyl acetal, propionaldehyde methyl n-butyl acetal, propionaldehyde ethyl n-butyl acetal, propionaldehyde n-propyl n-butyl acetal, propionaldehyde isopropyl n-butyl acetal, propionaldehyde diisobutyl acetal, propionaldehyde methyl isobutyl acetal, propionaldehyde ethyl isobutyl acetal, propionaldehyde n-propyl isobutyl acetal, propionaldehyde isopropyl isobutyl acetal, propionaldehyde n-butyl isobutyl acetal, propionaldehyde di-sec-butyl acetal, propionaldehyde methyl sec-butyl acetal, propionaldehyde ethyl sec-butyl acetal, propionaldehyde n-propyl sec-butyl acetal, propionaldehyde isopropyl sec-butyl acetal, propionaldehyde n-butyl sec-butyl acetal, propionaldehyde isobutyl sec-butyl acetal, propionaldehyde di-tert-butyl acetal, propionaldehyde methyl tert-butyl acetal, propionaldehyde ethyl tert-butyl acetal, propionaldehyde n-propyl tert-butyl acetal, propionaldehyde isopropyl tert-butyl acetal, propionaldehyde n-butyl tert-butyl acetal, propionaldehyde isobutyl tert-butyl acetal, propionaldehyde sec-butyl tert-butyl acetal, propionaldehyde di(β-methoxyethyl) acetal, propionaldehyde di(β-methoxyisopropyl) acetal, n-butyraldehyde dimethyl acetal, n-butyraldehyde diethyl acetal, n-butyraldehyde methyl ethyl acetal, n-butyraldehyde di-n-propyl acetal, n-butyraldehyde methyl n-propyl acetal, n-butyraldehyde ethyl n-propyl acetal, n-butyraldehyde diisopropyl acetal, n-butyraldehyde methyl isopropyl acetal, n-butyraldehyde ethyl isopropyl acetal, n-butyraldehyde n-propyl isopropyl acetal, n-butyraldehyde di-n-butyl acetal, n-butyraldehyde methyl n-butyl acetal, n-butyraldehyde ethyl n-butyl acetal, n-butyraldehyde n-propyl n-butyl acetal, n-butyraldehyde isopropyl n-butyl acetal, n-butyraldehyde diisobutyl acetal, n-butyraldehyde methyl isobutyl acetal, n-butyraldehyde ethyl isobutyl acetal, n-butyraldehyde n-propyl isobutyl acetal, n-butyraldehyde isopropyl isobutyl acetal, n-butyraldehyde n-butyl isobutyl acetal, n-butyraldehyde di-sec-butyl acetal, n-butyraldehyde methyl sec-butyl acetal, n-butyraldehyde ethyl sec-butyl acetal, n-butyraldehyde n-propyl sec-butyl acetal, n-butyraldehyde isopropyl sec-butyl acetal, n-butyraldehyde n-butyl sec-butyl acetal, n-butyraldehyde isobutyl sec-butyl acetal, n-butyraldehyde di-tert-butyl acetal, n-butyraldehyde methyl tert-butyl acetal, n-butyraldehyde ethyl tert-butyl acetal, n-butyraldehyde n-propyl tert-butyl acetal, n-butyraldehyde isopropyl tert-butyl acetal, n-butyraldehyde n-butyl tert-butyl acetal, n-butyraldehyde isobutyl tert-butyl acetal, n-butyraldehyde sec-butyl tert-butyl acetal, n-butyraldehyde di(β-methoxyethyl) acetal, n-butyraldehyde di(β-methoxyisopropyl) acetal and the like.

Examples of the carboxylic acid utilized for forming the adduct with a vinyl ether are acetic acid, propionic acid, n-butyric acid, isobutyric acid, n-valeric acid, isovaleric acid, 2-methylbutyric acid, pivalic acid, n-caproic acid, 2,2-dimehylbutyric acid, 2-methylvaleric acid, 3-methylvaleric acid, 4-methylvaleric acid, enanthic acid, 2-methylcaproic acid, caprylic acid, 2-ethylcaproic acid, 2-n-propylvaleric acid, n-nonanoic acid, 3,5,5-trimethylcaproic acid, undecanoic acid and the like.

The vinyl ether may be the same as or different from those utilized for the polymerization and the specific examples are the same compounds as those mentioned as examples in the description of the vinyl ether monomers expressed by the formula (IX). The adduct of the vinyl ether and the carboxylic acid can be obtained by mixing these compounds and conducting the reaction at a temperature around 0 to 100°C. The adduct may be utilized for the reaction after isolation with distillation or as such without isolation.

To the initiated end of the polymer, hydrogen is attached when water, the alcohol or the phenol is used and, when the acetal is used, the group formed by elimination of one of the alkoxy groups from the used acetal is attached. When the adduct of a vinyl ether with a carboxylic acid is used, the group formed by elimination of the alkylcarbonyloxy group derived from the carboxylic acid from the adduct of the vinyl ether with the carboxylic acid is attached.

On the other hand, to the terminated end of the polymer, an acetal, an olefin or an aldehyde is formed when water, the alcohol, the phenol or the acetal is used. When the adduct of a vinyl ether with a carboxylic acid is used, a carboxylic acid ester of hemiacetal is formed. When the carboxylic acid ester of hemiacetal is hydrolyzed in the presence of an acid, an aldehyde is formed.

Polymerization temperature of the vinyl ether monomer expressed by the formula (IX) is generally in the range of -80 to 150°C and preferably 0 to 100°C although the temperature is varied depending on the kinds of the raw materials and the initiator. The polymerization reaction is finished in a reaction time of about 10 seconds to 10 hours.

For adjusting the molecular weight in the polymerization reaction, the molecular weight of the polymer can be decreased by increasing the amount of water, the alcohol, the phenol, the acetal or the adduct of the vinyl ester with the carboxylic acid relative to the amount of the vinyl ether monomer expressed by the formula (IX), and also by increasing the amount of the Brønsted acid or the Lewis acid.

The polymerization can be performed without a solvent but a solvent which is inert under the reaction condition may be used. The kind of the solvent is not particularly limited. Preferable examples of the solvent are hydrocarbon solvents, such as hexane, benzene, toluene and the like, and ether solvents such as ethyl ether, 1,2-dimethoxyethane, tetrahydrofuran and the like. The polymerization reaction can be terminated by adding an alkali.

By the methods described above, there is obtained the polyvinyl ether compound having the constituting unit expressed by the formula (X): 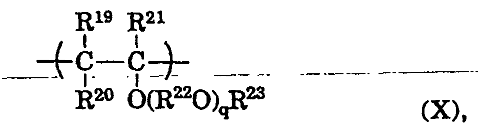 wherein R¹⁹ to R²³ and q are as previously defined.

Among the methods of production of the polyvinyl ether compounds, the two methods shown in the following are preferable.

The first of the preferable methods is the method of polymerizing the vinyl ether monomer in the presence of a Lewis acid catalyst and an acetal expressed by the formula (XI): 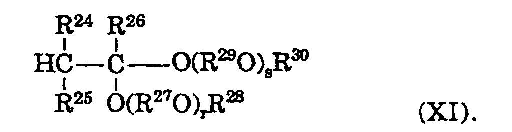 In the acetal compound expressed by the formula (XI) used in the method described above, R²⁴, R²⁵ and R²⁶ are each a hydrogen atom or a hydrocarbon group having 1 to 8 carbon atoms, and may be the same or different from each other. Examples of the hydrocarbon group are the same as those shown as examples in the description of R⁴² and R⁴⁴ in the formula (XXII). R²⁷ and R²⁹ are each a bivalent hydrocarbon group having 2 to 10 carbon atoms, and may the same or different from each other. Examples of the bivalent hydrocarbon group are the same as those shown as examples in the description of R⁴⁵ in the formula (XXII). A plurality of R²⁷O and R²⁹O may be the same or different from each other, respectively. Further, r and s are each a number of repeating the average of which is in the range of 1 to 10, preferably in the range of 0 to 5, and may be the same or different from each other. R²⁸ and R³⁰ are each a hydrocarbon group having 1 to 10 carbon atoms, and may be the same or different from each other. Examples of the hydrocarbon group having 1 to 10 carbon atoms are the same as those shown as examples in the description of R⁴⁶ in the formula (XXII). As R²⁸, an alkyl group having 1 to 6 carbon atoms is particularly preferable when r is 0, and an alkyl group having 1 to 4 carbon atoms is particularly preferable when r is 1 or more. As R³⁰, an alkyl group having 1 to 6 carbon atoms is particularly preferable when s is 0, and an alkyl group having 1 to 4 carbon atoms is particularly preferable when s is 1 or more.

Examples of the acetal expressed by the formula (XI) are the same as those shown as examples in the description of the acetals in the above.

The second of the preferable methods is the method in which an acetal having the formula (XIII): 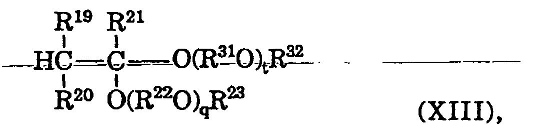 wherein R¹⁹ to R²³, R³¹, R³², q and t are as previously defined, is formed by adding the vinyl ether monomer expressed by the formula (IX) to an alcohol expressed by the formula (XII):

R³²(OR³¹)ₜOH (XII),

in the presence of a Lewis acid catalyst and then the vinyl ether monomer expressed by the formula (IX) is added further to the acetal and polymerized.

In the alcohol expressed by the formula (XII), R³¹ is a bivalent hydrocarbon group having 2 to 10 carbon atoms, which is exemplified by those shown as examples in the description of R⁴⁵ in the formula (XXII). A plurality of R³¹O may be the same or different from each other. Further, t is the number of repeating and the average thereof is in the range of 0 to 10 and preferably in the range of 0 to 5. R³² is a hydrocarbon group having 1 to 10 carbon atoms, which is exemplified by those shown as examples in the description of R⁴⁶ in the formula (XXII). As R³², an alkyl group having 1 to 6 carbon atoms is particularly preferable when t is 0, and an alkyl group having 1 to 4 carbon atoms is particularly preferable when t is 1 or more.

Examples of the alcohol expressed by the formula (XII) are the same as those shown as examples in the description of the alcohols in the above.

Examples of the Lewis acid catalyst utilized in the first and in the second of the preferable methods are the same as those shown as examples in the description of the Lewis acid catalyst in the above. Among these compounds, boron trifluoride and complexes thereof are particularly preferable.

The polymerization of the vinyl ether monomer in the first and in the second of the preferable methods can be performed at a temperature generally in the range of -80 to +150°C, preferably in the range of 0 to 100°C, although the temperature is varied depending on kinds of the raw materials and the initiator. When an acetal is utilized, generation of heat by the heat of polymerization is observed without an induction period and, when an alcohol is utilized, generation of heat by the heat of polymerization is observed without an induction period after the formation of the acetal. Thus, the control of the temperature during the reaction can easily be performed by adding the vinyl ether monomer with a rate which balances with the ability of the apparatus to eliminate the heat. The polymerization is finished generally in the time of 10 seconds to 10 hours after the start of the reaction.

After the polymerization is performed as described in the above, the desired polyvinyl ether compound can be obtained by separation and purification by any of the conventional methods according to necessity.

The method of production of an ether compound as the third object of the invention is described in the following.

In the method of production of an ether compound of the invention, an acetal compound or a ketal compound expressed by the formula (XIV): 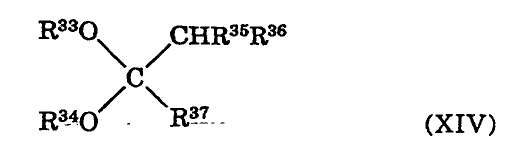 is used as the starting material. In the formula (XIV), R³³ and R³⁴ are each a hydrocarbon group such as methyl group, ethyl group, n-propyl group, isopropyl group and the like, or a hydrocarbon group containing ether oxygens in the main chain, in the side chain or in the both of them, such as the groups expressed by the formulae:

-(CH₂CH₂O)_{w}-R

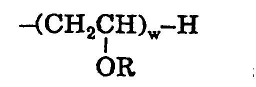

and 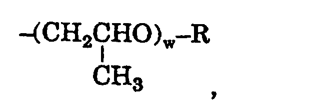 wherein R is a hydrocarbon group having 1 to 10 carbon atoms and w is an integer of 1 to 500. R³³ and R³⁴ may be the same or different from each other. R³⁵, R³⁶ and R³⁷ are each a hydrogen atom, a hydrocarbon group or a hydrocarbon group containing ether oxygens in the main chain, in the side chain or in the both of them. Examples of the hydrocarbon group or the hydrocarbon group containing ether oxygens are the same groups as those shown as examples in the description of R³³ and R³⁴, groups expressed by the formula: 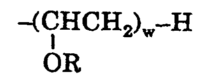 wherein R is a hydrocarbon group having 1 to 10 carbon atoms and w is an integer of 1 to 500, and the like groups. R³⁵, R³⁶ and R³⁷ may be the same or different from each other.

In the invention, an ether compound expressed by the formula (XV): 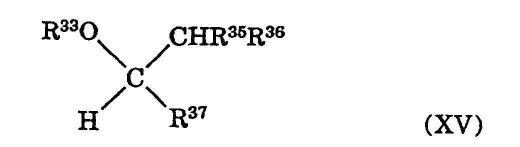 or the formula (XVI): 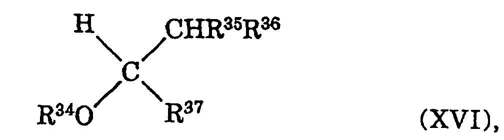 wherein R³³, R³⁴ R³⁵, R³⁶ and R³⁷ are as previously defined, is obtained by the reaction of the acetal compound or the ketal compound expressed by the formula (XIV) with hydrogen in the presence of a solid catalyst having acidic property and hydrogenating ability.

As the acetal compound or the ketal compound expressed by the formula (XIV), is preferable a compound expressed by the formula (XVII): 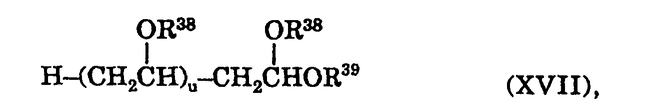 wherein R³⁸ and R³⁹ are each a hydrocarbon group having 1 to 20 carbon atoms or a hydrocarbon group containing ether oxygens, and may be the same or different from each other, R³⁸ is the same or different between the constituting units and u is an integer of 1 to 500. In this case, there is obtained as the ether compound, a compound expressed by the formula (VIII): 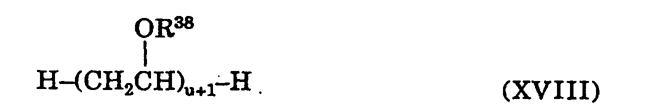 or the formula (XIX): 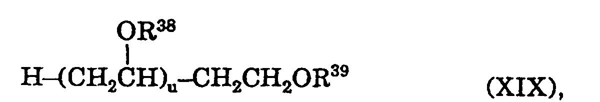 wherein R³⁸, R³⁹ and u are as previously defined.

In the compound expressed by the formula (XVII), a compound expressed by the formula (XXVII): 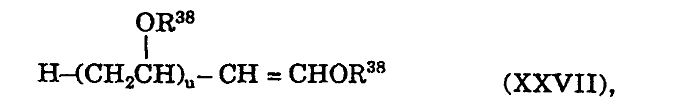 wherein R³⁸ and u are as previously defined, is occasionally contained and, in this case, the ether compound obtained is the compound expressed by the formula (XVIII) described above or a mixture of the compound expressed by the formula (XVIII) and the compound expressed by the formula (XIX).

As the acetal compound and the ketal compound expressed by the formula (XIV), a compound expressed by the formula (XX):

R⁴⁰CH(OR⁴¹)₂ (XX),

wherein R⁴⁰ and R⁴¹ are each a hydrocarbon group having 1 to 20 carbon atoms, and may the same or different from each other, is also favorably used. In this case, a compound expressed by the formula (XXI):

R⁴⁰CH₂OR⁴¹ (XXI)

wherein R⁴⁰ and R⁴¹ are as previously defined, is obtained as the ether compound.

In the method of the invention, a solid catalyst having acidic property and hydrogenating ability is utilized. As the solid catalyst having acidic property and hydrogenating ability, a combination of two kinds of catalyst which are a hydrogenation catalyst and a solid acid catalyst or a solid acid catalyst having hydrogenating ability is utilized.

The hydrogenation catalyst is not particularly limited but various kinds of generally used hydrogenation catalyst can be utilized. Examples of particularly effective catalyst include (1) metals such as nickel, palladium, rhodium and ruthenium alone or as the main component, (2) catalysts having the metal catalyst component of (1) supported on activated charcoal, alumina, diatomaceous earth or the like and (3) Raney catalysts such as Raney nickel and Raney cobalt.

The solid acid catalyst is not particularly limited but various kinds of generally used solid acid catalyst can be used. For example, activated clay, acidic clay, various kinds of zeolite, ion exchange resins, silica-alumina and heteropolyacids are particularly effective.

The solid acid catalyst having hydrogenating ability is not particularly limited but various kinds of generally used solid acid catalyst having hydrogenating ability can be used. For example, catalysts in which nickel, palladium, platinum, ruthenium or the like is supported on various kinds of zeolite are particularly effective.

The amount of the catalyst preferable for performing the method of the invention is as follows. When the combined catalyst is used, the amount of the hydrogenation catalyst is 0.1 to 50 weight % and the amount of the solid catalyst is 0.1 to 50 weight % based on the amount of the reacting materials, respectively. When the solid acid catalyst having hydrogenating ability is used, the amount is 0.1 to 50 weight % based on the amount of the reacting materials. When the amount is less than 0.1 weight %, the reaction does not proceed sufficiently and, when the amount is more than 50 weight %, the amount of the catalyst based on the amount of the reacting materials is too much, causing the problem of decrease in the productivity.

In the present invention, the acetal compound or the ketal compound expressed by the formula (XIV) reacts with hydrogen in the presence of the catalyst described above. It is preferable that hydrogen gas and the acetal compound or the ketal compound be brought into contact with each other in a molar ratio of 1 : 10 to 200 : 1. When the molar ratio is less than the specified range, the reaction does not proceed sufficiently and, when it is more than the specified range, the problem of decrease in the productivity arises.

The preferred reaction conditions for performing the reaction by the method of the invention are: a reaction temperature of 10 to 250°C; a partial pressure of hydrogen of 1 to 200 kg/cm²; a reaction time in the case of the batch reaction of 0.1 to 10 hours; and in the case of the reaction in the liquid flow system, a weight hourly space velocity (WHSV) of the reacting fluid of 0.01 to 100 /hour; and a gas hourly space velocity (GHSV) of hydrogen gas of 100 to 10000 /hour.

The reaction can be performed without a solvent but a solvent may be used, provided it is stable under the reaction conditions. Examples of the usable solvents are hydrocarbon solvents, such as hexane, petane and octane.

By the reaction described above, -OR³³ group or -OR³⁴ group which constitutes a part of the acetal compound or the ketal compound expressed by the formula (XIV) is eliminated from the acetal compound or the ketal compound and replaced with a hydrogen to form the ether compound expressed by the formula (XV) or the formula (XVI). It was made clear in this reaction that, even when any of R³³ to R³⁷ is a hydrocarbon group containing ether oxygens, hydrogen does not react with the ether oxygen but reacts with the oxygen in the part of the acetal or the ketal alone.

After the reaction is finished, the reaction product can be separated from the catalyst by the ordinary filtration or decantation. The catalyst separated here can be used again without particular treatment.

The reaction product may be formed into the product through processes such as distillation, extraction, washing and drying according to necessity.

The novel polyvinyl ether compound as the fourth object of the invention is described in the following.

In the present invention, two kinds of the compounds are provided as the novel polyvinyl ether compound.

The first of the novel polyvinyl ether compounds comprises a homopolymer or a copolymer of an alkyl vinyl ether having an alkyl group of 1 to 3 carbon atoms. It is necessary that the weight-average molecular weight of the polymer be in the range of 300 to 1200, preferably in the range of 400 to 1000. The ratio of the weight-average molecular weight to the number-average molecular weight is generally in the range of 1.05 to 1.50, preferably in the range of 1.06 to 1.40.

The polyvinyl ether compound of the invention can be produced by polymerization of the corresponding vinyl ether monomer. Examples of the vinyl ether monomer are vinyl methyl ether, vinyl ethyl ether, vinyl n-propyl ether, vinyl isopropyl ether, 1-methoxy-1-propene, 1-ethoxy-1-propene and 1-propoxy-1-propene. The vinyl ether monomer can be used singly or as a combination of two or more kinds.

As the method of polymerizing the vinyl ether monomer, the method of production of the polyvinyl ether compound as the second object of the invention described above can be adopted. In this method, the polyvinyl ether compound of the invention is obtained by polymerizing the vinyl ether monomer by using a combination of a Brønsted acid, a Lewis acid or an organometallic compound with an alcohol or an acetal as the initiator. As the Brønsted acid, there are usable the Lewis acid, the organometallic compound and the acetal, the compounds shown as examples in the description of the method of production of the polyvinyl ether compound as the second object of the invention.

Examples of the alcohol are methanol, ethanol, propanol, isopropanol, butanol, isobutanol, sec-butanol, tert-butanol, various kinds of pentanol, various kinds of hexanol and the like. Among these compounds, alcohols having 3 or less carbon atoms are preferable. Particularly, methanol and ethanol are preferred.

The polyvinyl ether compound of the invention thus obtained comprises a homopolymer or a copolymer of an alkyl vinyl ether comprising one or more kinds of the constituting units expressed by the formula (I) or (I'): 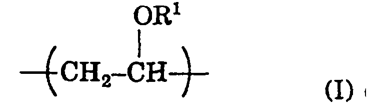 or 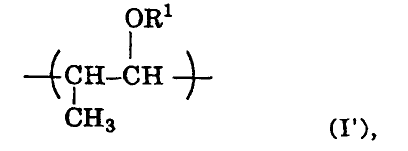 wherein R¹ is an alkyl group having 1 to 3 carbon atoms and one end having the structure expressed by the formula (II): 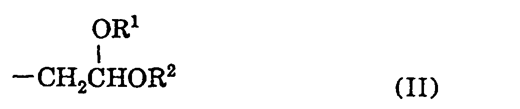 or the formula (III):

-CH = CHOR^{a} (III),

wherein R¹ is as previously defined, R² is a hydrocarbon group having 1 to 8 carbon atoms and R^{a} is either one of R¹ and R².

In the formula (II), R² is derived from the residual group formed by eliminating the hydroxyl group from the alcohol or from the residual group formed by eliminating the oxygen atom from the alkoxy group in the acetal, both used for the initiation of the polymerization.

When the alcohol is used, a hydrogen is attached to the initiated end of the polymer and, when the acetal is used, the residual group formed by eliminating one of the alkoxy groups from the acetal is attached to the initiated end. To the terminated end, the acetal group expressed by the formula (II) described above or the olefin group expressed by the formula (III) described above is attached.

The homopolymer or the copolymer of the alkyl vinyl ether may comprise the terminated end expressed by the formula (II) alone, the terminated end expressed by the formula (III) alone or the terminated end having a mixed structure of them.

The polymerization can be performed at a temperature in the range of -80 to 150°C, preferably 0 to 100°C, although it varies depending on the kinds of the starting materials and the initiator. The polymerization reaction is finished generally in a time of 10 seconds to 10 hours after the start of the reaction.

For adjusting the molecular weight in the polymerization reaction, the molecular weight of the polymer is decreased by an increased amount of the alcohol or the acetal.

The polymerization can be performed without a solvent but a solvent may be used provided it is stable under the reaction condition. The solvent is not particularly limited. Preferable examples of the solvent are hydrocarbon solvents, such as hexane, benzene and toluene and ether solvents, such as ethyl ether, 1,2-dimethoxyethanem and tetrahydrofuran. The polymerization reaction can be terminated by addition of an alkali.

The second of the polyvinyl ether compounds of the invention comprises the constituting units expressed by the formula (IV): 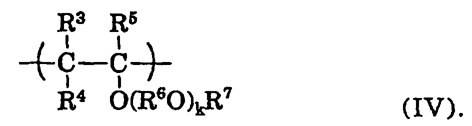 In the formula (IV) described above, R³, R⁴ and R⁵ are each a hydrogen atom or an alkyl group having 1 to 4 carbon atoms. Examples of the alkyl group are methyl group, ethyl group, n-propyl group, isopropyl group, n-butyl group, isobutyl group, sec-butyl group and tert-butyl group. R³, R⁴ and R⁵ may be the same or different from each other. Among hydrogen and the groups described above, hydrogen, methyl group and ethyl group are preferable and it is preferred that at least one of R³, R⁴ and R⁵ be hydrogen atom.

R⁶ in the formula (IV) is an alkylene group having 2 to 4 carbon atoms and, more specifically, ethylene group, trimethylene group, methylethylene group, tetramethylene group, 1,1-dimethylethylene group or 1,2-dimethylethylene group. Among them, alkylene groups having 2 or 3 carbon atoms are particularly preferable. In the formula (IV), k is the number of repeating of R⁶O, the average of which is in the range of 0 to 10, preferably 0 to 5.

R⁷ in the formula (IV) is an alkyl group having 1 to 10 carbon atoms. Examples of the alkyl group are alkyl groups, such as methyl group, ethyl group, n-propyl group, isopropyl group, n-butyl group, isobutyl group, sec-butyl group, tert-butyl group, various kinds of pentyl group, various kinds of hexyl group and various kinds of octyl group, cycloalkyl groups, such as cyclopentyl group, cyclohexyl group, various kinds of methylcyclohexyl group, various kinds of ethylcyclohexyl group, various kinds of dimethylcyclohexyl group and the like groups. Among them, alkyl groups having 8 or less carbon atoms are preferable. Alkyl groups having 1 to 6 carbon atoms are particularly preferable when k is 0, and alkyl groups having 1 to 4 carbon atoms are particularly preferable when k is 1 or more.

R³ to R⁷ may be the same or different among the constituting units and a plurality of R⁶O may be the same or different from each other when the constituting unit contains a plurality of R⁶O.

It is necessary that the polyvinyl ether compound of the invention does not contain any of an unsaturated bond, an acetal structure and an aldehyde structure in the molecule. The polyvinyl ether compound generally has the structure in which one end is expressed by the formula (V): 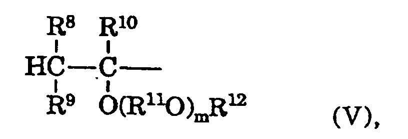 wherein R⁸, R⁹ and R¹⁰ are each a hydrogen atom or an alkyl group having 1 to 4 carbon atoms, and may be the same or different from each other, R¹¹ is an alkylene group having 2 to 4 carbon atoms, R¹² is an alkyl group having 1 to 10 carbon atoms, m is a number the average of which is in the range of 0 to 10 and a plurality of R¹¹O may be the same or different from each other, and the other end is expressed by the formula (VI): 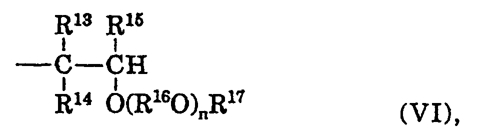 wherein R¹³, R¹⁴ and R¹⁵ are each a hydrogen atom or an alkyl group having 1 to 4 carbon atoms, may be the same or different from each other, R¹⁶ is an alkylene group having 2 to 4 carbon atoms, R¹⁷ is an alkyl group having 1 to 10 carbon atoms, n is a number the average of which is in the range of 0 to 10 and a plurality of R¹⁶O may be the same or different from each other.

In the formula (V) described above, R⁸, R⁹ and R¹⁰ are each a hydrogen atom or an alkyl group having 1 to 4 carbon atoms. Examples of the alkyl group are the same as those shown as examples in the description of R³ to R⁵ in the formula (IV). R⁸, R⁹ and R¹⁰ may be the same or different from each other. Among them, a hydrogen atom, methyl group and ethyl group are preferable and it is preferred that at least one of R⁸, R⁹ and R¹⁰ be a hydrogen atom.

R¹¹ in the formula (V) is an alkylene group having 2 to 4 carbon atoms. Examples of the alkylene group are the same as those shown as examples in the description of R⁶ in the formula (IV). Among them, alkylene groups having 2 or 3 carbon atoms are particularly preferable. In the formula (V), m shows the repeating number of R¹¹O, the average of which is in the range of 0 to 10, preferably 0 to 5. When a plurality of R¹¹O are present, a plurality of R¹¹O may be the same or different from each other.

R¹² in the formula (V) is an alkyl group having 1 to 10 carbon atoms. Examples of the alkyl group are the same as those shown as examples in the description of R⁷ in the formula (IV). Among them, alkyl groups having 8 or less carbon atoms are preferable. Alkyl groups having 1 to 6 carbon atoms are particularly preferable when m is 0, and alkyl groups having 1 to 4 carbon atoms are particularly preferable when m is 1 or more.

In the formula (VI) described above, R¹³, R¹⁴ and R¹⁵ are each a hydrogen atom or an alkyl group having 1 to 4 carbon atoms. Examples of the alkyl group are the same as those shown as examples in the description of R³ to R⁵ in the formula (IV). R¹³, R¹⁴ and R¹⁵ may be the same or different from each other. Among them, a hydrogen atom, methyl group and ethyl group are preferable and it is preferred that at least one of R¹³, R¹⁴ and R¹⁵ be a hydrogen atom.

R¹⁶ in the formula (VI) is an alkylene group having 2 to 4 carbon atoms. Examples of the alkylene group are the same as those shown as examples in the description of R⁶ in the formula (IV). Among them, alkylene groups having 2 or 3 carbon atoms are particularly preferable. In the formula (VI), n shows the repeating number of R¹⁶O, the average of which is in the range of 0 to 10, preferably 0 to 5. When a plurality of R¹⁶O are present, a plurality of R¹⁶O may be the same or different from each other.

R¹⁷ in the formula (VI) is an alkyl group having 1 to 10 carbon atoms. Examples of the alkyl group are the same as those shown as examples in the description of R⁷ in the formula (IV). Among them, alkyl groups having 8 or less carbon atoms are preferable. Alkyl groups having 1 to 6 carbon atoms are particularly preferable when n is 0, and alkyl groups having 1 to 4 carbon atoms are particularly preferable when n is 1 or more.

The polyvinyl ether compound of the invention has a weight-average molecular weight in the range of 300 to 3000, preferably 400 to 2000, more preferably 400 to 1000 and, a degree of polymerization preferably in the range of 5 to 10. The ratio of the weight-average molecular weight to the number-average molecular weight is in the range of 1.05 to 2.00, preferably 1.06 to 1.90.

The preferable compounds among the polyvinyl ether compounds of the invention are compounds which have the constituting units expressed by the formula (VII): 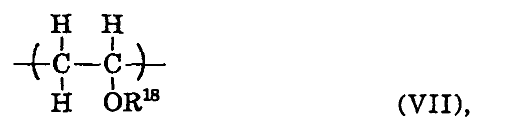 wherein R¹⁸ is an alkyl group having 1 to 4 carbon atoms and may be the same or different among the constituting units, do not contain any of an unsaturated bond, an acetal structure and an aldehyde structure and have a weight-average molecular weight in the range of 300 to 3000. The more preferable compounds among them are compounds having the constituting units expressed by the formula (VIII): 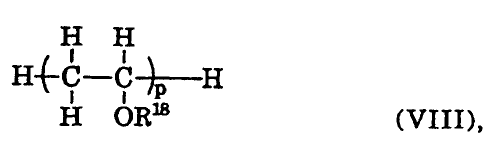 wherein R¹⁸ is as previously defined and p is a degree of polymerization, and a weight-average molecular weight in the range of 300 to 3000, preferably 400 to 1000.

The polyvinyl ether compound of the invention can be produced by (a) the process of polymerizing the corresponding vinyl ether monomer and (b) the process of treating olefins, acetals and aldehydes in the polymerized product.
(a) The process of polymerization: As the method of polymerization of the vinyl ether compound, the method of production of the polyvinyl ether compound as the second object of the invention described above can be adopted.
(b) The process of treatment: In this process, unsaturated bonds, acetals and aldehydes in the polymerized product are converted into saturated bonds and ethers.

### (1) Unsaturated bonds

Unsaturated bonds formed at the end of the polymer by the polymerization can be converted into saturated bonds in conventional conditions, such as a reaction temperature of 20 to 200°C, a hydrogen pressure of 1 to 100 kg/cm² and in the presence of a hydrogenation catalyst. As the hydrogenation catalyst, platinum catalysts, rhodium catalysts, ruthenium catalysts, nickel catalysts, cobalt catalysts and the like are preferable.

### (2) Aldehydes

Aldehydes formed by the hydrolysis of acetals or esters made from carboxylic acids and hemiacetals can be converted to alcohols by the hydrogenation reaction and then further converted to ethers by the Williamson synthesis. More specifically, platinum catalysts, palladium catalysts, ruthenium catalysts and nickel catalysts are particularly preferable as the hydrogenation catalyst and the reaction can be performed at a reaction temperature of 20 to 200°C and a hydrogen pressure of 1 to 100 kg/cm². The reaction for converting to ethers is generally performed by the method in which the hydroxyl group is brought into the reaction with an alkali metal such as metallic sodium, an alkali metal hydride such as sodium hydride, an alkali hydroxide such as sodium hydroxide or an alkali metal salt of a lower alcohol such as sodium methoxide to obtain the alkali salt of the polymerization product and the product is then brought into the reaction with an alkyl halogen compound or an ester of a sulfonic acid respectively having 1 to 10 carbon atoms or by the method in which the hydroxyl group in the polymerization product is converted to esters of a sulfonic acid or halides and the product is then brought into reaction with an aliphatic alcohol having 1 to 10 carbon atoms or an alkali metal salt thereof.

### (3) Acetal

The acetal formed at the end by the polymerization forms aldehydes by the reaction with water in the presence of an acid catalyst such as hydrochloric acid, sulfuric acid and sulfonic acid can be converted to ethers by the methods described above. Examples of the method of directly converting the acetals to ethers are the method of using LiAlH₄-BF₃, LiAlH₄-AlCl₃, B₂H₆, NaB(CN)H₃-HCl or (CH₃O)AlH₂, the method of using silicon reagents and the like described in Jikken Kagaku Koza, the 4th edition, Volume 20, Page 202 (published by Maruzen), and the method of reaction in a hydrogen atmosphere in the presence of a catalyst system in combination of platinum oxide and hydrochloric acid described in Ian T. Harrison and Shuyen Harrison, Compendium of Organic Synthetic Methods, Page 317 (1971).

Further, acetals can be efficiently converted to ethers by the method of the production of ether compound as the third object of the invention which comprises hydrogenation in the presence of a solid catalyst having acidic property and hydrogenating ability. As the solid catalyst having acidic property and hydrogenating ability utilized for the reaction and the conditions of the reaction, the solid catalysts and the conditions of the reaction described in the production of the ether compound can be adopted.

The lubricating oil for compression-type refrigerators of the invention has excellent compatibility with the refrigerant, stability, electric insulation and lubricating property. Therefore, it is particularly favorable as the lubricating oil for compression-type refrigerators using Flon 134a as the refrigerant.

According to the method of production of the polyvinyl ether compound of the invention, the polyvinyl ether with a desired degree of polymerization and having a wide range of applications, such as solvents, lubricating oils, adhesives and resins, can be produced reliably, safely, efficiently and industrially advantageously with simple processes.

According to the method of production of the ether compound, the ether compound can be produced from the acetal compound or the ketal compound with a high conversion and a high selectivity and the apparatus used for ordinary productions can be utilized because the problem of corrosion does not arise. According to the method of the invention, the acetals or the ketals alone are hydrogenated and even when the raw material acetal or ketal compound contains hydrocarbon groups having ether oxygens, the part of the ether oxygen remains intact and the acetal bond is converted to the ether bond.

Further, the polyvinyl ether compound of the invention comprises a homopolymer or a copolymer of an alkyl vinyl ether having alkyl group of 1 to 3 carbon atoms and having a relatively low molecular weight and a narrower dispersion thereof and a homopolymer or a copolymer of a vinyl ether monomer having none of unsaturated bonds, acetal structures and aldehyde structures in the molecule. The polyvinyl ether compound has excellent compatibility particularly with hydrogen-containing Flons such as Flon 134a, excellent stability and lubricating property and a volume intrinsic resistance at 80°C of 10¹² Ω·cm or more and is favorably used as the lubricating oil for compression-type refrigerators.

The polyvinyl ether compound is useful also as an electric insulating oil, an organic solvent, a surface active agent and the like.

The present invention is described with reference to examples in more detail in the following. However, the invention is not limited by the examples.

The methods of measurements of the kinematic viscosity, the average molecular weight, the compatibility with Flon and the volume intrinsic resistance and the method of hydrolysis testing of the polyvinyl ether compound are described in the following.

### (1) Kinematic viscosity

Kinematic viscosity was measured according to the method of Japanese Industrial Standard K-2283 (1983) by using a glass capillary viscometer.

### (2) Average molecular weight (Examples 8 to 25 and 25A)

Weight-average molecular weight and number-average molecular weight were measured by using the apparatus and under the conditions shown in the following and dispersion (weight-average molecular weight / number-average molecular weight) was obtained from these results.
Apparatus: a product of Nippon Bunko Kogyo Co., Ltd., 880-PU® (pump).
   Shodex RI SE-61® (detector)
Column: TSK H8 + G2000 H8 + G1000 H8
Temperature: room temperature
Solvent: THF (tetrahydrofuran)
Rate of elution: 1.4 ml/minute
Standard substance: polyethylene glycol

### (3) Compatibility test

### (a) Flon 134a

A sample of a specified amount adjusted to make 5 weight % or 10 weight % based on Flon 134a (1,1,1,2-tetrafluoroethane) was charged into a pressure resistant glass ampoule and the ampoule was connected to a vacuum line and the line for Flon 134a gas. The ampoule was degassed in vacuum at room temperature, cooled with liquid nitrogen and a specified amount of Flon 134a was taken into the ampoule. The ampoule was then sealed and the temperature at which the phase separation starts was measured as follows: For the measurement of the compatibility at the low temperature side, the sample was slowly cooled from room temperature to -60°C in a thermostat and, for the measurement of the compatibility at the higher temperature side, the sample was slowly heated from room temperature to +80°C. It is preferable that the phase separation temperature be lower in the lower temperature side, but be higher in the higher temperature side.

### (b) Flon 32

A sample of a specified amount adjusted to make 10 weight % or 20 weight % based on Flon 32 (difluoromethane) was charged into a pressure resistant glass ampoule and the ampoule was connected to a vacuum line and the line for Flon 32 gas. The ampoule was degassed in vacuum at room temperature, cooled with liquid nitrogen and a specified amount of Flon 32 was taken into the ampoule. The ampoule was then sealed and the temperature at which the phase separation starts was measured as follows: For the measurement of the compatibility at the low temperature side, the sample was slowly cooled from room temperature in a thermostat and, for the measurement of the compatibility at the higher temperature side, the sample was slowly heated from room temperature to +40°C. It is preferable that the phase separation temperature be lower in the lower temperature side, but be higher in the higher temperature side.

### (4) Volume intrinsic resistance

A sample was dried under a reduced pressure (0.3 to 0.8 mmHg) at 100°C for 1 hour and then charged into a liquid cell for the measurement of volume intrinsic resistance which is placed into a thermostat at 80°C. After the sample was kept in the thermostat at 80°C for 40 minutes, the volume intrinsic resistance was measured at an impressed voltage of 250 V by using an ultrainsulation meter R8340® produced by Advantest Co.

### (5) Hydrolysis test

Into a 250 ml pressure resistant glass bottle, 75 g of a sample, 25 g of water and a piece of copper (13 × 50 mm) were placed and the atmosphere in the bottle was replaced with nitrogen. The sample was kept in a rotatory thermostat at a temperature of 102°C for 192 hours. After finishing the test, appearance of the sample and condition of the copper piece were visually observed and the total acid value was measured.

### Preparation Example 1

Into a flask, 100 g (containing water) of Raney nickel (a product of Kawaken Fine Chemical Co., Ltd., M300T®) was charged and 100 g of absolute ethanol was added to it with sufficient stirring. The mixture was left standing to have Raney nickel precipitated and the supernatant was removed by decantation. The Raney nickel remaining in the flask was treated in the same manner as above 5 times repeatedly. Raney nickels used in examples were those obtained in this preparation example which were wet with ethanol.

### Preparation Example 2

Into a 100 ml flask of egg-plant type, 20 g of zeolite (a product of Toso Co., Ltd., HSZ330HUA®) was placed. The flask was then dipped in an oil bath at 150°C and evacuated by an oil rotary vacuum pump for 1 hour. After cooling to room temperature, the flask was brought to the atmospheric pressure by introducing dry nitrogen. The zeolites used in examples were those obtained in this preparation example.

### Preparation Example 3

Into a 100 ml flask of egg-plant type, 20 g of activated clay (a product of Wako Jun-yaku Co., Ltd.) was placed. The flask was then dipped in an oil bath at 150°C and evacuated by an oil rotary vacuum pump for 1 hour. After cooling to room temperature, the flask was brought to the atmospheric pressure by introducing dry nitrogen. The activated days used in examples were those obtained in this preparation example.

### Example 1

Into a 5 liter glass flask equipped with a dropping funnel, a cooler and a stirrer, 600 g of ethyl vinyl ether, 72 g of methanol and 2400 g of hexane were charged and cooled with water under stirring. When the temperature reached 5°C, a solution prepared by dissolving 3.6 g of boron trifluoride diethyl etherate in 20 g of tetrahydrofuran was added and the mixture was stirred for 1 hour. During this period, the reaction started and the temperature of the reaction liquid increased, and refluxing of ethyl vinyl ether was observed in the cooler. The reaction mixture was transferred to a washing vessel and washed with 1500 ml of a 3 weight % aqueous solution of sodium hydroxide 3 times, then with 1500 ml of water 3 times. The product was concentrated by using a rotary evaporator and dried under reduced pressure (0.2 mmHg) at 50°C for 1 hour to obtain 468 g of the crude product.

Into a 5 liter glass flask equipped with a dropping funnel, a cooler and a stirrer, 400 g of the crude product obtained above, 500 g of acetone, 800 g of water and 10 g of a concentrated hydrochloric acid (35 weight %) were charged and stirred at 50 to 60°C for 3 hours. After hydrochloric acid was neutralized with sodium hydrogencarbonate, acetone and the like were removed under reduced pressure by using a rotary evaporator and the remaining product was poured into 400 ml of hexane. After removing the aqueous phase, the product was washed with 400 ml of water once. The hexane phase was transferred to an autoclave and hydrogenated by using 0.8 g of a platinum oxide as catalyst at a hydrogen pressure of 20 kg/cm² at 50°C for 1 hour. After removing the platinum oxide catalyst by filtration, the product was transferred to a 2 liter glass flask, incorporated with 40 g of methanol and 8 g of sodium borohydride and stirred for 1 hour at room temperature. The product was made weakly acidic with an aqueous solution of acetic acid and then acetic acid was neutralized with sodium hydrogencarbonate. The product was washed with 400 ml of water once and the solvent and water were removed under reduced pressure by using a rotary evaporator.

The residual product thus obtained was dissolved in 300 ml of tetrahydrofuran and the reaction with 12 g of sodium hydride was conducted for 1 hour. Generation of hydrogen gas was observed during this reaction. Then, 120 g of methyl iodide was dropped in 30 minutes to the reaction mixture. Generation of heat was observed during this period. After dropping methyl iodide, the reaction mixture was stirred for further 1 hour. The solvent and unreacted materials were removed under reduced pressure by using a rotary evaporator. The residue was transferred to a 2 liter washing vessel and, after dissolving into 400 ml of hexane, washed with 400 ml of water 5 times. Then, after adding 40 g of an ion exchange resin, the product was stirred for 3 hours. The ion exchange resin was removed by filtration and hexane was removed from the product under reduced pressure by using a rotary evaporator. The yield of polyvinyl ether thus obtained was 200 g.

The nuclear magnetic resonance spectrum (hereinafter abbreviated to NMR) and the infrared absorption spectrum (hereinafter abbreviated to IR) of the product were measured and it was proved that one of the end structures of the polymer was (A) and the other was (B):

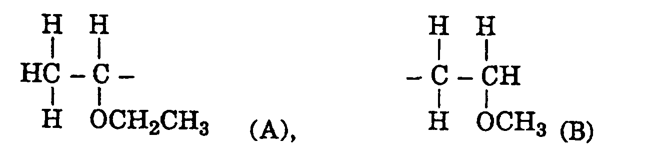

Kinematic viscosity, compatibility with Flon, volume intrinsic resistance and stability to hydrolysis of the polymer of ethyl vinyl ether were measured. The results are shown in Table 1.

### Example 2

Into a 5 liter glass flask equipped with a dropping funnel, a cooler and a stirrer, 600 g of ethyl vinyl ether, 60 g of methanol and 2400 g of hexane were charged and cooled with water under stirring. When the temperature reached 5°C, a solution prepared by dissolving 3.6 g of boron trifluoride diethyl etherate in 20 g of tetrahydrofuran was added and the mixture was stirred for 1 hour. During this period, the reaction started and the temperature of the reaction increased, and refluxing of ethyl vinyl ether was observed in the cooler. The reaction mixture was transferred to a washing vessel and washed with 1500 ml of a 3 weight % aqueous solution of sodium hydroxide 3 times, then with 1500 ml of water 3 times. The product was concentrated by using a rotary evaporator and dried under reduced pressure (0.2 mmHg) at 50°C for 1 hour to obtain 452 g of the crude product.

Into a 5 liter glass flask equipped with a dropping funnel, a cooler and a stirrer, 400 g of the crude product obtained above, 500 g of acetone, 800 g of water and 10 g of a concentrated hydrochloric acid (35 weight %) were charged and stirred at 50 to 60°C for 3 hours. After hydrochloric acid was neutralized with sodium hydrogencarbonate, acetone and the like were removed under reduced pressure by using a rotary evaporator and the remaining product was poured into 400 ml of hexane. After removing the aqueous phase, the product was washed with 400 ml of water once. The hexane phase was transferred to an autoclave and hydrogenated by using 0.8 g of a platinum oxide as catalyst at the hydrogen pressure of 20 kg/cm² at 50°C for 1 hour. After removing the platinum oxide catalyst by filtration, the product was transferred to a 2 liter glass flask, incorporated with 40 g of methanol and 8 g of sodium borohydride and stirred for 1 hour at room temperature. The product was made weakly acidic with an aqueous solution of acetic acid and then acetic acid was neutralized with sodium hydrogencarbonate. The product was washed with 400 ml of water once and the solvent and water were removed under reduced pressure by using a rotary evaporator.

The residual product thus obtained was dissolved in 300 ml of tetrahydrofuran and the reaction with 12 g of sodium hydride was conducted for 1 hour. Generation of hydrogen gas was observed during this reaction. Then, 120 g of methyl iodide was dropped in 30 minutes to the reaction mixture. Generation of heat was observed during this period. After dropping methyl iodide, the reaction mixture was stirred for further 1 hour. The solvent and unreacted materials were removed under reduced pressure by using a rotary evaporator. The residue was transferred to a 2 liter washing vessel and, after dissolving into 400 ml of hexane, washed with 400 ml of water 5 times. Then, after adding 40 g of an ion exchange resin, the product was stirred for 3 hours. The ion exchange resin was removed by filtration and hexane was removed from the product under reduced pressure by using a rotary evaporator. The yield of polyvinyl ether thus obtained was 208 g. NMR and IR of the product were measured and it was proved that one of the end structures of the polymer was (A) and the other was (B).

Kinematic viscosity, compatibility with Flon, volume intrinsic resistance and stability to hydrolysis of the polymer of ethyl vinyl ether were measured. The results are shown in Table 1.

### Example 3

Into a 5 liter glass flask equipped with a dropping funnel, a cooler and a stirrer, 500 g of hexane was charged, a solution prepared by dissolving 12 g of boron trifluoride diethyl etherate in 24 g of tetrahydrofuran was added to it and the mixture was cooled to 5°C with an ice water bath. In a dropping funnel, 2000 g of ethyl vinyl ether and 120 g of methanol were charged and the mixture was dropped in 1 hour and 30 minutes. During this period, the reaction started and the temperature of the reaction liquid increased. The temperature was kept at about 30°C by cooling with the ice water bath. After finishing the dropping, the stirring was kept for further 30 minutes. The reaction mixture was transferred to a washing vessel and washed with 1500 ml of a 3 weight % aqueous solution of sodium hydroxide 3 times, then with 1500 ml of water 3 times. The solvent and unreacted raw materials were removed under reduced pressure by using a rotary evaporator to obtain 1806 g of the crude product.

Into a 2 liter glass flask equipped with a dropping funnel, a cooler and a stirrer, 400 g of the crude product obtained above and 300 g of tetrahydrofuran were charged and then 15 g of lithium aluminum hydride was added to the mixture, followed by stirring for 30 minutes. In a dropping funnel, 196 g of boron trifluoride diethyl etherate was charge and dropped in 1 hour. During the dropping, generation of heat was observed and the temperature was kept at 10 to 20°C by cooling with an ice water bath. The reaction mixture was further stirred for 30 minutes after finishing the dropping and then neutralized by adding sodium hydroxide. The precipitate thus obtained was removed by filtration and the liquid phase was treated with a rotary evaporator to remove the alcohol produced, the solvent and water under reduced pressure. The remaining product was transferred to a 2 liter washing vessel and dissolved into 500 ml of hexane. The solution was washed with 200 ml of a 5 weight % aqueous solution of sodium hydroxide 10 times and then with 200 ml of water 3 times. To the solution, 100 g of an ion exchange resin was added and the mixture was stirred for 3 hours. After removing the ion exchange resin by filtration, hexane was removed under reduced pressure by using a rotary evaporator. The yield of the polymer of ethyl vinyl ether was 235 g. NMR and IR of the product were measured and it was proved that one of the end structures of the polymer was (A) and the other was a mixture of (B) and (C):

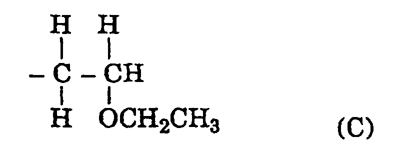

Kinematic viscosity, compatibility with Flon, volume intrinsic resistance and stability to hydrolysis of the polymer of ethyl vinyl ether were measured. The results are shown in Table 1.

### Example 4

Into a 5 liter glass flask equipped with a dropping funnel, a cooler and a stirrer, 1000 g of toluene, 500 g of acetaldehyde diethyl acetal and 5.0 g of boron trifluoride diethyl etherate were charged. Into a dropping funnel, 2500 g of ethyl vinyl ether was charged and dropped in 2 hours and 30 minutes. During this period, the reaction started and the temperature of the reaction liquid increased. The temperature was kept at about 25°C by cooling with an ice water bath. After finishing the dropping, the solution was further stirred for 5 minutes. The reaction mixture was transferred to a washing vessel and washed with 1000 ml of a 5 weight % aqueous solution of sodium hydroxide 3 times and then with 1000 ml of water 3 times. The solvent and unreacted raw materials were removed under reduced pressure by using a rotary evaporator to obtain 2833 g of the crude product.

Into an autoclave, 600 g of the crude product, 600 g of hexane, 60 g of Raney nickel and 60 g of zeolite were charged. Hydrogen was introduced into the autoclave and the pressure of hydrogen was adjusted to 20 kg/cm². After stirring for about 30 seconds, the pressure was released. Hydrogen was introduced into the autoclave again to adjust the pressure of hydrogen to 20 kg/cm² and, after stirring for about 30 seconds, the pressure of hydrogen was released. After repeating this operation once more, the pressure of hydrogen was increased to 50 kg/cm² and the temperature was increased to 130°C in 30 minutes under stirring. The reaction was conducted at 130°C for 1 hour. The reaction proceeded during and after the increase in the temperature and decrease in the pressure was observed. The increase in the pressure with increase in the temperature and the decrease in the pressure by the reaction were suitably compensated by decreasing or increasing the pressure, and the pressure of hydrogen was kept at 50 kg/cm² during the reaction. After finishing the reaction, the reaction mixture was cooled to room temperature and the pressure was decreased to atmospheric pressure. The catalyst was precipitated by standing for 1 hour and the reaction liquid was separated by decantation. The catalyst was washed with 100 ml of hexane twice. The washing liquid was combined with the reaction liquid and filtered with a filter paper. The combined liquid was then transferred to a washing vessel and washed with 500 ml of a 5 weight % aqueous solution of sodium hydroxide 3 times and then with 500 ml of distilled water 5 times. Hexane, water and the like were removed under reduced pressure by using a rotary evaporator. The yield was 468 g.

NMR and IR of the product were measured and it was confirmed that one of the end structures of the polymer was (A) and the other was a mixture of (C) and (D), in which (C) was the major structure and (D) was the minor structure.

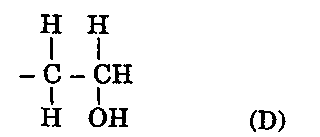

Kinematic viscosity, compatibility with Flon, volume intrinsic resistance and stability to hydrolysis of the polymer of ethyl vinyl ether were measured. The results are shown in Table 1.

### Example 5

By using the catalyst recovered by decantation in Example 4, the reaction was conducted with 600 g of the crude product prepared in Example 4 by the same method as in Example 4. The yield was 501 g. It was shown that the catalyst can be recycled further.

NMR and IR of the product were measured and it was confirmed that one of the end structures of the polymer was (A) and the other was a mixture of (C) and (D), in which (C) was the major structure and (D) was the minor structure.

Kinematic viscosity, compatibility with Flon, volume intrinsic resistance and stability to hydrolysis of the polymer of ethyl vinyl ether were measured. The results are shown in Table 1.

### Example 6

Into a 1 liter glass flask equipped with a dropping funnel, a cooler and a stirrer, 250 g of toluene, 36.82 g of isopropyl alcohol and 4.35 g of boron trifluoride diethyl etherate were charged. Into a dropping funnel, 500 g of isopropyl vinyl ether was charged and dropped in 30 minutes. During this period, the reaction started and the temperature of the reaction solution increased. The temperature was kept at about 30°C by cooling with an ice water bath. After finishing the dropping, the solution was further stirred for 5 minutes. The reaction mixture was transferred to a washing vessel and washed with 130 ml of a 3 weight % aqueous solution of sodium hydroxide 3 times and then with 200 ml of water 3 times. The solvent and unreacted raw materials were removed under reduced pressure by using a rotary evaporator to obtain 475.3 g of the crude product.

Into an autoclave, 380 g of the crude product, 100 g of hexane, 45 g of Raney nickel and 45 g of zeolite were charged. Hydrogen was introduced into the autoclave and the pressure of hydrogen was adjusted to 20 kg/cm². After stirring for about 30 seconds, the pressure was released. Hydrogen was introduced into the autoclave again to make the pressure of hydrogen 20 kg/cm² and, after stirring for about 30 seconds, the pressure of hydrogen was released. After repeating this operation once more, the pressure of hydrogen was increased to 50 kg/cm² and the temperature was increased to 130°C in 30 minutes under stirring. The reaction was conducted at 130°C for 1 hour. The reaction proceeded during and after the increase in the temperature and decrease in the pressure was observed. The increase in the pressure with increase in the temperature and the decrease in pressure with reaction were suitably compensated by decreasing or increasing the pressure and the pressure of hydrogen was kept at 50 kg/cm² during the reaction. After finishing the reaction, the reaction mixture was cooled to room temperature and the pressure was decreased to atmospheric pressure. The catalyst was precipitated by standing for 1 hour and the reaction liquid was separated by decantation. The catalyst was washed with 100 ml of hexane twice. The washing liquid was combined with the reaction liquid and filtered with a filter paper. The catalyst could be used by recycling further. The combined liquid was then transferred to a washing vessel and washed with 200 ml of a 5 weight % aqueous solution of sodium hydroxide 3 times and then with 200 ml of distilled water 5 times. Hexane, water and the like were removed under reduced pressure by using a rotary evaporator. The yield was 287 g.

NMR and IR of the product were measured and it was confirmed that one of the end structures of the polymer was (E) and the other was a mixture of (F) and (D), in which (F) was the major structure and (D) was the minor structure.

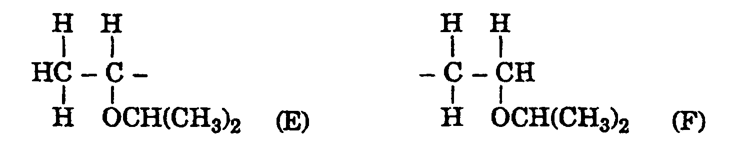

Kinematic viscosity, compatibility with Flon, volume intrinsic resistance and stability to hydrolysis of the polymer of isopropyl vinyl ether were measured. The results are shown in Table 1.

### Example 7

Into a 1 liter autoclave, 200 g of toluene, 5.5 g of methanol and 1.2 g of boron trifluoride diethyl etherate were charged and the atmosphere inside of the autoclave was replaced with nitrogen. While the content of the autoclave was stirred, 200 g of methyl vinyl ether was pressurized therein in 4 hours. During this period, the reaction started and the generation of heat was observed. The reaction was conducted while the temperature inside of the autoclave was kept at about 5°C by cooling the autoclave with an ice water bath. After finishing the addition of methyl vinyl ether under pressure, the solution was further stirred for 5 minutes. The reaction mixture was transferred to a washing vessel and washed with 100 ml of a 5 weight % aqueous solution of sodium hydroxide 3 times and then with 100 ml of water 3 times. The solvent and unreacted raw materials were removed under reduced pressure by using a rotary evaporator to obtain 165 g of the crude product.

Into an autoclave, 165 g of the crude product, 200 g of hexane, 15 g of Raney nickel and 15 g of zeolite were charged. Hydrogen was introduced into the autoclave and the pressure of hydrogen was adjusted to 20 kg/cm². After stirring for about 30 seconds, the pressure was released. Hydrogen was introduced into the autoclave again to make the pressure of hydrogen 20 kg/cm² and, after stirring for about 30 seconds, the pressure of hydrogen was released. After repeating this operation once more, the pressure of hydrogen was increased to 50 kg/cm² and the temperature was increased to 130°C in 30 minutes under stirring. The reaction was conducted at 130°C for 1 hour. The reaction proceeded during and after the increase in the temperature and decrease in the pressure was observed. The increase in the pressure with increase in the temperature and the decrease in the pressure with reaction were suitably compensated by decreasing or increasing the pressure and the pressure of hydrogen was kept at 50 kg/cm² during the reaction. After finishing the reaction, the reaction mixture was cooled to room temperature and the pressure was decreased to atmospheric pressure. The catalyst was precipitated by standing for 1 hour and the reaction liquid was separated by decantation. The catalyst was washed with 50 ml of hexane twice. The washing liquid was combined with the reaction liquid and filtered with a filter paper. Hexane was removed under reduced pressure by using a rotary evaporator and 300 g of chloroform was added. The liquid was then transferred to a washing vessel and washed with 100 ml of a 5 weight % aqueous solution of sodium hydroxide 3 times and then with 100 ml of distilled water 5 times. Chloroform, water and the like were removed under reduced pressure by using a rotary evaporator. The yield was 150 g.

NMR and IR of the product were measured and it was confirmed that one of the end structures of the polymer was (G) and the other was a mixture of (B) and (D), in which (B) was the major structure and (D) was the minor structure.

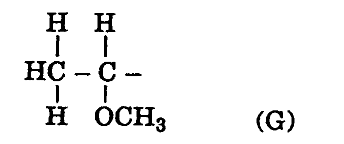

Kinematic viscosity, compatibility with Flon, volume intrinsic resistance and stability to hydrolysis of the polymer of methyl vinyl ether were measured. The results are shown in Table 1.

### Example 7A

Into a 5 liter glass flask equipped with a dropping funnel, a cooler and a stirrer, 1000 g of toluene, 500 g of acetaldehyde diethyl acetal and 5.0 g of boron trifluoride diethyl etherate were charged. Into a dropping funnel, 2500 g of ethyl vinyl ether was charged and dropped in 2 hours and 30 minutes. During this period, the reaction started and the temperature of the reaction solution increased. The temperature was kept at about 25°C by cooling with an ice water bath. After finishing the dropping, the solution was further stirred for 5 minutes. The reaction mixture was transferred to a washing vessel and washed with 1000 ml of a 5 weight % aqueous solution of sodium hydroxide 3 time and then with 1000 ml of water 3 times. The solvent and unreacted materials were removed under reduced pressure by using a rotary evaporator to obtain 2833 g of the crude product.

As the results of NMR and IR of the product, one of the end structures of the polymer was (A) and the other was a mixture of (H) and (I) in which the ratio (H)/(I) was 1/4.5.

- CH = CHOCH₂CH₃ (H)

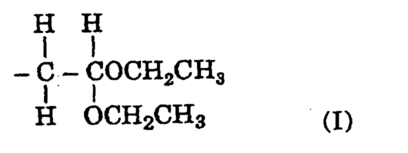

### Example 7B

Into a 2 liter autoclave made of SUS-316L, 700 g of the polymer of ethyl vinyl ether obtained in Example 7A, 35 g of Raney nickel, 35 g of zeolite and 1.5 g of water were charged. Hydrogen was introduced into the autoclave and the pressure of hydrogen was adjusted to 10 kg/cm². After stirring for about 30 seconds, the pressure was released. Hydrogen was introduced into the autoclave again to make the pressure of hydrogen 10 kg/cm² and, after stirring for about 30 seconds, the pressure of hydrogen was released. After repeating this operation once more, the pressure of hydrogen was increased to 35 kg/cm² and the temperature was increased to 140°C in 30 minutes under stirring. The reaction was conducted at 140°C for 2 hours. The reaction proceeded during and after the increase in the temperature and decrease in the pressure was observed. The increase in the pressure with increase in the temperature and the decrease in the pressure with reaction were suitably compensated by decreasing or increasing the pressure and the pressure of hydrogen was kept at 35 kg/cm² during the reaction. After finishing the reaction, the reaction mixture was cooled to room temperature and the pressure was decreased to atmospheric pressure. The reaction product was filtered with a filter paper. The reaction product was then transferred to a 2 liter washing vessel, diluted with 300 g of hexane and washed with 250 ml of a 5 weight % aqueous solution of sodium hydroxide 3 times and then with 250 ml of distilled water 5 times. Hexane, water and the like were removed under reduced pressure by using a rotary evaporator. The yield was 550 g. As the results of NMR and IR of the product, one of the end structures of the polymer was (A) and the other was a mixture of (C) and (D).

### Example 7C

Into a 500 ml glass flask equipped with a dropping funnel, a cooler and a stirrer, 50 g of toluene, 17.7 g of acetaldehyde diethyl acetal and 1.5 g of boron trifluoride diethyl etherate were charged. Into a dropping funnel, 43 g of ethyl vinyl ether and 65 g of isopropyl vinyl ether were charged and dropped in 50 minutes. The temperature of the reaction solution increased by the heat of reaction and the temperature was kept at about 30°C by cooling with an ice water bath. After finishing the dropping, the solution was further stirred for 5 minutes. The reaction mixture was transferred to a washing vessel and washed with 100 ml of a 5 weight % aqueous solution of sodium hydroxide 3 times and then with 150 ml of water 3 times. The solvent and unreacted raw materials were removed under reduced pressure by using a rotary evaporator to obtain 120 g of the crude product. The crude product had the kinematic viscosity of 48.8 cSt at 40°C. As the results of NMR and IR of the product, one of the end structures of the polymer was a mixture of (A) and (E), and the other was a mixture of (H), (I) with structures in which the oxyethyl groups in (H) and (I) were replaced by an oxyisopropyl group, respectively.

### Example 7D

Into a 1 liter autoclave made of SUS-316L, 110 g of the copolymer of ethyl vinyl ether and isopropyl vinyl ether obtained in Example 7C, 300 g of hexane, 5.5 g of Raney nickel and 5.5 g of zeolite were charged. Hydrogen was introduced into the autoclave and the pressure of hydrogen was adjusted to 20 kg/cm². After stirring for about 30 seconds, the pressure was released. Hydrogen was introduced into the autoclave again to make the pressure of hydrogen 20 kg/cm² and, after stirring for about 30 seconds, the pressure of hydrogen was released. After repeating this operation once more, the pressure of hydrogen was increased to 50 kg/cm² and the temperature was increased to 140°C in 30 minutes under stirring. The reaction was conducted at 140°C for 2 hours. The reaction proceeded during and after increase in the temperature and decrease in the pressure was observed. The increase in the pressure with increase in the temperature and the decrease in the pressure with reaction were suitably compensated by decreasing or increasing the pressure and the pressure of hydrogen was kept at 50 kg/cm² during the reaction. After finishing the reaction, the reaction mixture was cooled to room temperature and the pressure was decreased to atmospheric pressure. The reaction product was filtered with a filter paper. The reaction product was then transferred to a washing vessel and washed with 100 ml of a 5 weight % aqueous solution of sodium hydroxide 3 times and then with 150 ml of distilled water 5 times. Hexane, water and the like were removed under reduced pressure by using a rotary evaporator. The yield was 97 g. As the results of NMR and IR of the product, one of the end structures of the polymer was a mixture of (A) and (E) and the other was a mixture of (C), (F) and (D), in which (C) and (F) were the major structures and (D) was the minor structure.

### Example 7E

Into a 500 ml glass flask equipped with a dropping funnel, a cooler and a stirrer, 80 g of toluene, 40 g of propionaldehyde diethyl acetal and 0.4 g of boron trifluoride diethyl etherate were charged. Into a dropping funnel, 116 g of 1-ethoxy-1-propene was charged and dropped in 60 minutes. The temperature of the reaction solution increased by the heat of reaction and the temperature was kept at about 30°C by cooling with an ice water bath. After finishing the dropping, the solution was further stirred for 40 minutes. The reaction mixture was transferred to a washing vessel and washed with 150 ml of a 5 weight % aqueous solution of sodium hydroxide 3 times and then with 200 ml of water 3 times. The solvent and unreacted raw materials were removed under reduced pressure by using a rotary evaporator to obtain 140 g of the crude product. The crude product had a kinematic viscosity of 34.4 cSt at 40°C.

Into a 1 liter autoclave made of SUS-316L, 120 g of the crude product, 300 g of hexane, 6 g of Raney nickel and 6 g of zeolite were charged. Hydrogen was introduced into the autoclave and the pressure of hydrogen was adjusted to 20 kg/cm². After stirring for about 30 seconds, the pressure was released. Hydrogen was introduced into the autoclave again to make the pressure of hydrogen 20 kg/cm² and, after stirring for about 30 seconds, the pressure of hydrogen was released. After repeating this operation once more, the pressure of hydrogen was increased to 50 kg/cm² and the temperature was increased to 130°C in 30 minutes under stirring. The reaction was conducted at 130°C for 2 hours. The reaction proceeded during and after the increase in the temperature and decrease in the pressure was observed. The increase in the pressure with increase in the temperature and the decrease in the pressure with the reaction were suitably compensated by decreasing or increasing the pressure and the pressure of hydrogen was kept at 50 kg/cm² during the reaction. After finishing the reaction, the reaction mixture was cooled to room temperature and the pressure was decreased to atmospheric pressure. The catalyst was precipitated by standing for 1 hour and the reaction liquid was separated by decantation. The catalyst was washed with 50 ml of hexane twice. The washing liquid was combined with the reaction liquid and filtered with a filter paper. The combined liquid was then transferred to a 1 liter washing vessel and washed with 150 ml of a 5 weight % aqueous solution of sodium hydroxide 3 times and then with 200 ml of distilled water 5 times. Hexane, water and the like were removed under reduced pressure by using a rotary evaporator. The yield was 95 g. As the results of NMR and IR of the product, one of the end structures of the polymer was (J) and the other was a mixture of (K) and (L), in which (K) was the major structure and (L) was the minor structure.

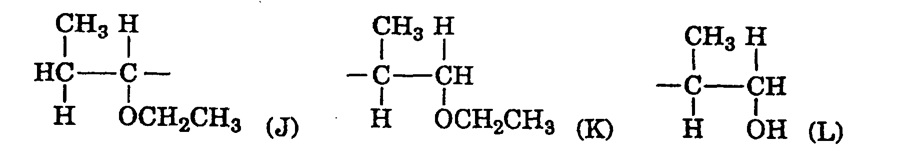

### Example 7F

Into a 5 liter glass flask equipped with a dropping funnel, a cooler and a stirrer, 1000 g of toluene, 304 g of ethanol and 7.8 g of boron trifluoride diethyl etherate were charged. Into a dropping funnel, 3284 g of ethyl vinyl ether was charged and dropped in 4 hours and 30 minutes. During this period, the temperature of the reaction solution increased by the heat of reaction and the temperature was kept at about 25°C by cooling with an ice water bath. After finishing the dropping, the solution was further stirred for 5 minutes. The reaction mixture was transferred to a washing vessel and washed with 1100 ml of a 5 weight % aqueous solution of sodium hydroxide 3 times and then with 1100 ml of water 3 times. The solvent and unreacted raw materials were removed under reduced pressure by using a rotary evaporator to obtain 3397 g of the crude product.

Into a 2 liter autoclave made of SUS-316L, 600 g of the- crude product, 600 g of hexane, 18 g of Raney nickel and 18 g of zeolite were charged. Hydrogen was introduced into the autoclave and the pressure of hydrogen was adjusted to 20 kg/cm². After stirring for about 30 seconds, the pressure was released. Hydrogen was introduced into the autoclave again to make the pressure of hydrogen 20 kg/cm² and, after stirring for about 30 seconds, the pressure of hydrogen was released. After repeating this operation once more, the pressure of hydrogen was increased to 50 kg/cm² and the temperature was increased to 140°C in 30 minutes under stirring. The reaction was conducted at 140°C for 2 hours. The reaction proceeded during and after the increase in the temperature and decrease in the pressure was observed. The increase in the pressure with increase in the temperature and the decrease in the pressure with the reaction were suitably compensated by decreasing or increasing the pressure and the pressure of hydrogen was kept at 50 kg/cm² during the reaction. After finishing the reaction, the reaction mixture was cooled to room temperature and the pressure was decreased to atmospheric pressure. The catalyst was precipitated by standing for 1 hour and the reaction liquid was separated by decantation. The catalyst was washed with 100 ml of hexane twice. The washing liquid was combined with the reaction liquid and filtered with a filter paper. The combined liquid was then transferred to a washing vessel and washed with 500 ml of a 5 weight % aqueous solution of sodium hydroxide 3 times and then with 500 ml of distilled water 5 times. Hexane, water and the like were removed under reduced pressure by using a rotary evaporator. The yield was 492 g. As the results of NMR and IR of the product, one of the end structures of the polymer was (A) and the other was a mixture of (C) and (D), in which (C) was the major structure and (D) was the minor structure.

### Example 7G

Into a 5 liter glass flask equipped with a dropping funnel, a cooler and a stirrer, 1000 g of toluene, 500 g of acetaldehyde diethyl acetal and 5.0 g of boron trifluoride diethyl etherate were charged. Into a dropping funnel, 2700 g of ethyl vinyl ether was charged and dropped in 3 hours. The temperature of the reaction solution increased by the heat of reaction and the temperature was kept at about 25°C by cooling with an ice water bath. After finishing the dropping, the solution was further stirred for 5 minutes. The reaction mixture was transferred to a washing vessel and washed with 1000 ml of a 5 weight % aqueous solution of sodium hydroxide 3 times and then with 1000 ml of water 3 times. The solvent and unreacted raw materials were removed under reduced pressure by using a rotary evaporator to obtain 3040 g of the crude product. The crude product had a kinematic viscosity of 42.1 cSt at 40°C.

Into a 2 liter autoclave made of SUS-316L, 600 g of the crude product, 600 g of hexane, 18 g of Raney nickel and 18 g of zeolite were charged. Hydrogen was introduced into the autoclave and the pressure of hydrogen was adjusted to 20 kg/cm². After stirring for about 30 seconds, the pressure was released. Hydrogen was introduced into the autoclave again to make the pressure of hydrogen 20 kg/cm² and, after stirring for about 30 seconds, the pressure of hydrogen was released. After repeating this operation once more, the pressure of hydrogen was increased to 50 kg/cm² and the temperature was increased to 140°C in 30 minutes under stirring. The reaction was conducted at 140°C for 2 hours. The reaction proceeded during and after the increase in the temperature and decrease in the pressure was observed. The increase in the pressure with increase in the temperature and the decrease in the pressure with the reaction were suitably compensated by decreasing or increasing the pressure and the pressure of hydrogen was kept at 50 kg/cm² during the reaction. After finishing the reaction, the reaction mixture was cooled to room temperature and the pressure was decreased to atmospheric pressure. The catalyst was precipitated by standing for 1 hour and the reaction liquid was separated by decantation. The catalyst was washed with 100 ml of hexane twice. The washing liquid was combined with the reaction liquid and filtered with a filter paper. The combined liquid was then transferred to a washing vessel and washed with 500 ml of a 5 weight % aqueous solution of sodium hydroxide 3 times and then with 500 ml of distilled water 5 times. Hexane, water and the like were removed under reduced pressure by using a rotary evaporator. The yield was 495 g. The polymer had the same end structures as those in Example 4.

### Example 7H

Into a 5 liter glass flask equipped with a dropping funnel, a cooler and a stirrer, 1000 g of toluene, 450 g of acetaldehyde diethyl acetal and 4.5 g of boron trifluoride diethyl etherate were charged. Into a dropping funnel, 3200 g of ethyl vinyl ether was charged and dropped in 4 hours and 10 minutes. The temperature of the reaction solution increased by the heat of reaction and the temperature was kept at about 25°C by cooling with an ice water bath. After finishing the dropping, the solution was further stirred for 5 minutes. The reaction mixture was transferred to a washing vessel and washed with 1000 ml of a 5 weight % aqueous solution of sodium hydroxide 3 times and then with 1000 ml of water 3 times. The solvent and unreacted raw materials were removed under reduced pressure by using a rotary evaporator to obtain 3466 g of the crude product. The crude product had a kinematic viscosity of 76.1 cSt at 40°C.

Into a 2 liter autoclave made of SUS-316L, 600 g of the crude product, 600 g of hexane, 18 g of Raney nickel and 18 g of zeolite were charged. Hydrogen was introduced into the autoclave and the pressure of hydrogen was adjusted to 20 kg/cm². After stirring for about 30 seconds, the pressure was released. Hydrogen was introduced into the autoclave again to make the pressure of hydrogen 20 kg/cm² and, after stirring for about 30 seconds, the pressure of hydrogen was released. After repeating this operation once more, the pressure of hydrogen was increased to 50 kg/cm² and the temperature was increased to 140°C in 30 minutes under stirring. The reaction was conducted at 140°C for 2 hours. The reaction proceeded during and after the increase in the temperature and decrease in the pressure was observed. The increase in the pressure with increase in the temperature and the decrease in the pressure with the reaction were suitably compensated by decreasing or increasing the pressure and the pressure of hydrogen was kept at 50 kg/cm² during the reaction. After finishing the reaction, the reaction mixture was cooled to room temperature and the pressure was decreased to atmospheric pressure. The catalyst was precipitated by standing for 1 hour and the reaction liquid was separated by decantation. The catalyst was washed with 100 ml of hexane twice. The washing liquid was combined with the reaction liquid and filtered with a filter paper. The combined liquid was then transferred to a washing vessel and washed with 500 ml of a 5 weight % aqueous solution of sodium hydroxide 3 times and then with 500 ml of distilled water 5 times. Hexane, water and the like were removed under reduced pressure by using a rotary evaporator. The yield was 498 g. The polymer had the same end structures as those in Example 4.

Kinematic viscosity, compatibility with Flon (Flon 134a), volume intrinsic resistance and stability to hydrolysis of the polymers obtained in Examples 7A to 7H were measured. The results are shown in Table 1A. Compatibility of the polymers obtained in Examples 7G and 7H with Flon 32 was also measured and the results are shown in Table 1B.

### Comparative Example 1

Kinematic viscosity, compatibility with Flon, volume intrinsic resistance and stability to hydrolysis were measured by using a commercial paraffinic mineral oil (VG32). The results are shown in Table 1A.

### Comparative Example 2

Kinematic viscosity, compatibility with Flon, volume intrinsic resistance and stability to hydrolysis were measured by using polypropylene glycol Unilub MB11® (a product of Nippon Yushi Co., Ltd.). The results are shown in Table 1A.

### Comparative Example 3

Into a 5 liter glass flask equipped with a Dean and Stark tube, a cooler and a stirrer, 1091 g of pentaerythritol and 3909 g of n-hexanoic acid were charged and the mixture was heated under stirring. When the temperature of the solution reached to 200°C, the temperature was kept constant for 3 hours. Then the temperature was increased to 220°C and kept at this temperature for 10 hours. During this period, the reaction started and water was formed. After the reaction was finished, the reaction solution was cooled to 150°C and the major part of the unreacted hexanoic acid was recovered under reduced pressure. The remaining solution was transferred to a washing vessel and, after being dissolved in 2 liter of hexane, washed with 1500 ml of a 3 weight % aqueous solution of sodium hydroxide 3 times and with 1500 ml of water 3 times. Further, 800 g of an ion exchange resin was added and the mixture was stirred for 3 hours. The ion exchange resin was removed by filtration and hexane was removed under reduced pressure by using a rotary evaporator. The yield of the polyolester was 3390 g.

Kinematic viscosity, compatibility with Flon, volume intrinsic resistance and stability to hydrolysis of the polymer obtained above were measured. The results are shown in Table 1A.

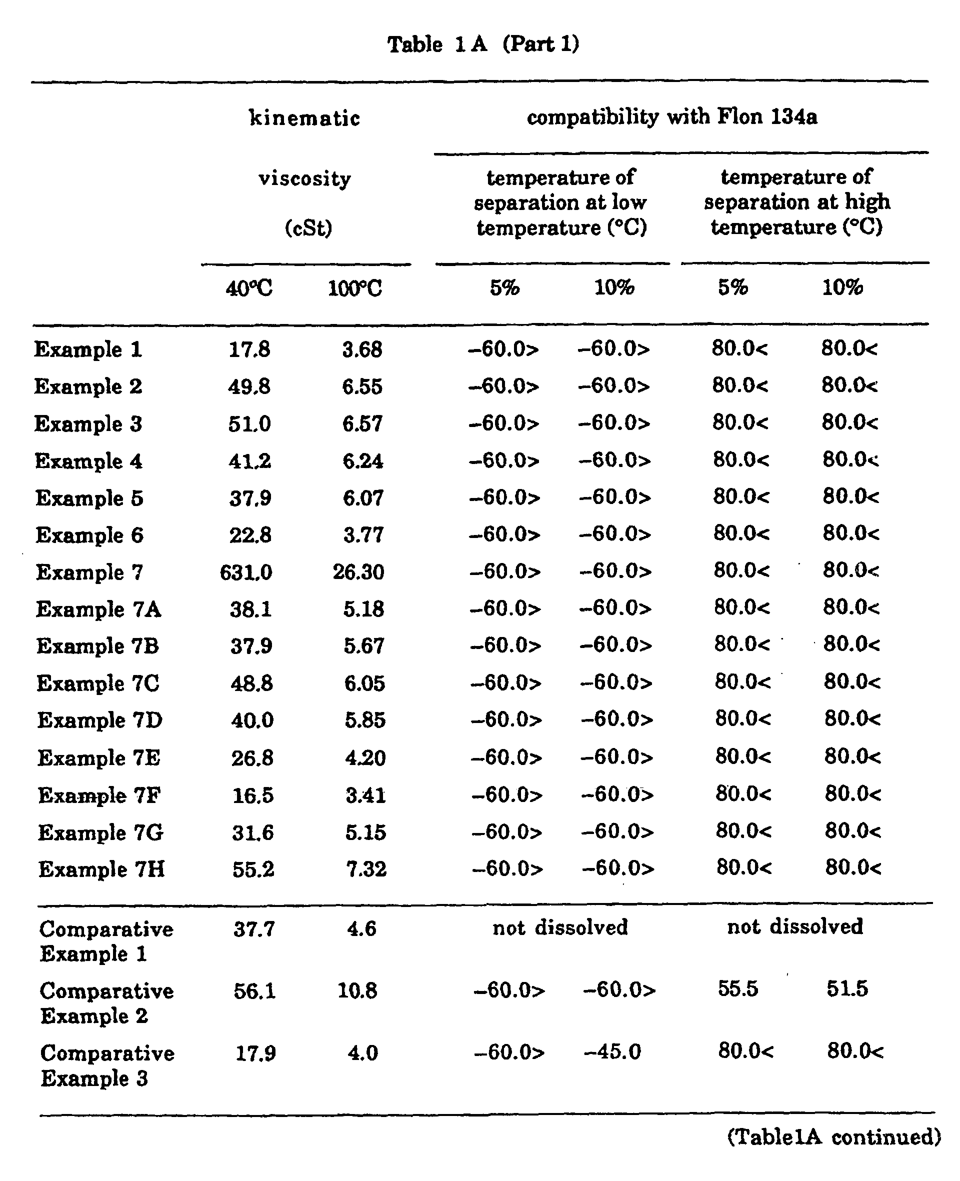

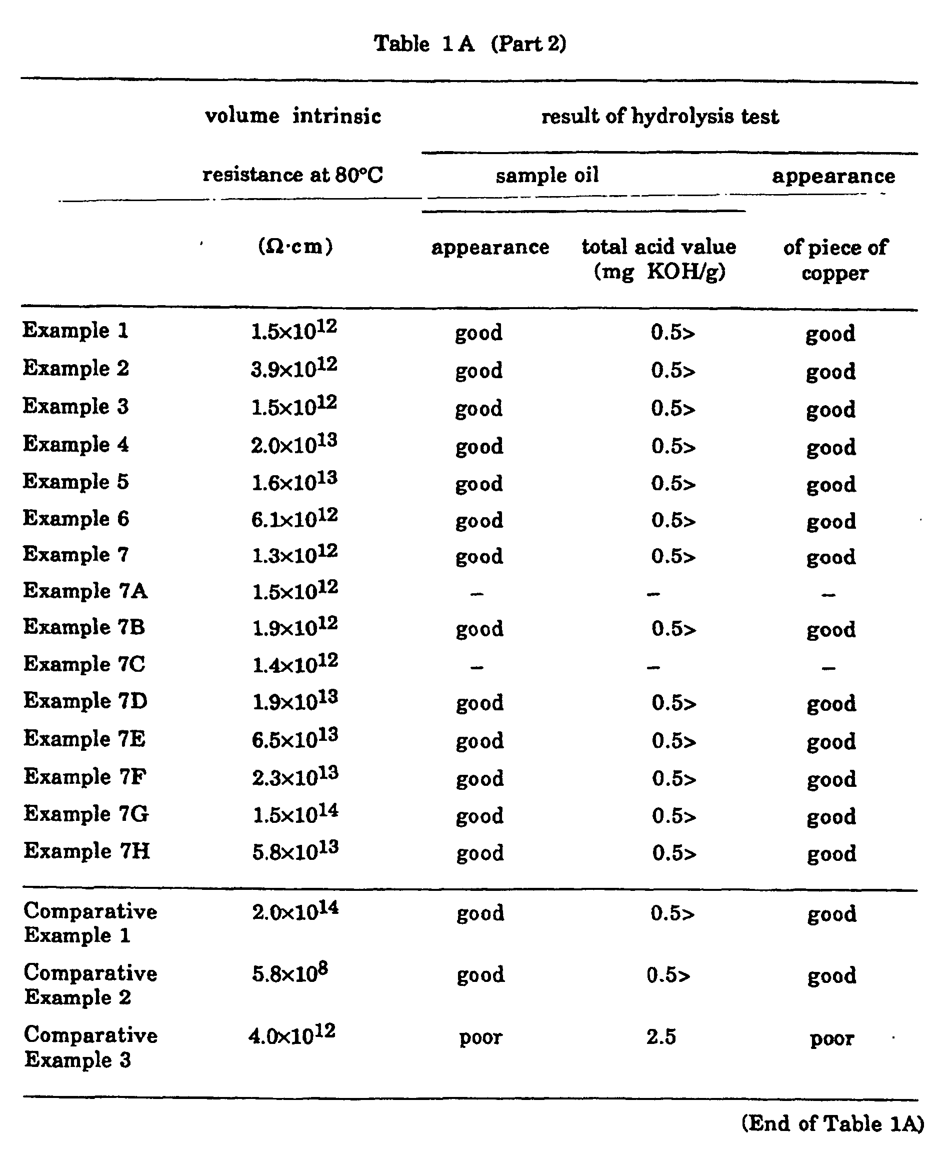

**Table 1B**

| compatibility with Flon 32 | | | | |
|---|---|---|---|---|
| | temperature of separation at low temperature (°C) | | temperature of separation at high temperature (°C) | |
| | Flon/sample (wt. %) | | Flon/sample (wt. %) | |
| | 90/10 | 80/20 | 90/10 | 80/20 |
| Example 7G | -20 | -15 | 40< | 40< |
| Example 7H | -5 | 0 | 40< | 40< |

### Example 8

Into a 5 liter glass flask equipped with a dropping funnel, a cooler and a stirrer, 1000 g of toluene, 234 g of ethanol and 6.0 g of boron trifluoride diethyl etherate were charged. Into the dropping funnel, 2526 g of ethyl vinyl ether was charged and dropped in 3 hours and 20 minutes. During this period, increase in the temperature of the reaction solution by the heat of reaction was observed and the temperature was kept at about 25°C by cooling with an ice water bath. Until the amount of the monomer reached the equivalent amount to ethanol, the monomer was dropped slowly because there was an induction period for the heat generation. After finishing the dropping, the solution was further stirred for 5 minutes. The reaction mixture was transferred to a washing vessel and washed with 870 ml of a 5 weight % aqueous solution of sodium hydroxide 3 times and then with 870 ml of water 3 times. The solvent and unreacted raw materials were removed under reduced pressure by using a rotary evaporator to obtain 2622 g of the polymer of ethyl vinyl ether. The product had light yellow color.

Kinematic viscosity, average molecular weights, dispersion of molecular weight, compatibility with Flon and volume intrinsic resistance of the polymer of ethyl vinyl ether obtained in the above were measured. The results are shown in Table 2.

The infrared absorption spectrum, the ¹³C-NMR chart and the ¹H-NMR chart are shown in Figure 1, Figure 2 and Figure 3, respectively.

In Figure 2, the peaks at 101 ppm, 129 ppm and 134 ppm are peaks assigned to the carbon atom underlined in the following formulae, respectively:

- CH₂ - CH(OC₂H₅)₂

-CH = CH - OC₂H₅

- CH = CH - OC₂H₅.

In Figure 3, the peaks at 4.7 ppm, 5.35 ppm and 5.6 ppm are peaks assigned to the hydrogen atom underlined in the following formulae, respectively:

-CH₂-CH(OC₂H₅)₂

-CH = CH - OC₂H₅

-CH = CH - OC₂H₅.

Therefore, the end of the compound was a mixture of the formula (II) and the formula (III) and the ratio of the numbers of molecule obtained from the ratio of proton was: (II) : (III) = 5.1 : 1.

### Example 9

Into a 5 liter glass flask equipped with a dropping funnel, a cooler and a stirrer, 1000 g of toluene, 195 g of ethanol and 5.0 g of boron trifluoride diethyl etherate were charged. Into a dropping funnel, 3005 g of ethyl vinyl ether was charged and dropped in 3 hours and 50 minutes. During this period, increase in the temperature of the reaction solution by the heat of reaction was observed and the temperature was kept at about 25°C by cooling with an ice water bath. Until the amount of the monomer reached the equivalent amount to ethanol, the monomer was dropped slowly because there was an induction period for the heat generation. After finishing the dropping, the solution was further stirred for 5 minutes. The reaction mixture was transferred to a washing vessel and washed with 1000 ml of a 5 weight % aqueous solution of sodium hydroxide 3 times and then with 1000 ml of water 3 times. The solvent and unreacted raw materials were removed under reduced pressure by using a rotary evaporator to obtain 3008 g of the polymer of ethyl vinyl ether. The product had light yellow color.

Kinematic viscosity, average molecular weights, dispersion of molecular weight, compatibility with Flon and volume intrinsic resistance of the polymer of ethyl vinyl ether obtained in the above were measured. The results are shown in Table 2.

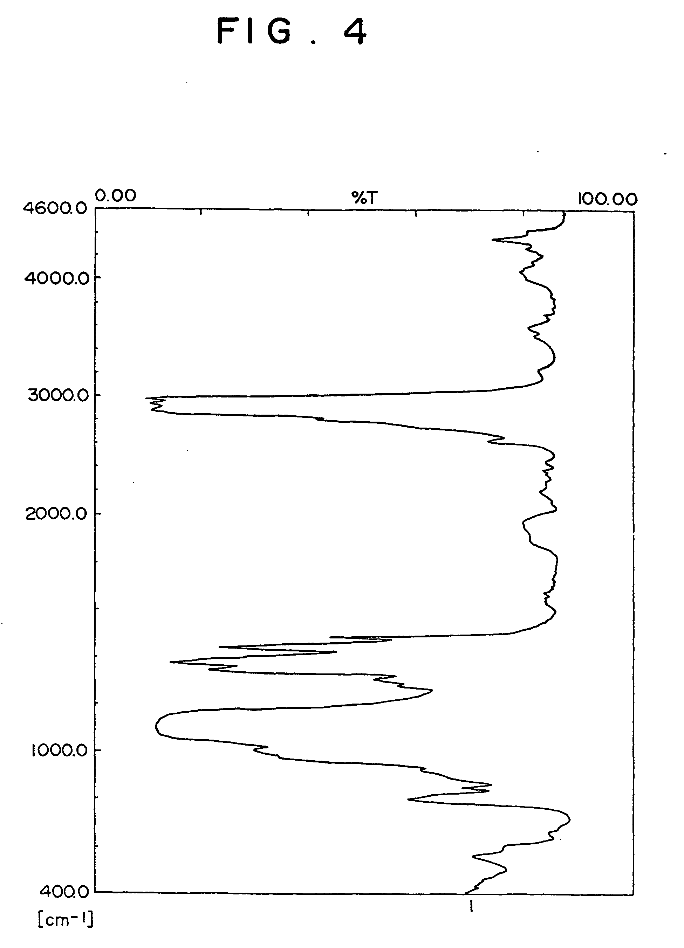

The infrared absorption spectrum, the ¹³C-NMR chart and the ¹H-NMR chart are shown in Figure 4, Figure 5 and Figure 6, respectively. The end structure of the compound was a mixture of the formula (II) and the formula (III) and the ratio of the numbers of molecule obtained by the same method as in Example 8 was: (II) : (III) = 3.9 : 1.

### Example 10

Into a 5 liter glass flask equipped with a dropping funnel, a cooler and a stirrer, 1000 g of toluene, 450 g of acetaldehyde diethyl acetal and 4.5 g of boron trifluoride diethyl etherate were charged. Into a dropping funnel, 3200 g of ethyl vinyl ether was charged and dropped in 4 hours and 10 minutes. During this period, increase in the temperature of the reaction solution by the heat of reaction was observed and the temperature was kept at about 25°C by cooling with an ice water bath. After finishing the dropping, the solution was further stirred for 5 minutes. The reaction mixture was transferred to a washing vessel and washed with 1000 ml of a 5 weight % aqueous solution of sodium hydroxide 3 times and then with 1000 ml of water 3 times. The solvent and unreacted raw materials were removed under reduced pressure by using a rotary evaporator to obtain 3466 g of the polymer of ethyl vinyl ether. The product had light yellow color.

Kinematic viscosity, average molecular weights, dispersion of molecular weight, compatibility with Flon and volume intrinsic resistance of the polymer of ethyl vinyl ether obtained in the above were measured. The results are shown in Table 2.

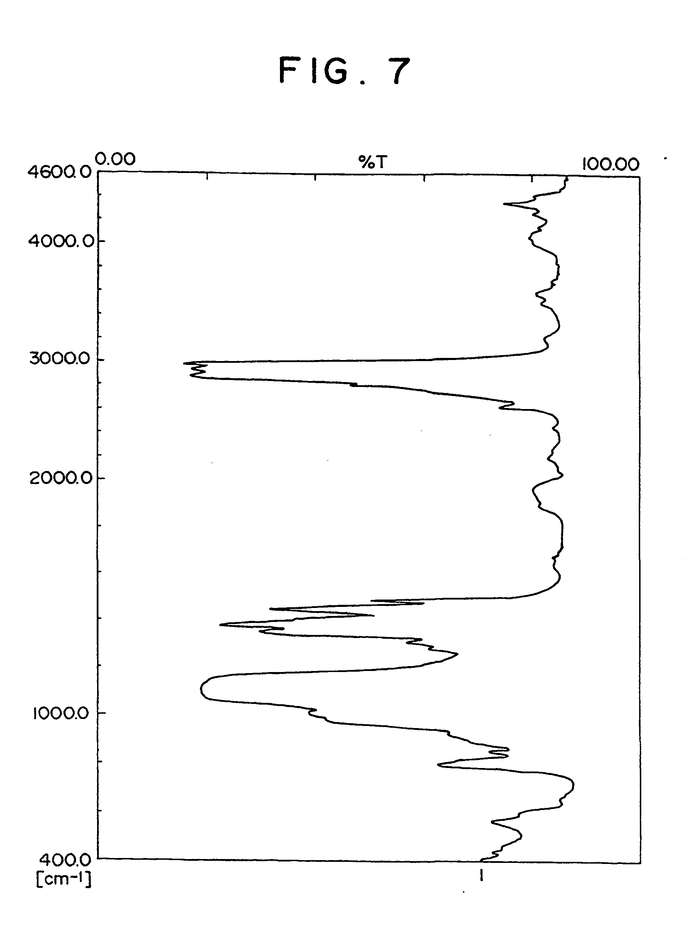

The infrared absorption spectrum is shown in Figure 7.

### Example 11

Into a 5 liter glass flask equipped with a dropping funnel, a cooler and a stirrer, 1000 g of toluene, 195 g of ethanol and 4.5 g of boron trifluoride diethyl etherate were charged. Into a dropping funnel, 3875 g of ethyl vinyl ether was charged and dropped in 4 hours and 40 minutes. During this period, increase in the temperature of the reaction solution by the heat of reaction was observed and the temperature was kept at about 25°C by cooling with an ice water bath. Until the amount of the monomer reached the equivalent amount to ethanol, the monomer was dropped slowly because there was an induction period for the heat generation. After finishing the dropping, the solution was further stirred for 5 minutes. The reaction mixture was transferred to a washing vessel and washed with 1100 ml of a 5 weight % aqueous solution of sodium hydroxide 3 times and then with 1100 ml of water 3 times. The solvent and unreacted raw materials were removed under reduced pressure by using a rotary evaporator to obtain 3867 g of the polymer of ethyl vinyl ether. The product had light yellow color.

Kinematic viscosity, average molecular weights, dispersion of molecular weight, compatibility with Flon and volume intrinsic resistance of the polymer of ethyl vinyl ether obtained in the above were measured. The results are shown in Table 2.

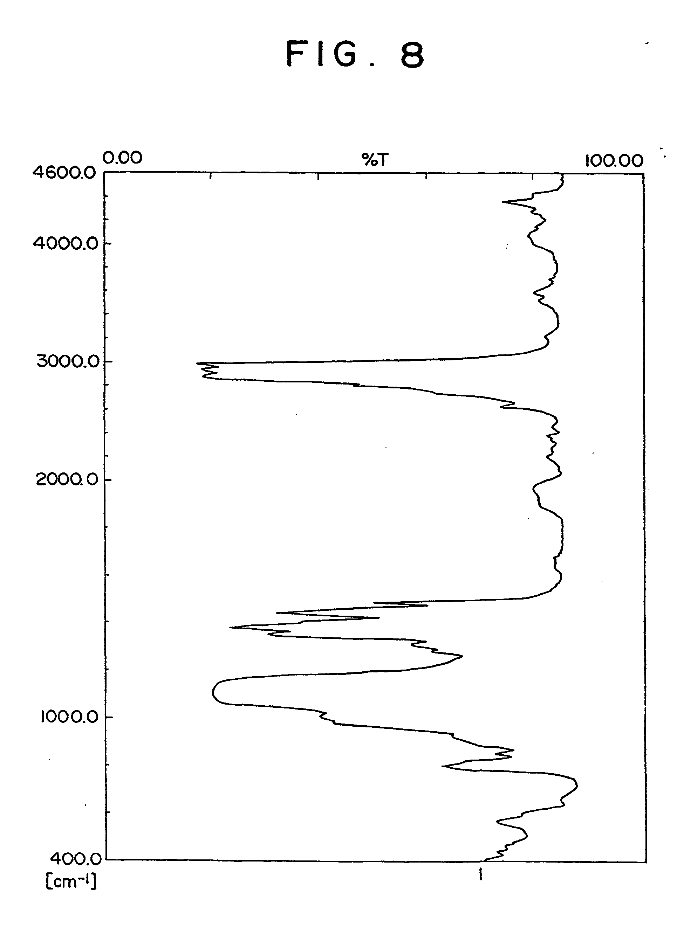

The infrared absorption spectrum, the ¹³C-NMR chart and the ¹H-NMR chart are shown in Figure 8, Figure 9 and Figure 10, respectively.

The ratio of the numbers of molecule of the formula (II) and the formula (III) obtained by the same method as in Example 8 was: (II) : (III) = 2.9 : 1.

### Example 12

Into a 500 ml glass flask equipped with a dropping funnel, a cooler and a stirrer, 125 g of toluene, 19.4 g of isopropyl alcohol and 2.3 g of boron trifluoride diethyl etherate were charged. Into a dropping funnel, 250 g of isopropyl vinyl ether was charged and dropped in 30 minutes. During this period, increase in the temperature of the reaction solution by the heat of reaction was observed and the temperature was kept at about 25°C by cooling with an ice water bath. After finishing the dropping, the solution was further stirred for 5 minutes. The reaction mixture was transferred to a washing vessel and washed with 80 ml of a 5 weight % aqueous solution of sodium hydroxide 3 times and then with 80 ml of water 3 times. The solvent and unreacted raw materials were removed under reduced pressure by using a rotary evaporator to obtain 238 g of the polymer of isopropyl vinyl ether. The product had light yellow color.

Kinematic viscosity, average molecular weights, dispersion of molecular weight, compatibility with Flon and volume intrinsic resistance of the polymer of isopropyl vinyl ether obtained in the above were measured. The results are shown in Table 2.

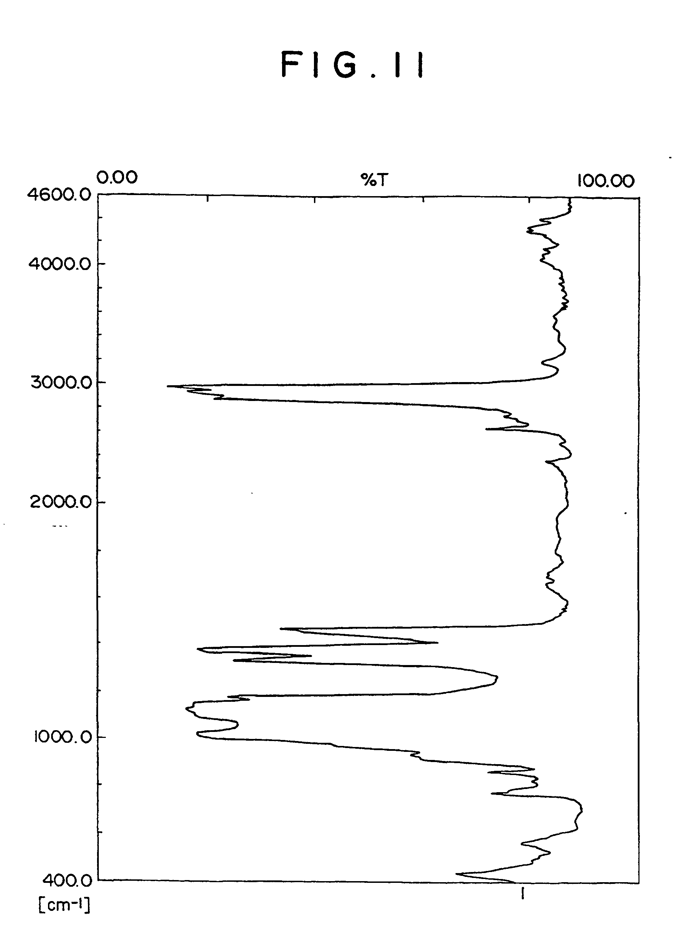

The infrared absorption spectrum is shown in Figure 11.

### Example 13

Into a 500 ml glass flask equipped with a dropping funnel, a cooler and a stirrer, 125 g of toluene, 17.4 g of isopropyl alcohol and 2.1 g of boron trifluoride diethyl etherate were charged. Into a dropping funnel, 250 g of isopropyl vinyl ether was charged and dropped in 30 minutes. During this period, increase in the temperature of the reaction solution by the heat of reaction was observed and the temperature was kept at about 25°C by cooling with an ice water bath. After finishing the dropping, the solution was further stirred for 5 minutes. The reaction mixture was transferred to a washing vessel and washed with 80 ml of a 5 weight % aqueous solution of sodium hydroxide 3 times and then with 80 ml of water 3 times. The solvent and unreacted raw materials were removed under reduced pressure by using a rotary evaporator to obtain 236 g of the polymer of isopropyl vinyl ether. The product had light yellow color.

Kinematic viscosity, average molecular weights, dispersion of molecular weight, compatibility with Flon and volume intrinsic resistance of the polymer of isopropyl vinyl ether obtained in the above were measured. The results are shown in Table 2.

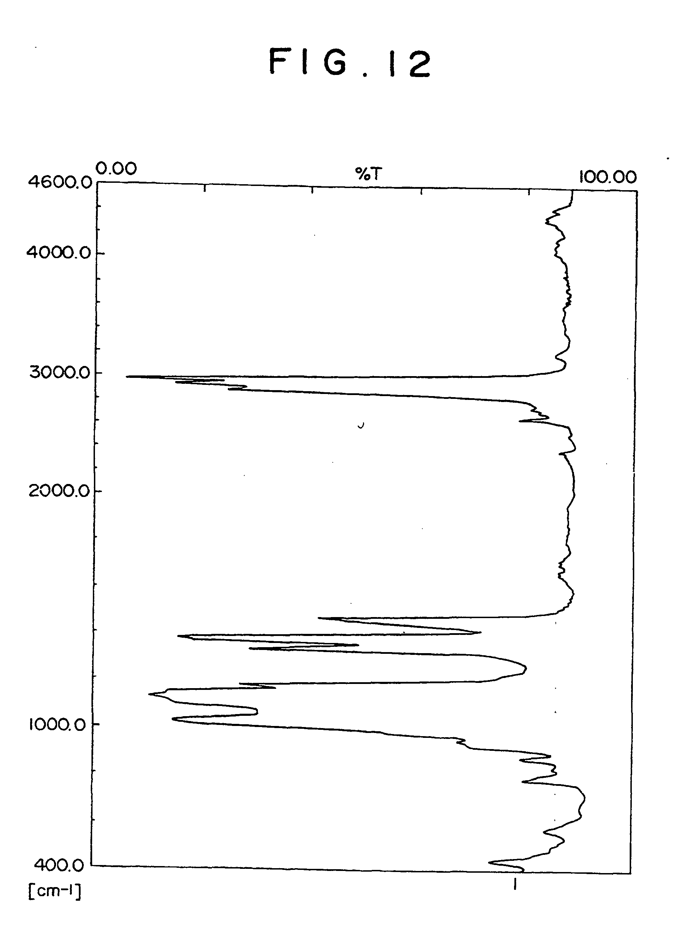

The infrared absorption spectrum, the ¹³C-NMR chart and the ¹H-NMR chart are shown in Figure 12, Figure 13 and Figure 14, respectively.

The ratio of the numbers of molecule of the formula (II) and the formula (III) obtained by the same method as in Example 8 was: (II) : (III) = 3.8 : 1.

### Example 14

Into a 200 ml stainless steel autoclave equipped with a stirrer, 40 g of toluene, 2.5 g of methanol and 0.18 g of boron trifluoride diethyl etherate were charged. After sealing the autoclave, the atmosphere inside of the autoclave was replaced with nitrogen. Into the autoclave, 47 g of methyl vinyl ether was added from a bomb by the pressure of the compound in 5 hours. During this period, increase in the temperature of the reaction solution by the heat of reaction was observed and the temperature was kept at about 25°C by cooling with an ice water bath. After finishing the addition, the solution was further stirred for 5 minutes. The reaction mixture was transferred to a washing vessel and washed with 50 ml of a 5 weight % aqueous solution of sodium hydroxide 3 times and then with 100 ml of water 3 times. The solvent and unreacted raw materials were removed under reduced pressure by using a rotary evaporator to obtain 42 g of the polymer of methyl vinyl ether. The product had light yellow color.

Kinematic viscosity, average molecular weights, dispersion of molecular weight, compatibility with Flon and volume intrinsic resistance of the polymer of methyl vinyl ether obtained in the above were measured. The results are shown in Table 2.

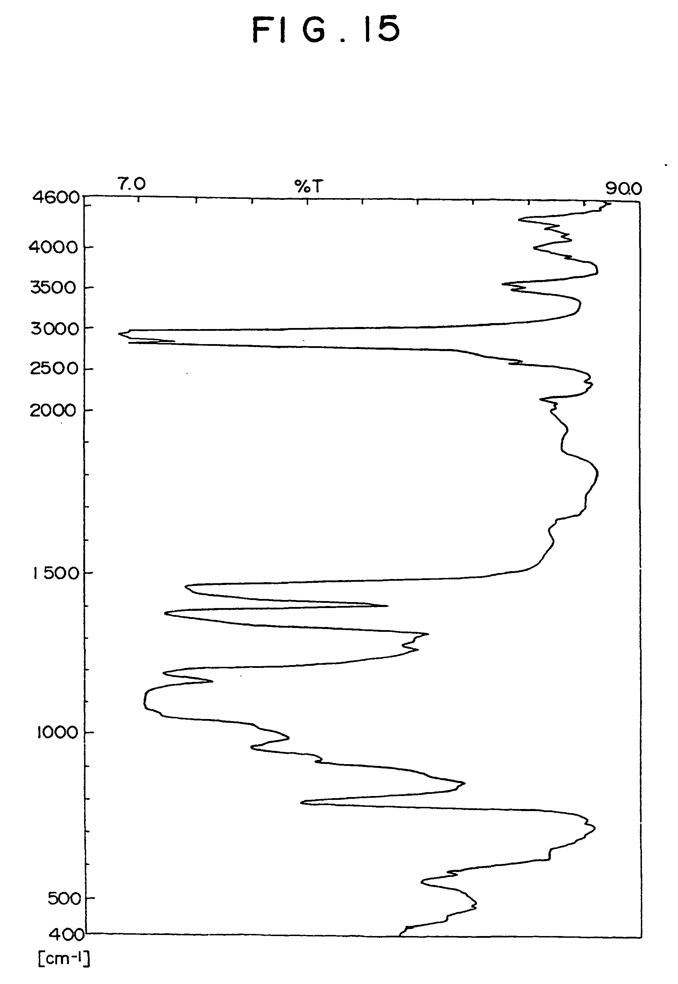

The infrared absorption spectrum, the ¹³C-NMR chart and the ¹H-NMR chart are shown in Figure 15, Figure 16 and Figure 17, respectively. The ratio of the numbers of molecule of the formula (II) and the formula (III) obtained by the same method as in Example 8 was: (II) : (III) = 8.9 : 1.

### Example 15

Average molecular weights and dispersion of molecular weight of the ethyl vinyl ether/isopropyl vinyl ether copolymer obtained in Example 7C described above were measured. The results of the measurements are shown in Table 2 together with the results of measurements of kinematic viscosity, compatibility with Flon and volume intrinsic resistance.

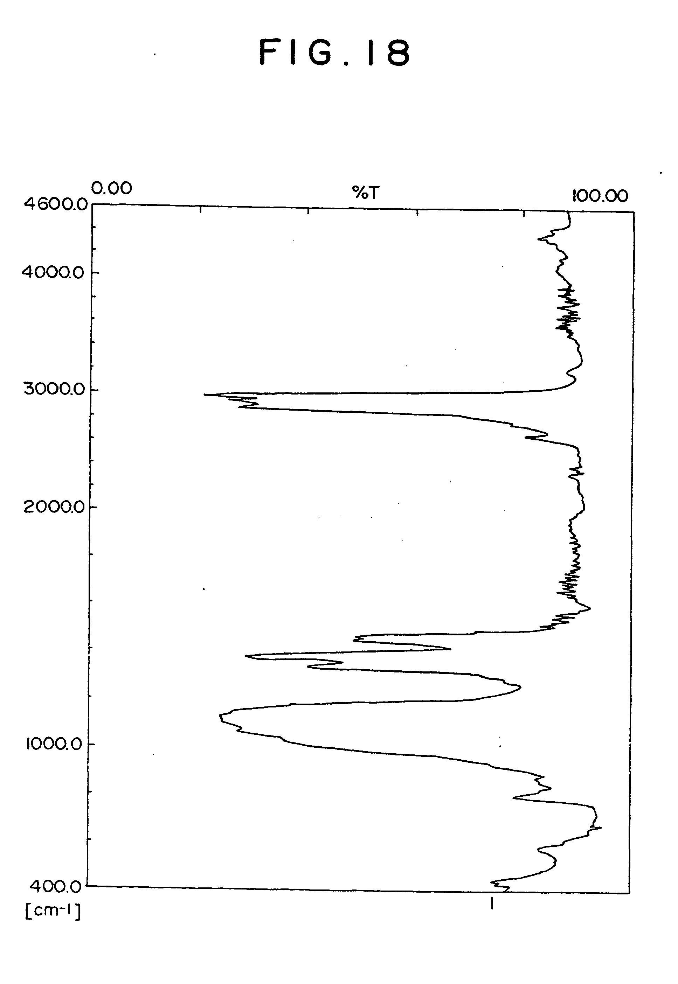

The infrared absorption spectrum, the ¹³C-NMR chart and the ¹H-NMR chart are shown in Figure 18, Figure 19 and Figure 20, respectively.

The ratio of the numbers of molecule of the formula (II) and the formula (III) obtained by the same method as in Example 8 was: (II) : (III) = 3.1 : 1.

### Example 16

Into a 500 ml glass flask equipped with a dropping funnel, a cooler and a stirrer, 50 g of toluene, 4.6 g of ethyl alcohol and 0.2 g of boron trifluoride diethyl etherate were charged. Into a dropping funnel, 86 g of 1-ethoxy-1-propene was charged and dropped in 50 minutes. During this period, increase in the temperature of the reaction solution by the heat of reaction was observed and the temperature was kept at about 30°C by cooling with an ice water bath. After finishing the dropping, the solution was further stirred for 30 minutes. The reaction mixture was transferred to a washing vessel and washed with 100 ml of a 5 weight % aqueous solution of sodium hydroxide 3 times and then with 150 ml of water 3 times. The solvent and unreacted raw materials were removed under reduced pressure by using a rotary evaporator to obtain 84 g of the polymer of 1-ethoxy-1-propene.

Kinematic viscosity, average molecular weights, dispersion of molecular weight, compatibility with Flon and volume intrinsic resistance of the polymer of 1-ethoxy-1-propene obtained in the above were measured. The results are shown in Table 2.

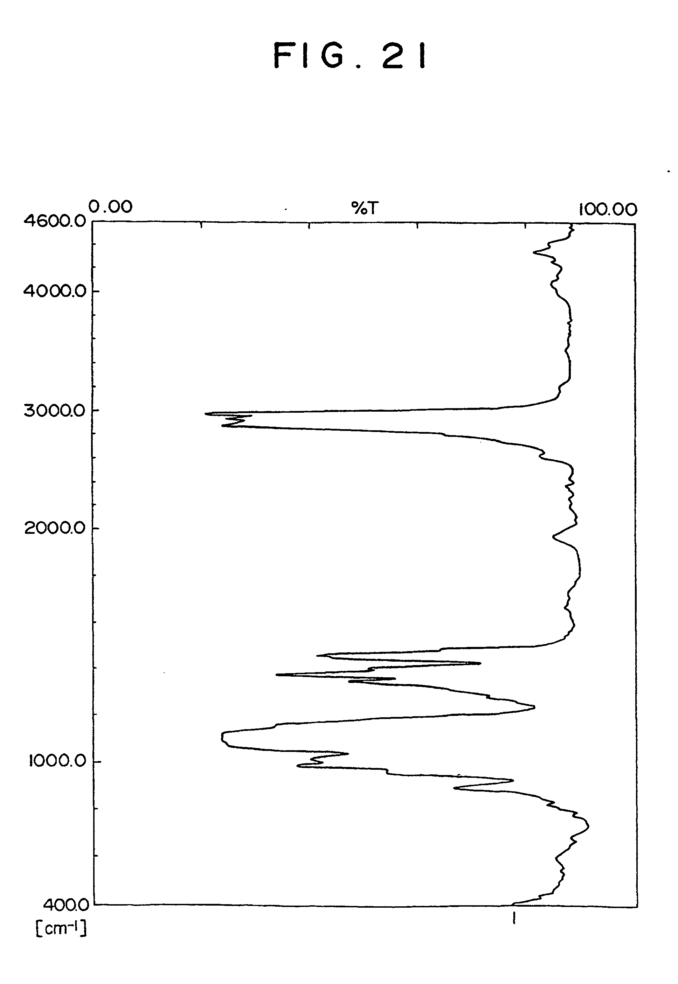

The infrared absorption spectrum, the ¹³C-NMR chart and the ¹H-NMR chart are shown in Figure 21, Figure 22 and Figure 23, respectively.

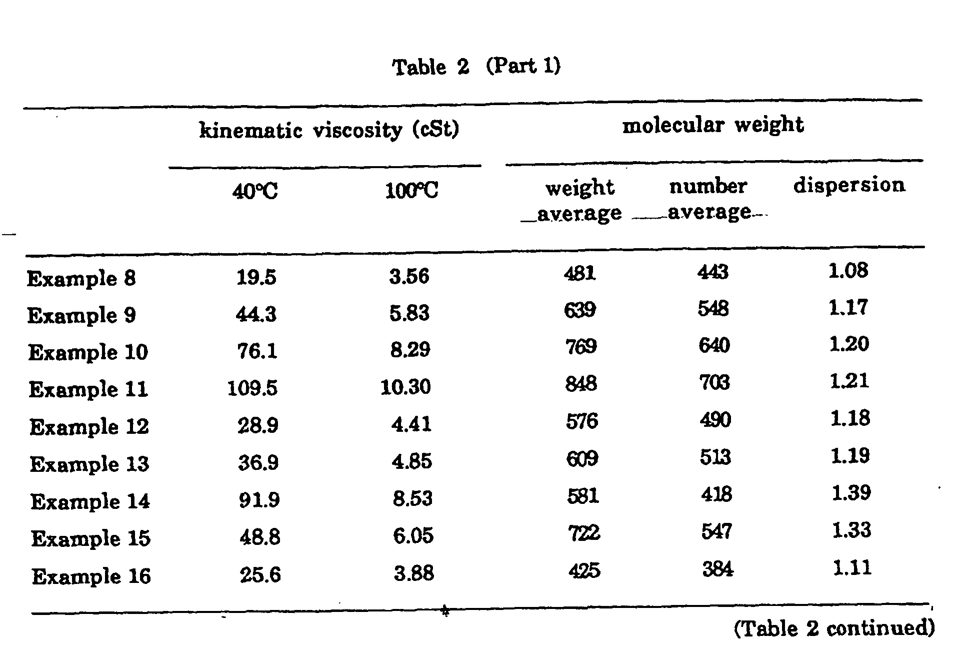

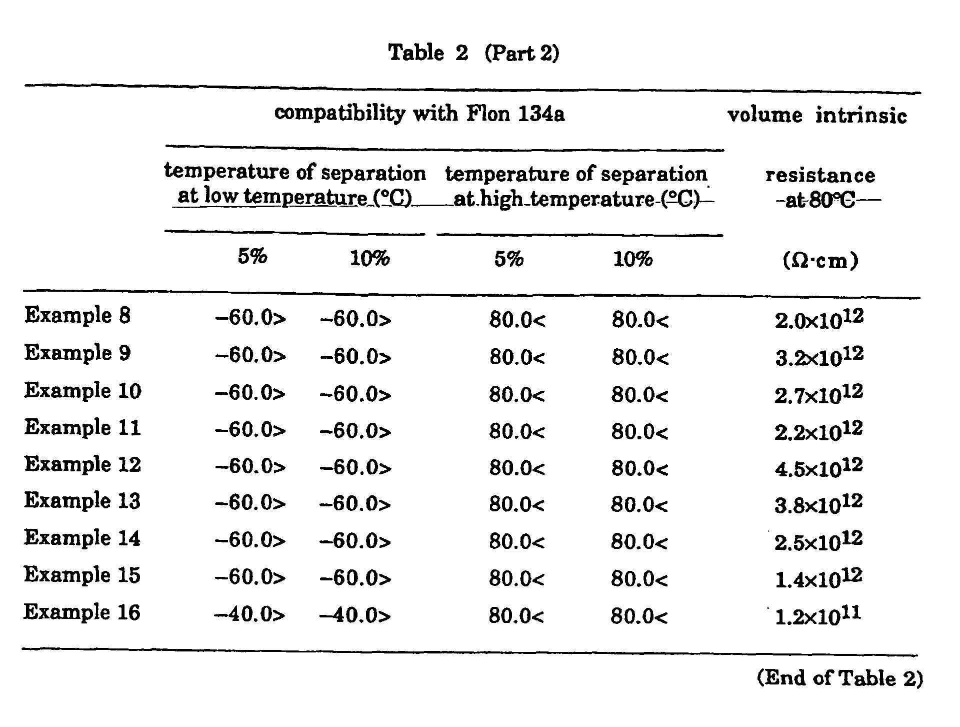

### Example 17

Into a 5 liter glass flask equipped with a dropping funnel, a cooler and a stirrer, 1000 g of toluene, 304 g of ethanol and 7.8 g of boron trifluoride diethyl etherate were charged. Into a dropping funnel, 3284 g of ethyl vinyl ether was charged and dropped in 4 hours and 30 minutes. The temperature of the reaction solution increased by the heat of reaction and the temperature was kept at about 25°C by cooling with an ice water bath. After finishing the dropping, the solution was further stirred for 5 minutes. The reaction mixture was transferred to a washing vessel and washed with 1100 ml of a 5 weight % aqueous solution of sodium hydroxide 3 times and then with 1100 ml of water 3 times. The solvent and unreacted raw materials were removed under reduced pressure by using a rotary evaporator to obtain 3397 g of the crude product. ¹H-NMR and ¹³C-NMR of the crude product were measured. The ¹H-NMR showed peaks at 4.7 ppm, 5.35 ppm and 5.6 ppm and the ¹³C-NMR showed peaks at 101 ppm, 129 ppm and 134 ppm.

The crude product had a kinematic viscosity of 17.8 cSt at 40°C.

By treating the crude product further by the same method as in Example 7F, 492 g of a polymer of ethyl vinyl ether was obtained.

Average molecular weights and dispersion of molecular weight of the ethyl vinyl ether polymer obtained above were measured. The results of the measurements are shown in Table 3 together with the results of measurements of kinematic viscosity, compatibility with Flon and volume intrinsic resistance.

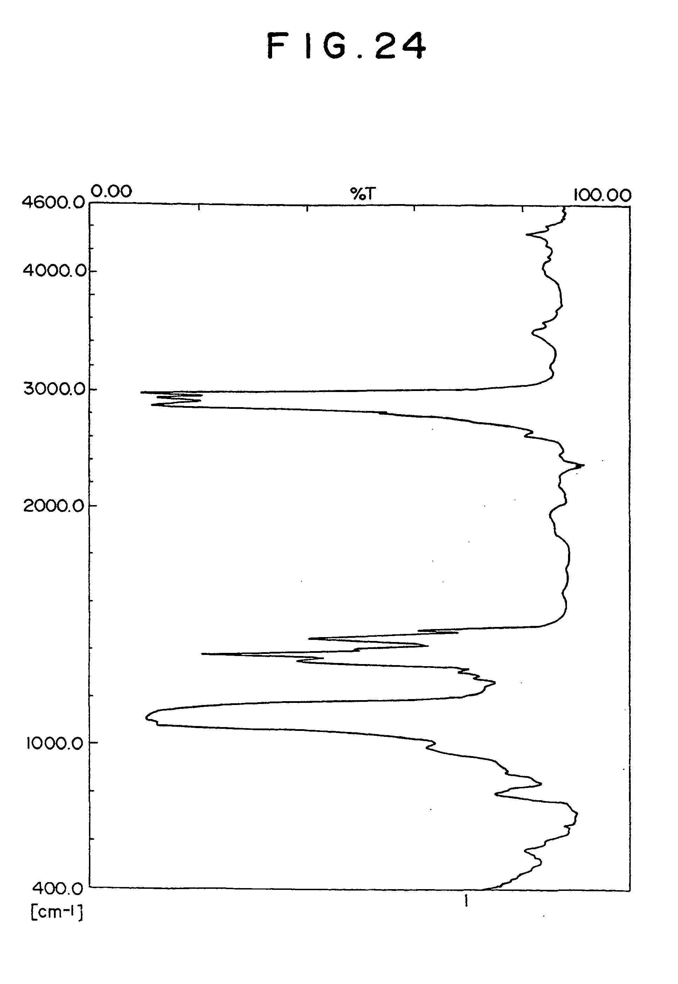

The infrared absorption spectrum, the ¹³C-NMR chart and the ¹H-NMR chart are shown in Figure 24, Figure 25 and Figure 26, respectively.

In the ¹H-NMR chart of this polymer, the peaks at 4.7 ppm, 5.35 ppm and 5.6 ppm observed in the chart of the crude product described above were not found. In the ¹³C-NMR chart of this polymer, the peaks at 101 ppm, 129 ppm and 134 ppm were not found either. Furthermore, the ¹H-NMR peak assigned to the hydrogen of aldehyde is generally found in the area of 9.7 ppm and the ¹³C-NMR peak assigned to the carbon of aldehyde is generally found in the area of 200 ppm. None of these peaks were found in the spectra of the polymer obtained above. From these findings, it can be found that the polymer obtained above did not contain any of an unsaturated bond, an acetal structure and an aldehyde structure.

### Example 18

Average molecular weights and dispersion of molecular weight of the ethyl vinyl ether polymer obtained in Example 7G described above were measured. The results of the measurements are shown in Table 3 together with the results of measurements of kinematic viscosity, compatibility with Flon and volume intrinsic resistance.

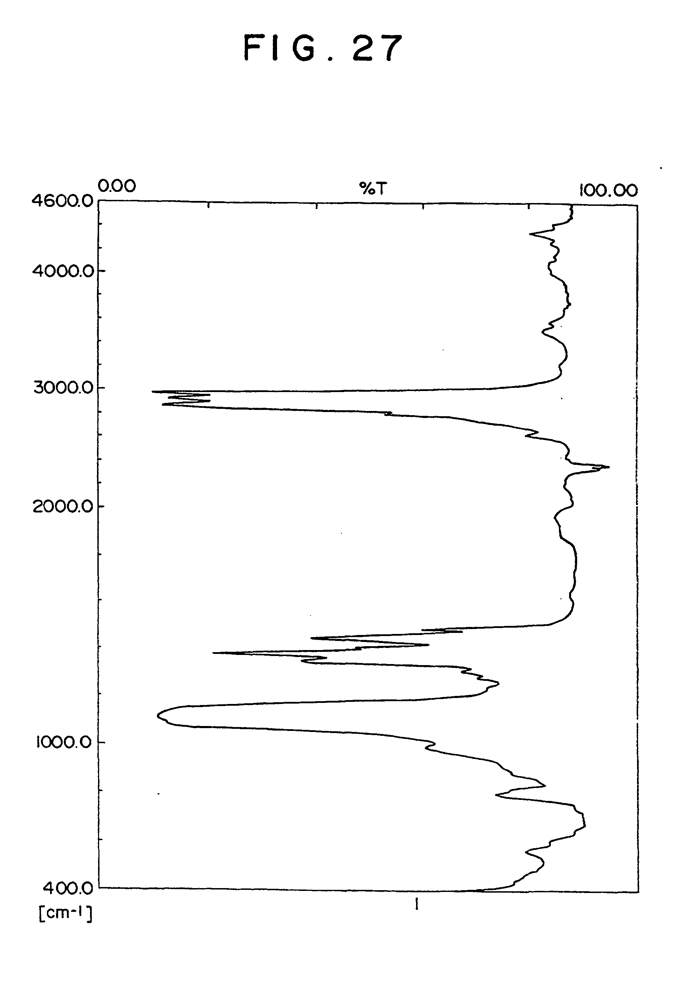

The infrared absorption spectrum, the ¹³C-NMR chart and the ¹H-NMR chart are shown in Figure 27, Figure 28 and Figure 29, respectively.

By the same reason as in Example 17, the polymer obtained above did not contain any of an unsaturated bond, an acetal structure and an aldehyde structure.

### Example 19

Average molecular weights and dispersion of molecular weight of the ethyl vinyl ether polymer obtained in Example 7H described above were measured. The results of the measurements are shown in Table 3 together with the results of measurements of kinematic viscosity, compatibility with Flon and volume intrinsic resistance.

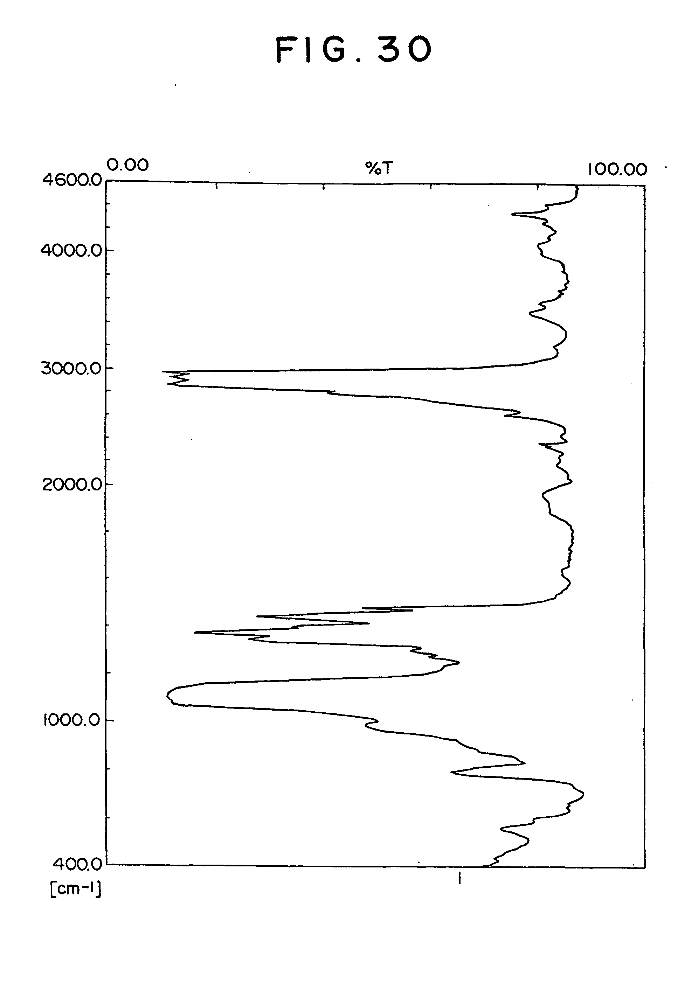

The infrared absorption spectrum, the ¹³C-NMR chart and the ¹H-NMR chart are shown in Figure 30, Figure 31 and Figure 32, respectively.

By the same reason as in Example 17, the polymer obtained above did not contain any of an unsaturated bond, an acetal structure and an aldehyde structure.

### Example 20

Into a 500 ml glass flask equipped with a dropping funnel, a cooler and a stirrer, 95 g of toluene, 14.7 g of isopropyl alcohol and 1.8 g of boron trifluoride diethyl etherate were charged. Into a dropping funnel, 190 g of isopropyl vinyl ether was charged and dropped in 30 minutes. The temperature of the reaction solution increased by the heat of reaction and the temperature was kept at about 25°C by cooling with an ice water bath. After finishing the dropping, the solution was further stirred for 5 minutes. The reaction mixture was transferred to a washing vessel and washed with 70 ml of a 5 weight % aqueous solution of sodium hydroxide 3 times and then with 70 ml of water 3 times. The solvent and unreacted raw materials were removed under reduced pressure by using a rotary evaporator to obtain 179 g of the crude product. The crude product had a kinematic viscosity of 27.0 cSt at 40°C. Into a 1 liter autoclave made of SUS-316L, 171 g of the crude product, 130 g of hexane, 20 g of Raney nickel and 20 g of zeolite were charged. Hydrogen was introduced into the autoclave and the pressure of hydrogen was adjusted to 20 kg/cm². After stirring for about 30 seconds, the pressure was released. Hydrogen was introduced into the autoclave again to make the pressure of hydrogen 20 kg/cm² and, after stirring for about 30 seconds, the pressure of hydrogen was released. After repeating this operation once more, the pressure of hydrogen was increased to 50 kg/cm² and the temperature was increased to 130°C in 30 minutes under stirring. The reaction was conducted at 130°C for 1 hour. The reaction proceeded during and after the increase in the temperature and decrease in the pressure was observed. The increase in the pressure with increase in the temperature and the decrease in the pressure with the reaction were suitably compensated by decreasing or increasing the pressure and the pressure of hydrogen was kept at 50 kg/cm² during the reaction. After finishing the reaction, the reaction mixture was cooled to room temperature and the pressure was decreased to atmospheric pressure. The catalyst was precipitated by standing for 1 hour and the reaction liquid was separated by decantation. The catalyst was washed with 100 ml of hexane twice. The washing liquid was combined with the reaction liquid and filtered with a filter paper. The combined liquid was then transferred to a 2 liter washing vessel and washed with 50 ml of a 5 weight % aqueous solution of sodium hydroxide 3 times and then with 50 ml of distilled water 5 times. Hexane, water and the like were removed under reduced pressure by using a rotary evaporator. The yield was 131 g.

Kinematic viscosity, average molecular weights, dispersion of the molecular weight, compatibility with Flon, volume intrinsic resistance and stability to hydrolysis of the polymer of isopropyl vinyl ether obtained above were measured. The results are shown in Table 3.

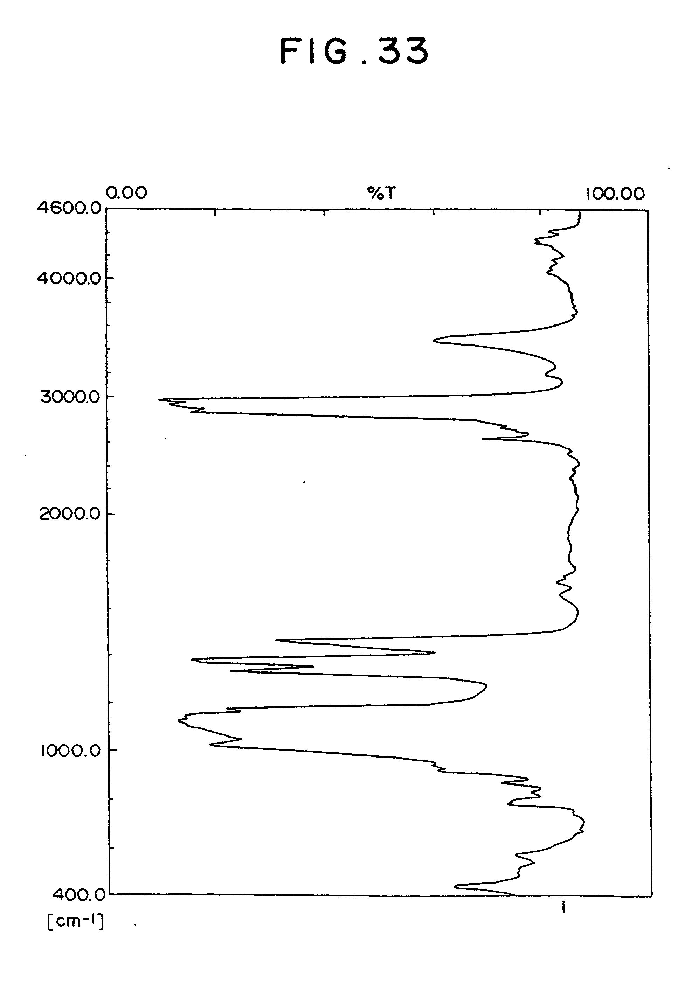

The infrared absorption spectrum, the ¹³C-NMR chart and the ¹H-NMR chart are shown in Figure 33, Figure 34 and Figure 35, respectively.

By the same reason as in Example 17, the polymer obtained above did not contain any of an unsaturated bond, an acetal structure and an aldehyde structure.

### Example 21

Average molecular weights and dispersion of molecular weight of the isopropyl vinyl ether polymer obtained in Example 6 described above were measured. The results of the measurements are shown in Table 3 together with the results of measurements of kinematic viscosity, compatibility with Flon and volume intrinsic resistance.

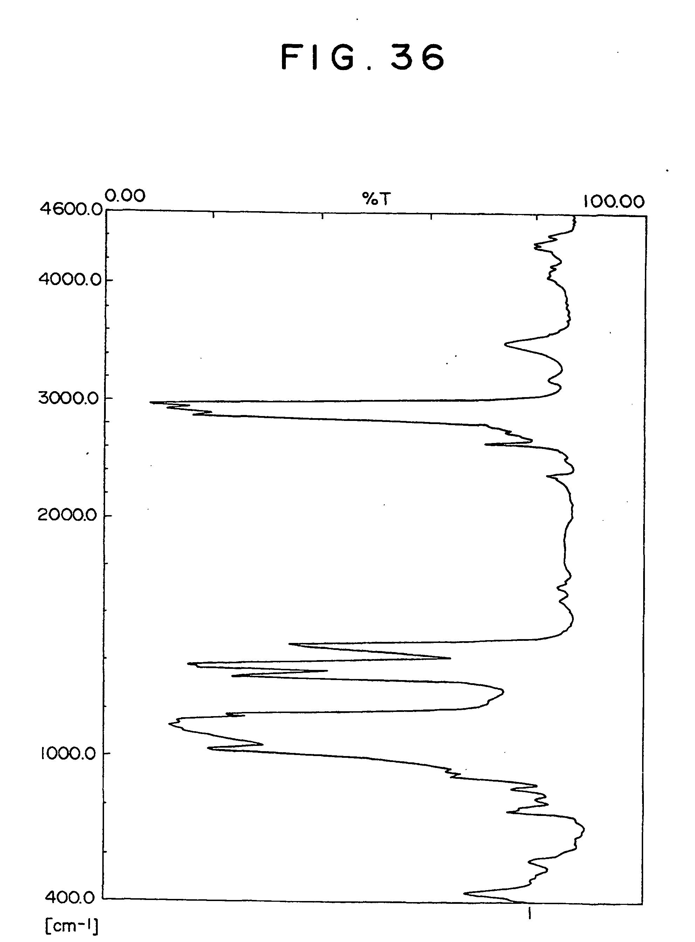

The infrared absorption spectrum is shown in Figure 36.

By the same reason as in Example 17, the polymer obtained above did not contain any of an unsaturated bond, an acetal structure and an aldehyde structure.

### Example 22

Into a 200 ml stainless steel autoclave equipped with a stirrer, 40 g of toluene, 6.4 g of methanol and 0.45 g of boron trifluoride diethyl etherate were charged. The autoclave was tightly closed and the atmosphere inside of the autoclave was replaced with nitrogen. Into the autoclave, 107 g of methyl vinyl ether was added from a bomb by the pressure of the compound in 5 hours. The temperature of the reaction solution increased by the heat of reaction and the temperature was kept at about 25°C by cooling with an ice water bath. After finishing the addition, the solution was further stirred for 10 minutes. The reaction mixture was transferred to a washing vessel and washed with 100 ml of a 5 weight % aqueous solution of sodium hydroxide 3 times and then with 150 ml of water 3 times. The solvent and unreacted raw materials were removed under reduced pressure by using a rotary evaporator to obtain 95 g of the crude product. The crude product had a kinematic viscosity of 56.9 cSt at 40°C.

Into a 1 liter autoclave made of SUS-316L, 90 g of the crude product, 300 g of hexane, 4.5 g of Raney nickel and 4.5 g of zeolite were charged. Hydrogen was introduced into the autoclave and the pressure of hydrogen was adjusted to 20 kg/cm². After stirring for about 30 seconds, the pressure was released. Hydrogen was introduced into the autoclave again to make the pressure of hydrogen 20 kg/cm² and, after stirring for about 30 seconds, the pressure of hydrogen was released. After repeating this operation once more, the pressure of hydrogen was increased to 50 kg/cm² and the temperature was increased to 130°C in 30 minutes under stirring. The reaction was conducted at 130°C for 1 hour. The reaction proceeded during and after the increase in the temperature and decrease in the pressure was observed. The increase in the pressure with increase in the temperature and the decrease in the pressure with the reaction were suitably compensated by decreasing or increasing the pressure and the pressure of hydrogen was kept at 50 kg/cm² during the reaction. After finishing the reaction, the reaction mixture was cooled to room temperature and the pressure was decreased to atmospheric pressure. The catalyst was precipitated by standing for 1 hour and the reaction liquid was separated by decantation. The catalyst was washed with 30 ml of hexane twice. The washing liquid was combined with the reaction liquid and filtered with a filter paper. After hexane was removed under reduced pressure by using a rotary evaporator, 100 ml of toluene was added to the residual product and the product was then transferred to a washing vessel and washed with 100 ml of a 5 weight % aqueous solution of sodium hydroxide 3 times and then with 150 ml of distilled water 5 times. Toluene, water and the like were removed under reduced pressure by using a rotary evaporator. The yield was 80.5 g.

Kinematic viscosity, average molecular weights, dispersion of the molecular weight, compatibility with Flon, volume intrinsic resistance and stability to hydrolysis of the polymer of methyl vinyl ether obtained above were measured. The results are shown in Table 3.

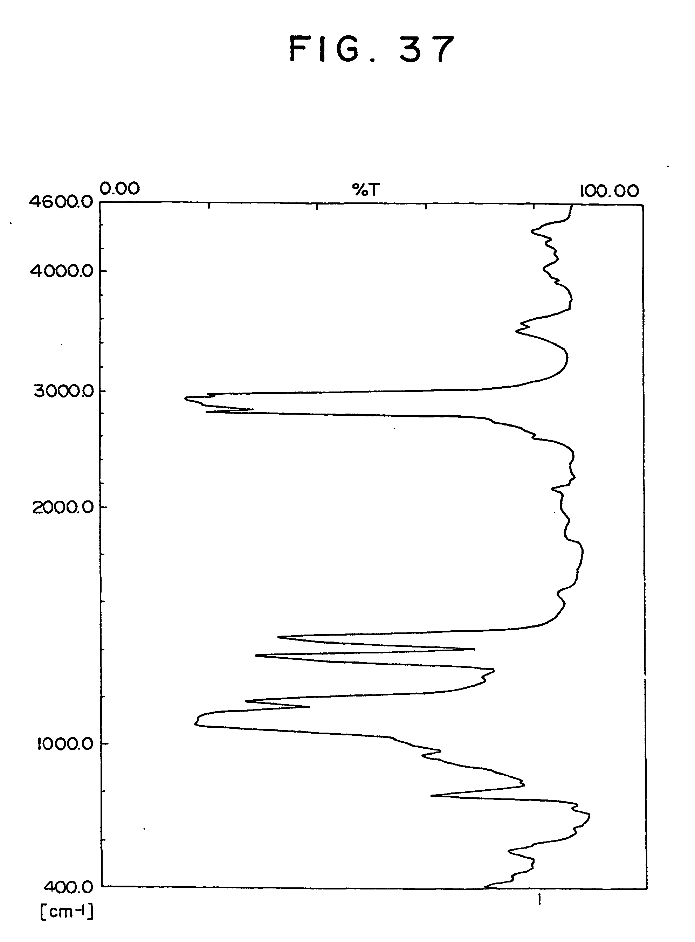

The infrared absorption spectrum, the ¹³C-NMR chart and the ¹H-NMR chart are shown in Figure 37, Figure 38 and Figure 39, respectively.

By the same reason as in Example 17, the polymer obtained above did not contain any of an unsaturated bond, an acetal structure and an aldehyde structure.

### Example 23

Average molecular weights and dispersion of molecular weight of the ethyl vinyl ether/isopropyl vinyl ether copolymer obtained in Example 7D described above were measured. The results of the measurements are shown in Table 3 together with the results of measurements of kinematic viscosity, compatibility with Flon and volume intrinsic resistance.

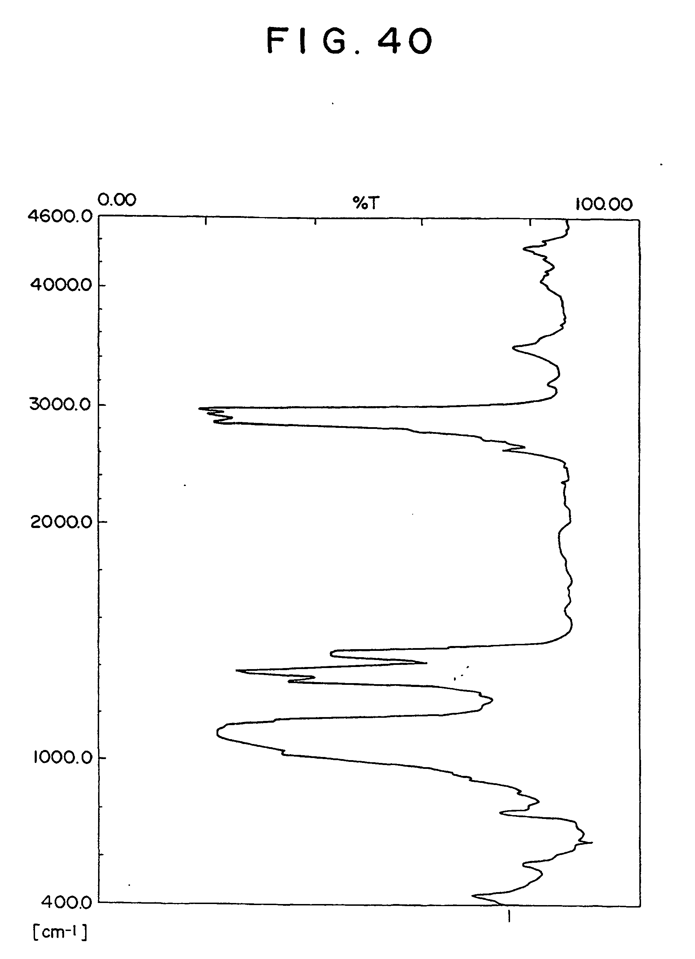

The infrared absorption spectrum, the ¹³C-NMR chart and the ¹H-NMR chart are shown in Figure 40, Figure 41 and Figure 42, respectively.

By the same reason as in Example 17, the polymer obtained above did not contain any of an unsaturated bond, an acetal structure and an aldehyde structure.

### Example 24

Into a 500 ml glass flask equipped with a dropping funnel, a cooler and a stirrer, 50 g of toluene, 11 g of isobutyl alcohol and 0.5 g of boron trifluoride diethyl etherate were charged. Into a dropping funnel, 100 g of isobutyl vinyl ether was charged and dropped in 55 minutes. The temperature of the reaction solution increased by the heat of reaction and the temperature was kept at about 30°C by cooling with an ice water bath. After finishing the dropping, the solution was further stirred for 5 minutes. The reaction mixture was transferred to a washing vessel and washed with 100 ml of a 5 weight % aqueous solution of sodium hydroxide 3 times and then with 150 ml of water 3 times. The solvent and unreacted raw materials were removed under reduced pressure by using a rotary evaporator to obtain 107 g of the crude product. The crude product had a kinematic viscosity of 52.4 cSt at 40°C.

Into a 1 liter autoclave made of SUS-316L, 90 g of the crude product, 300 g of hexane, 4.8 g of Raney nickel and 4.8 g of zeolite were charged. Hydrogen was introduced into the autoclave and the pressure of hydrogen was adjusted to 20 kg/cm². After stirring for about 30 seconds, the pressure was released. Hydrogen was introduced into the autoclave again to make the pressure of hydrogen 20 kg/cm² and, after stirring for about 30 seconds, the pressure of hydrogen was released. After repeating this operation once more, the pressure of hydrogen was increased to 50 kg/cm² and the temperature was increased to 140°C in 30 minutes under stirring. The reaction was conducted at 140°C for 2 hours. The reaction proceeded during and after the increase in the temperature and decrease in the pressure was observed. The increase in the pressure with increase in the temperature and the decrease in the pressure with the reaction were suitably compensated by decreasing or increasing the pressure and the pressure of hydrogen was kept at 50 kg/cm² during the reaction. After finishing the reaction, the reaction mixture was cooled to room temperature and the pressure was decreased to atmospheric pressure. The catalyst was precipitated by standing for 1 hour and the reaction liquid was separated by decantation. The catalyst was washed with 30 ml of hexane twice. The washing liquid was combined with the reaction liquid and filtered with a filter paper. The combined liquid was then transferred to a 1 liter washing vessel and washed with 100 ml of a 5 weight % aqueous solution of sodium hydroxide 3 times and then with 150 ml of distilled water 5 times. Hexane, water and the like were removed under reduced pressure by using a rotary evaporator. The yield was 80.5 g.

Kinematic viscosity, average molecular weights, dispersion of the molecular weight, compatibility with Flon, volume intrinsic resistance and stability to hydrolysis of the polymer of isobutyl vinyl ether obtained above were measured. The results are shown in Table 3.

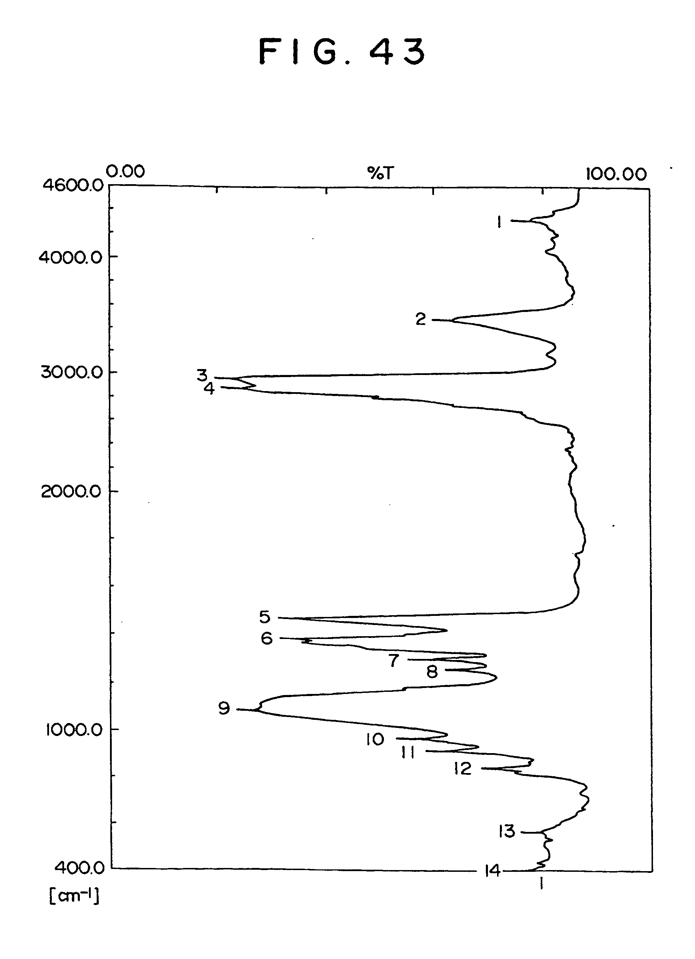

The infrared absorption spectrum, the ¹³C-NMR chart and the ¹H-NMR chart are shown in Figure 43, Figure 44 and Figure 45, respectively.

By the same reason as in Example 17, the polymer obtained above did not contain any of an unsaturated bond, an acetal structure and an aldehyde structure.

### Example 25

Average molecular weights and dispersion of molecular weight of the 1-ethoxy-1-propene polymer obtained in Example 7E described above were measured. The results of the measurements are shown in Table 3 together with the results of measurements of kinematic viscosity, compatibility with Flon and volume intrinsic resistance.

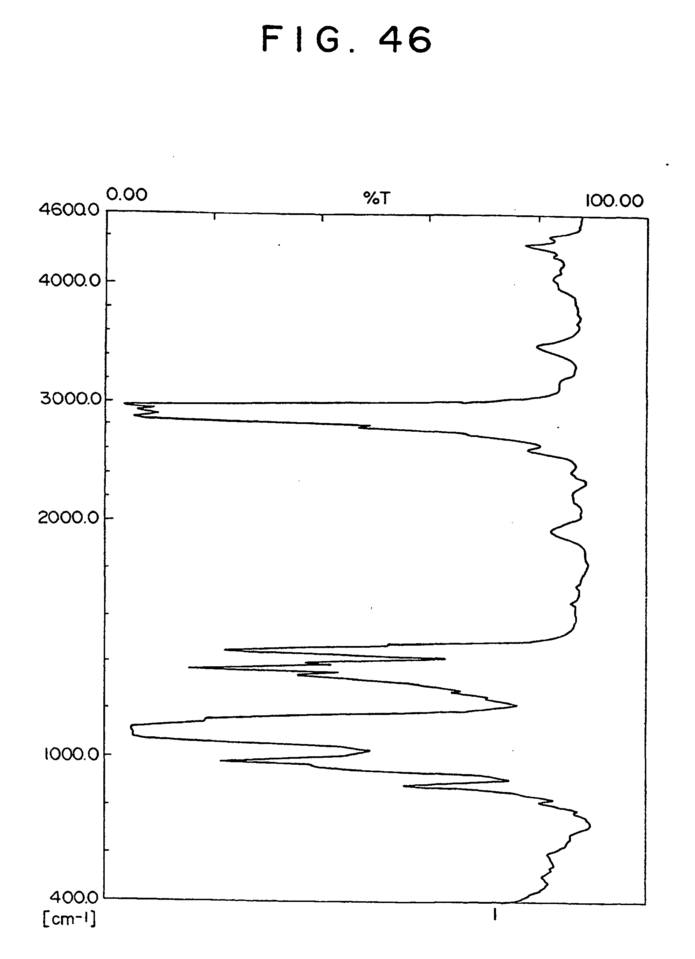

The infrared absorption spectrum, the ¹³C-NMR chart and the ¹H-NMR chart are shown in Figure 46, Figure 47 and Figure 48, respectively.

By the same reason as in Example 17, the polymer obtained above did not contain any of an unsaturated bond, an acetal structure and an aldehyde structure.

### Example 25A

Into a 500 ml glass flask equipped with a dropping funnel, a cooler and a stirrer, 100 g of toluene, 21.3 g of acetaldehyde dimethoxyethyl acetal and 0.45 g of boron trifluoride diethyl etherate were charged. Into a dropping funnel, 112 g of methoxyethyl vinyl ether was charged and dropped in 50 minutes. The temperature of the reaction solution increased by the heat of reaction and the temperature was kept at about 25°C by cooling with an ice water bath. After finishing the dropping, the solution was further stirred for 5 minutes. The reaction mixture was transferred to a washing vessel and 200 ml of chloroform were added to it. The product was washed with 100 ml of a 5 weight % aqueous solution of sodium hydroxide 3 times and then with 150 ml of water 3 times. The solvent and unreacted raw materials were removed under reduced pressure by using a rotary evaporator to obtain 129 g of the crude product. The crude product had a kinematic viscosity of 33.3 cSt at 40°C.

Into a 1 liter autoclave made of SUS-316L, 110 g of the crude product, 300 g of hexane, 5.5 g of Raney nickel and 5.5 g of zeolite were charged. Hydrogen was introduced into the autoclave and the pressure of hydrogen was adjusted to 20 kg/cm². After stirring for about 30 seconds, the pressure was released. Hydrogen was introduced into the autoclave again to make the pressure of hydrogen 20 kg/cm² and, after stirring for about 30 seconds, the pressure of hydrogen was released. After repeating this operation once more, the pressure of hydrogen was increased to 50 kg/cm² and the temperature was increased to 130°C in 30 minutes under stirring. The reaction was conducted at 130°C for 2 hours. The reaction proceeded during and after the increase in the temperature and decrease in the pressure was observed. The increase in the pressure with increase in the temperature and the decrease in the pressure with the reaction were suitably compensated by decreasing or increasing the pressure and the pressure of hydrogen was kept at 50 kg/cm² during the reaction. After finishing the reaction, the reaction mixture was cooled to room temperature and the pressure was decreased to atmospheric pressure. The catalyst was precipitated by standing for 1 hour and the reaction liquid was separated by decantation. The catalyst was washed with 30 ml of hexane twice. The washing liquid was combined with the reaction liquid and filtered with a filter paper. Hexane was removed from the combined liquid under reduced pressure by using a rotary evaporator and 200 ml of chloroform was added to the remaining product. The product was then transferred to a washing vessel and washed with 100 ml of a 5 weight % aqueous solution of sodium hydroxide 3 times and then with 150 ml of distilled water 5 times. The solvent, water and the like were removed under reduced pressure by using a rotary evaporator. The yield was 94 g.

Kinematic viscosity, compatibility with Flon and stability to hydrolysis of the polymer of methoxyethyl vinyl ether obtained above were measured. The results are shown in Table 3.

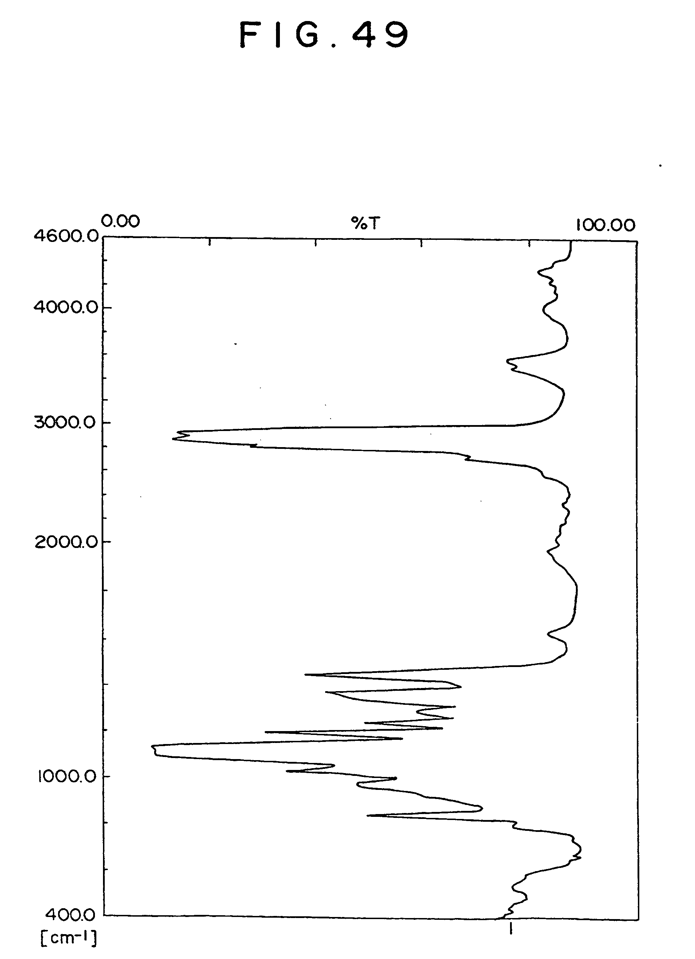

The infrared absorption spectrum, the ¹³C-NMR chart and the ¹H-NMR chart are shown in Figure 49, Figure 50 and Figure 51, respectively.

By the same reason as in Example 17, the polymer obtained above did not contain any of an unsaturated bond, an acetal structure and an aldehyde structure.

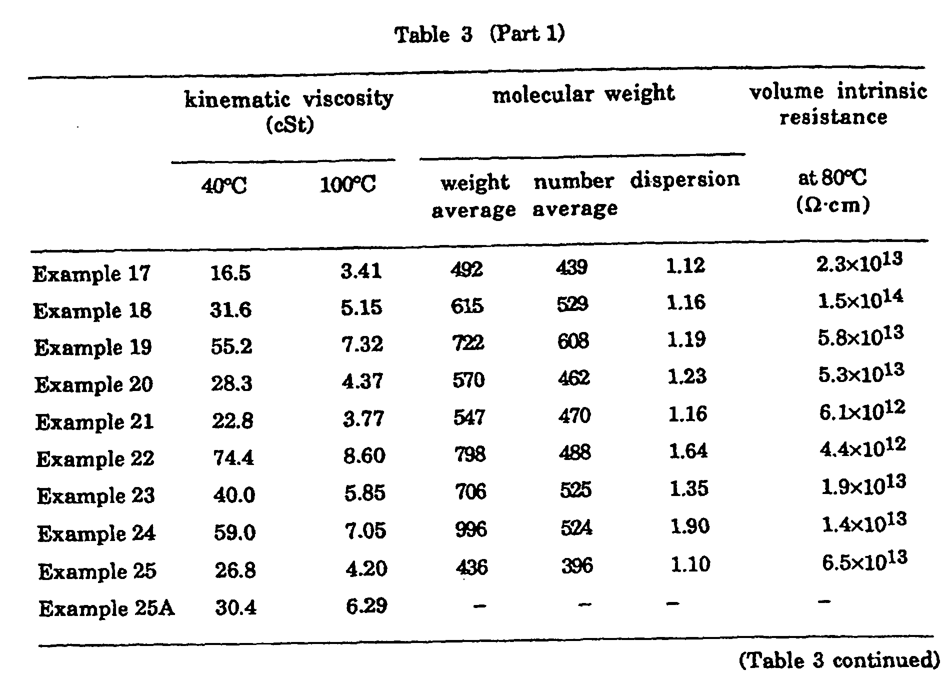

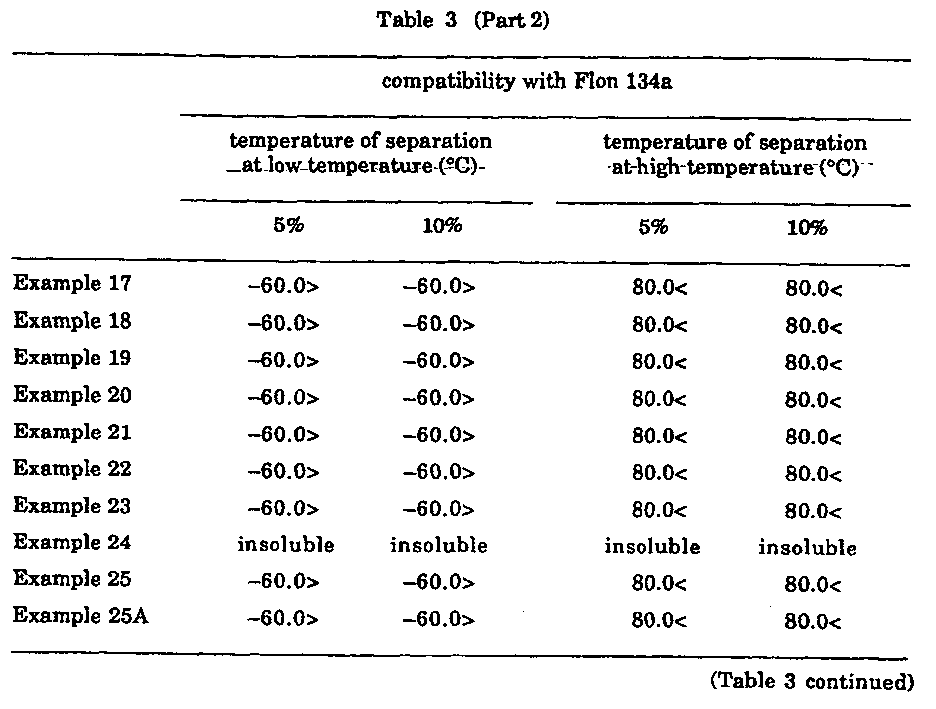

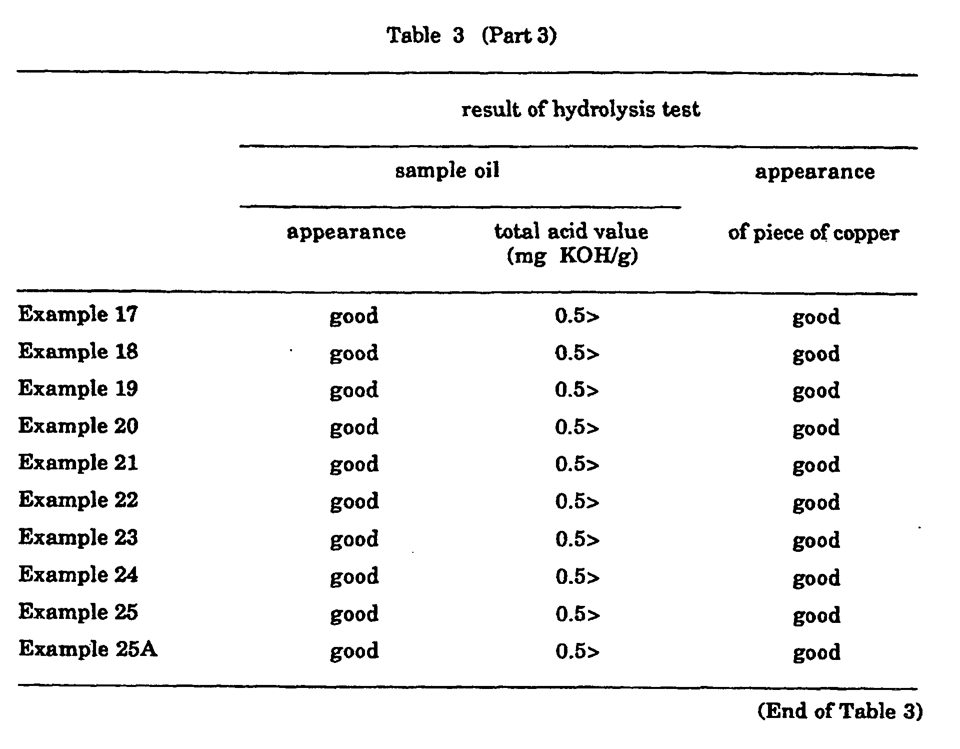

### Example 26

Into a 5 liter glass flask equipped with a dropping funnel, a cooler and a stirrer, 1000 g of toluene, 195 g of ethanol and 5.0 g of boron trifluoride diethyl etherate were charged. The temperature of the solution was 14°C. To the solution, 102 g of ethyl vinyl ether was added. The temperature of the reaction solution increased by the heat of reaction and the reaction solution was kept stirring while it was cooled with an ice water bath. (The temperature of the solution reached the maximum of 21°C 3 minutes after the addition of ethyl vinyl ether. The reaction was conducted by cooling with the ice water bath in all the following procedures.) When the temperature decreased to 15°C, 102 g of ethyl vinyl ether was added again. The generation of heat was observed again and the temperature increased. When the temperature decreased to 15°C, 102 g of ethyl vinyl ether was added further. The generation of heat was observed again and the temperature of the solution was also increased. When decrease in the temperature of the solution was observed, ethyl vinyl ether was dropped and the generation of heat was observed immediately in response to the addition. Thereafter, 2700 g of ethyl vinyl ether was dropped into the solution at about constant speed (about 20 cc/minute) in such a manner that the temperature of the reaction solution was kept constant at 25°C. After finishing the dropping, the reaction mixture was transferred to a washing vessel and washed with 1000 ml of a 3 weight % aqueous solution of sodium hydroxide 3 times and then with 1000 ml of water 3 times. The solvent and low boiling components were removed under reduced pressure by using a rotary evaporator to obtain 3040 g of the product. The product had light yellow color.

The product had a kinematic viscosity of 44.3 cSt at 40°C and 5.90 cSt at 100°C.

### Example 27

Into a 500 ml glass flask equipped with a dropping funnel, a cooler and a stirrer, 36 g of acetaldehyde diethyl acetal, 80 g of toluene and 0.36 g of boron trifluoride diethyl etherate were charged. The temperature of the solution was 15°C. When ethyl vinyl ether was dropped into the solution, generation of heat was observed immediately in response to the addition of ethyl vinyl ether and 256 g of ethyl vinyl ether was dropped at about constant speed (about 5 cc/minute) in such a manner that the temperature of the reaction solution was kept constant at 25°C while the reaction solution was cooled with an ice water bath. After finishing the dropping, the reaction mixture was transferred to a washing vessel and washed with 100 ml of a 3 weight % aqueous solution of sodium hydroxide 3 times and then with 100 ml of water 3 times. The solvent and low boiling components were removed under reduced pressure by using a rotary evaporator to obtain 277 g of the product. The product had light yellow color.

The product had the kinematic viscosity of 130.7 cSt at 40°C and 11.8 cSt at 100°C.

### Example 28

Into a 500 ml glass flask equipped with a dropping funnel, a cooler and a stirrer, 18 g of acetaldehyde diethyl acetal, 80 g of toluene and 0.18 g of boron trifluoride diethyl etherate were charged. The temperature of the solution was 15°C. When ethyl vinyl ether was dropped into the solution, generation of heat was observed immediately in response to the addition of ethyl vinyl ether and 256 g of ethyl vinyl ether was dropped at about constant speed (about 5 cc/minute) in such a manner that the temperature of the reaction solution was kept constant at 25°C while the reaction solution was cooled with an ice water bath. After finishing the dropping, the reaction mixture was transferred to a washing vessel and washed with 100 ml of a 3 weight % aqueous solution of sodium hydroxide 3 times and then with 100 ml of water 3 times. The solvent and low boiling components were removed under reduced pressure by using a rotary evaporator to obtain 261 g of the product. The product had light yellow color. The product had a kinematic viscosity of 993.1 cSt at 40°C and 44.7 cSt at 100°C.

### Example 29

Into a 500 ml glass flask equipped with a dropping funnel, a cooler and a stirrer, 9 g of acetaldehyde diethyl acetal, 80 g of toluene and 0.09 g of boron trifluoride diethyl etherate were charged. The temperature of the solution was 15°C. When ethyl vinyl ether was dropped into the solution, generation of heat was observed immediately in response to the addition of ethyl vinyl ether and 256 g of ethyl vinyl ether was dropped at about constant speed (about 5 cc/minute) in such a manner that the temperature of the reaction solution was kept constant at 25°C while the reaction solution was cooled with an ice water bath. After finishing the dropping, the reaction mixture was transferred to a washing vessel and washed with 100 ml of a 3 weight % aqueous solution of sodium hydroxide 3 times and then with 100 ml of water 3 times. The solvent and low boiling components were removed under reduced pressure by using a rotary evaporator to obtain 255 g of the product. The product had light yellow color.

The product had a kinematic viscosity of 9356 cSt at 40°C and 225.5 cSt at 100°C.

### Example 30

Into a 500 ml glass flask equipped with a dropping funnel, a cooler and a stirrer, 4 g of acetaldehyde diethyl acetal, 80 g of toluene and 0.04 g of boron trifluoride diethyl etherate were charged. The temperature of the solution was 15°C. When ethyl vinyl ether was dropped into the solution, generation of heat was observed immediately in response to the addition of ethyl vinyl ether and 256 g of ethyl vinyl ether was dropped at about constant speed (about 5 cc/minute) in such a manner that the temperature of the reaction solution was kept constant at 25°C while the reaction solution was cooled with an ice water bath. After finishing the dropping, the reaction mixture was transferred to a washing vessel and washed with 100 ml of a 3 weight % aqueous solution of sodium hydroxide 3 times and then with 100 ml of water 3 times. The solvent and low boiling components were removed under reduced pressure by using a rotary evaporator to obtain 252 g of the product. The product had light yellow color.

The product had a weight-average molecular weight of 2079 and a number-average molecular weight of 6750.

The average molecular weights were obtained by GPC measurement under the following conditions (These conditions were used in this Example only.):

| | |
|---|---|
| Apparatus | a product by Nippon Bunko Co., Ltd. (pump) SHODEX RI SE-61 (detector) |
| Column | TSK HM+GMH6×2+G2000H8 |
| Temperature | room temperature |
| Solvent | THF |
| Speed of elution | 1.4 ml/minute |
| Standard substance | polystyrene |

### Example 31

Into a 500 ml glass flask equipped with a dropping funnel, a cooler and a stirrer, 16 g of acetaldehyde diethyl acetal, 80 g of toluene and 0.16 g of boron trifluoride diethyl etherate were charged. The temperature of the solution was 15°C. When ethyl vinyl ether was dropped into the solution, generation of heat was observed immediately in response to the addition of ethyl vinyl ether and 256 g of ethyl vinyl ether was dropped at about constant speed (about 5 cc/minute) in such a manner that the temperature of the reaction solution was kept constant at 25°C while the reaction solution was cooled with an ice water bath. After finishing the dropping, the reaction mixture was transferred to a washing vessel and washed with 100 ml of a 3 weight % aqueous solution of sodium hydroxide 3 times and then with 100 ml of water 3 times. The solvent and low boiling components were removed under reduced pressure by using a rotary evaporator to obtain 262 g of the product. The product had light yellow color.

The product had a kinematic viscosity of 1746 cSt at 40°C and 64.6 cSt at 100°C.

### Example 32

Into a 500 ml glass flask equipped with a dropping funnel, a cooler and a stirrer, 15 g of acetaldehyde diethyl acetal, 80 g of toluene and 0.15 g of boron trifluoride diethyl etherate were charged. The temperature of the solution was 15°C. When ethyl vinyl ether was dropped into the solution, generation of heat was observed immediately in response to the addition of ethyl vinyl ether and 256 g of ethyl vinyl ether was dropped at about constant speed (about 5 cc/minute) in such a manner that the temperature of the reaction solution was kept constant at 25°C while the reaction solution was cooled with an ice water bath. After finishing the dropping, the reaction mixture was transferred to a washing vessel and washed with 100 ml of a 3 weight % aqueous solution of sodium hydroxide 3 times and then with 100 ml of water 3 times. The solvent and low boiling components were removed under reduced pressure by using a rotary evaporator to obtain 260 g of the product. The product had light yellow color.

The product had a kinematic viscosity of 1903 cSt at 40°C and 68.1 cSt at 100°C.

### Example 33

Into a 500 ml glass flask equipped with a dropping funnel, a cooler and a stirrer, 4 g of acetaldehyde diethyl acetal, 30 g of toluene and 0.4 g of FeCl₃·6H₂O were charged. The temperature of the solution was 10°C. When ethyl vinyl ether was dropped into the solution, generation of heat was observed immediately in response to the addition of ethyl vinyl ether and 30 g of ethyl vinyl ether was dropped at about constant speed in such a manner that the temperature of the reaction solution was kept constant at 10°C while the reaction solution was cooled with an ice water bath. After finishing the dropping, the reaction mixture was transferred to a washing vessel and washed with 15 ml of a 3 weight % aqueous solution of sodium hydroxide 3 times and then with 15 ml of water 3 times. The solvent and low boiling components were removed under reduced pressure by using a rotary evaporator to obtain 30.6 g of the product. The product had dark yellow color.

The product had a kinematic viscosity of 21.86 cSt at 40°C and 3.94 cSt at 100°C.

### Example 34

Into a 500 ml glass flask equipped with a dropping funnel, a cooler and a stirrer, 3 g of acetaldehyde diethyl acetal, 30 g of toluene and 0.18 g of FeCl₃ were charged. The temperature of the solution was 15°C. When ethyl vinyl ether was dropped into the solution, generation of heat was observed immediately in response to the addition of ethyl vinyl ether and 30 g of ethyl vinyl ether was dropped at about constant speed in such a manner that the temperature of the reaction solution was kept constant at 30°C while the reaction solution was cooled with an ice water bath. After finishing the dropping, the reaction mixture was transferred to a washing vessel and washed with 15 ml of a 3 weight % aqueous solution of sodium hydroxide 3 times and then with 15 ml of water 3 times. The solvent and low boiling components were removed under reduced pressure by using a rotary evaporator to obtain 29.4 g of the product. The product had dark yellow color.

The product had a kinematic viscosity of 326.7 cSt at 40°C and 25.69 cSt at 100°C.

### Example 35

Into a 1 liter autoclave made of SUS-316L, 100 g of acetaldehyde diethyl acetal, 100 g of n-heptane, 3.0 g of Raney nickel and 3.0 g of zeolite were charged. Hydrogen was introduced into the autoclave and the pressure of hydrogen was adjusted to 10 kg/cm². After stirring for about 30 seconds, the pressure was released. Hydrogen was introduced into the autoclave again to make the pressure of hydrogen 10 kg/cm² and, after stirring for about 30 seconds, the pressure of hydrogen was released. Then, the pressure of hydrogen was increased to 30 kg/cm² and the temperature was increased to 130°C in 30 minutes under stirring. The reaction was conducted at 130°C for 2 hours and 30 minutes. The reaction proceeded during and after the increase in the temperature and decrease in the pressure was observed. The increase in the pressure with increase in the temperature and the decrease in the pressure with the reaction were suitably compensated by decreasing or increasing the pressure and the pressure of hydrogen was kept at 30 kg/cm² during the reaction. After finishing the reaction, the reaction mixture was cooled to 20°C and the pressure was decreased to atmospheric pressure. Qualitative analysis and quantitative analysis were made with the reaction product by gas chromatography. The conversion of acetaldehyde diethyl acetal was 94.9 % and the selectivity to diethyl ether was 68.3 %.

### Example 36

Into a 1 liter autoclave made of SUS-316L, 100 g of propionaldehyde diethyl acetal, 100 g of n-octane, 6.0 g of Raney nickel and 6.0 g of zeolite were charged. Hydrogen was introduced into the autoclave and the pressure of hydrogen was adjusted to 10 kg/cm². After stirring for about 30 seconds, the pressure was released. Hydrogen was introduced into the autoclave again to make the pressure of hydrogen 10 kg/cm² and, after stirring for about 30 seconds, the pressure of hydrogen was released. Then, the pressure of hydrogen was increased to 30 kg/cm² and the temperature was increased to 130°C in 30 minutes under stirring. The reaction was conducted at 130°C for 1 hour and 30 minutes. The reaction proceeded during and after the increase in the temperature and decrease in the pressure was observed. The increase in the pressure with increase in the temperature and the decrease in the pressure with the reaction were suitably compensated by decreasing or increasing the pressure and the pressure of hydrogen was kept at 30 kg/cm² during the reaction. After finishing the reaction, the reaction mixture was cooled to 20°C and the pressure was decreased to atmospheric pressure. Qualitative analysis and quantitative analysis were conducted with the reaction product by gas chromatography. The conversion of propionaldehyde diethyl acetal was 97.0 % and the selectivity to ethyl n-propyl ether was 72.0 %.

### Example 37

### (1) Preparation of material

Into a 5 liter glass flask equipped with a dropping funnel, a cooler and a stirrer, 1000 g of toluene, 500 g of acetaldehyde diethyl acetal and 5.0 g of boron trifluoride diethyl etherate were charged. Into a dropping funnel, 2500 g of ethyl vinyl ether was charged and dropped in 2 hours and 30 minutes. During this period, the reaction started and the temperature of the reaction solution increased. The temperature was kept at about 25°C by cooling with an ice water bath. After finishing the dropping, the solution was further stirred for 5 minutes. The reaction mixture was transferred to a washing vessel and washed with 1000 ml of a 5 weight % aqueous solution of sodium hydroxide 3 times and then with 1000 ml of water 3 times. The solvent and unreacted raw materials were removed under reduced pressure by using a rotary evaporator to obtain 2833 g of the product. The ¹H-NMR chart of this product is shown in Figure 52. From this figure, the product was found to have the structures of the following formulae (A') and (B'). The product had a kinematic viscosity of 5.18 cSt at 100°C and 38.12 cSt at 40°C.

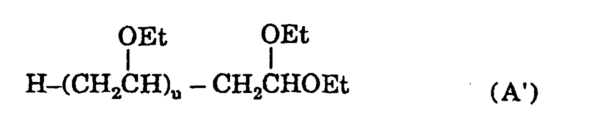

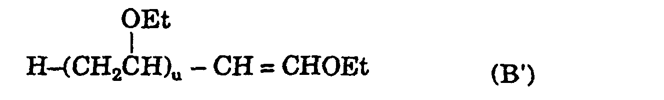

The ratio of numbers of molecule was (A') : (B') = 4.5 : 1 and the average value of u was 5.6.

(2) Into a 1 liter autoclave made of SUS-316L, 200 g of the oligomer prepared in (1) described above, 6.0 g of Raney nickel and 6.0 g of zeolite were charged. Hydrogen was introduced into the autoclave and the pressure of hydrogen was adjusted to 10 kg/cm². After stirring for about 30 seconds, the pressure was released. Hydrogen was introduced into the autoclave again to make the pressure of hydrogen 10 kg/cm² and, after stirring for about 30 seconds, the pressure of hydrogen was released. After repeating this operation once more, the pressure of hydrogen was increased to 25 kg/cm² and the temperature was increased to 140°C in 30 minutes under stirring. The reaction was conducted at 140°C for 2 hours. The reaction proceeded during and after the increase in the temperature and decrease in the pressure was observed. The increase in the pressure with increase in the temperature and the decrease in the pressure with the reaction were suitably compensated by decreasing or increasing the pressure and the pressure of hydrogen was kept at 25 kg/cm² during the reaction. After finishing the reaction, the reaction mixture was cooled to room temperature and the pressure was decreased to atmospheric pressure. To the reaction mixture, 100 ml of hexane was added. The catalyst was precipitated by standing for 30 minutes and the reaction solution was separated by decantation. The hexane solution was combined with the reaction solution and filtered with a filter paper. The catalyst was reused in Example 39. Hexane, water and the like were removed under reduced pressure by using a rotary evaporator. The yield was 162 g.

The ¹H-NMR chart of this product is shown in Figure 53. From this chart, the raw material acetal was found to have been converted to the ether compound shown by the formula (C'): 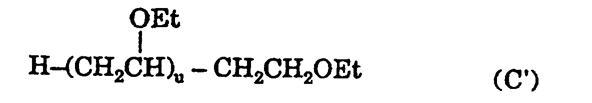 wherein Et is an ethyl group. The conversion was 100 %. The kinematic viscosity was 4.90 at 100°C and 29.50 at 40°C. The oligomer of ethyl vinyl ether having the formula (B') described above was also converted to the ether compound having the formula (C') described above.

### Example 38

Into a 1 liter autoclave made of SUS-316L, 200 g of the oligomer prepared in Example 37 (1) described above, 20 g of Raney nickel and 20 g of zeolite were charged. Hydrogen was introduced into the autoclave and the pressure of hydrogen was adjusted to 7 kg/cm². After stirring for about 30 seconds, the pressure was released. After repeating this operation once more, the pressure of hydrogen was brought to 7 kg/cm² and the temperature was increased to 130°C in 30 minutes under stirring. The reaction was conducted at 130°C for 2 hours and 30 minutes. The reaction proceeded during and after the increase in the temperature and decrease in the pressure was observed. The increase in the pressure with increase in the temperature and the decrease in the pressure with the reaction were suitably compensated by decreasing or increasing the pressure and the pressure of hydrogen was kept at 7 kg/cm² during the reaction. After finishing the reaction, the reaction mixture was cooled to room temperature and the pressure was decreased to atmospheric pressure. The reaction mixture was filtered and water and the like were removed from the solution part under reduced pressure by using a rotary evaporator. The yield was 160 g. By this procedure, the same ether compound as in Example 37 (2) was obtained from the raw material acetal and the conversion was 100 %. The kinematic viscosity was 4.77 at 100°C and 30.27 at 40°C.

### Example 39

In the autoclave used in Example 37 (2) in which the catalyst was remaining, 200 g of the oligomer prepared in Example 37 (1) was charged and the reaction was performed by the same method as in Example 37 (2). The yield was 164 g. By this procedure, the same ether compound was obtained as in Example 37 (2) from the material acetal and the conversion was 100 %. The kinematic viscosity was 4.93 at 100°C and 29.13 at 40°C.

### Example 40

### (1) Preparation of material

The reaction was performed by the same method as in Example 37 (1) except that the amount of acetaldehyde was 450 g, the amount of the boron trifluoride etherate was 4.5 g and the amount of ethyl vinyl ether was 2800 g. The yield was 3175 g. The product had the same structure as that in Example 37 (1). The kinematic viscosity was 6.79 at 100°C and 59.68 at 40°C. The ratio of the numbers of molecule was (A') : (B') = 8 : 1 and the average value of u was 6.8.

(2) Into a 1 liter autoclave made of SUS-316L, 200 g of the oligomer prepared in (1) described above, 10 g of Raney nickel and 15 g of activated clay were charged. Hydrogen was introduced into the autoclave and the pressure of hydrogen was adjusted to 10 kg/cm². After stirring for about 30 seconds, the pressure was released. Hydrogen was introduced into the autoclave again and the pressure of hydrogen was adjusted to 10 kg/cm². After stirring for about 30 seconds, the pressure was released. After repeating this operation once more, the pressure of hydrogen was increased to 30 kg/cm² and the temperature was increased to 150°C in 40 minutes under stirring. The reaction was conducted at 150°C for 1 hour. The reaction proceeded during and after the increase in the temperature and decrease in the pressure was observed. The increase in the pressure with increase in the temperature and the decrease in the pressure with the reaction were suitably compensated by decreasing or increasing the pressure and the pressure of hydrogen was kept at 30 kg/cm² during the reaction. After finishing the reaction, the reaction mixture was cooled to room temperature and the pressure was decreased to atmospheric pressure. The reaction mixture was filtered and water and the like were removed from the solution part under reduced pressure by using a rotary evaporator. The yield was 158 g. By this procedure, the same ether compound as in Example 37 (2) was obtained from the raw material acetal and the conversion was 100 %. The kinematic viscosity was 7.06 at 100°C and 57.32 at 40°C.

### Example 41

Into a 1 liter autoclave made of SUS-316L, 200 g of the oligomer prepared in Example 37 (1) described above, 10 g of zeolite and 5.0 g of Pd/C (supporting 5 % of Pd, a product of Wako Jun-yaku Co., Ltd.) were charged. Hydrogen was introduced into the autoclave and the pressure of hydrogen was adjusted to 7 kg/cm². After stirring for about 30 seconds, the pressure was released. Hydrogen was introduced into the autoclave again and the pressure of hydrogen was adjusted to 7 kg/cm². After stirring for about 30 seconds, the pressure was released. After repeating this operation once more, the pressure of hydrogen was brought to 7 kg/cm² and the temperature was increased to 120°C in 30 minutes under stirring. The reaction was conducted at 120°C for 7 hours. The reaction proceeded during and after the increase in the temperature and decrease in the pressure was observed. The increase in the pressure with increase in the temperature and the decrease in the pressure with the reaction were suitably compensated by decreasing or increasing the pressure and the pressure of hydrogen was kept at 7 kg/cm² during the reaction. After finishing the reaction, the reaction mixture was cooled to room temperature and the pressure was decreased to atmospheric pressure. The reaction mixture was filtered and water and the like were removed from the solution part under reduced pressure by using a rotary evaporator. The yield was 167.2 g. By this procedure, the same ether compound as in Example 37 (2) was obtained from the raw material acetal and the conversion was 100 %. The kinematic viscosity was 5.28 at 100°C and 32.93 at 40°C.

### Example 42

Into a 1 liter autoclave made of SUS-316L, 200 g of the oligomer prepared in (1) described above, 20 g of zeolite and 20 g of Ru/C (supporting 5 % of Ru, a product of Wako Jun-yaku Co., Ltd.) were charged. Hydrogen was introduced into the autoclave and the pressure of hydrogen was adjusted to 30 kg/cm². After stirring for about 30 seconds, the pressure was released. Hydrogen was introduced into the autoclave again and the pressure of hydrogen was adjusted to 30 kg/cm². After stirring for about 30 seconds, the pressure was released. After repeating this operation once more, the pressure of hydrogen was brought to 30 kg/cm² and the temperature was increased to 130°C in 30 minutes under stirring. The reaction was conducted at 130°C for 1 hour. The reaction proceeded during and after the increase in the temperature and decrease in the pressure was observed. The increase in the pressure with increase in the temperature and the decrease in the pressure with the reaction were suitably compensated by decreasing or increasing the pressure and the pressure of hydrogen was kept at 30 kg/cm² during the reaction. After finishing the reaction, the reaction mixture was cooled to room temperature and the pressure was decreased to atmospheric pressure. The reaction mixture was filtered and water and the like were removed from the solution part under reduced pressure by using a rotary evaporator. The yield was 156 g. By this procedure, the same ether compound as in Example 37 (2) was obtained from the raw material acetal and the conversion was 100 %. The kinematic viscosity was 5.18 at 100°C and 31.53 at 40°C.

### Example 43

Into a 2 liter autoclave made of SUS-316L, 15 g of Ni-diatomaceous earth and 350 g of hexane were charged. After replacing the atmosphere inside of the autoclave with hydrogen, the pressure of hydrogen was adjusted to 30 kg/cm². The temperature was increased to 150°C in 30 minutes under stirring and the activation treatment of the catalyst was conducted for 30 minutes. After cooling the autoclave, 300 g of the oligomer prepared in Example 37 (1) described above and 15 g of zeolite were charged into the autoclave. Hydrogen was introduced into the autoclave and the pressure of hydrogen was adjusted to 30 kg/cm². After stirring for about 30 seconds, the pressure was released. Hydrogen was introduced into the autoclave again and the pressure of hydrogen was adjusted to 30 kg/cm². After stirring for about 30 seconds, the pressure was released. After repeating this operation once more, the pressure of hydrogen was brought to 30 kg/cm² and the temperature was increased to 130°C in 30 minutes under stirring. The reaction was conducted at 130°C for 1 hour. The reaction proceeded during and after the increase in the temperature and decrease in the pressure was observed. The increase in the pressure with increase in the temperature and the decrease in the pressure with the reaction were suitably compensated by decreasing or increasing the pressure and the pressure of hydrogen was kept at 30 kg/cm² during the reaction. After finishing the reaction, the reaction mixture was cooled to room temperature and the pressure was decreased to atmospheric pressure. The reaction mixture was filtered and water and the like were removed from the solution part under reduced pressure by using a rotary evaporator. The yield was 240 g.

The conversion of the raw material acetal was 100 % like in Example 37. The kinematic viscosity was 5.38 at 100°C and 33.12 at 40°C.

## Claims

1. Use of a polyvinyl ether compound which comprises a homopolymer or a copolymer of an alkyl vinyl ether comprising the constituting units expressed by the general formula (I) or (I'): 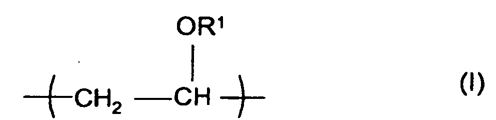 or 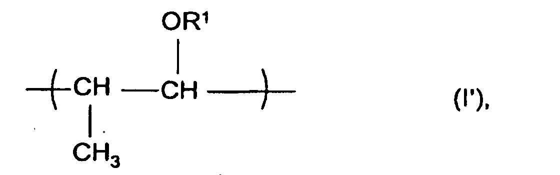 wherein R¹ is an alkyl group having 1 to 3 carbon atoms, and having a weight-average molecular weight of 400 to 1200 and one end having the structure expressed by the general formula (II): 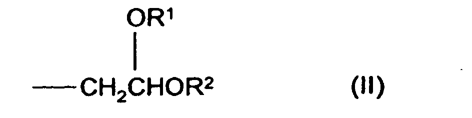 or the formula (III):
-CH=CHOR^{a} (III)
wherein R¹ is as previously defined, R² is a hydrocarbon group having 1 to 8 carbon atoms and R^{a} is either one of R¹ and R² as a lubricating oil for compression-type refrigerators.

2. The use according to Claim 1, wherein the constituting units expressed by the general formula (I) comprise one kind of the constituting unit.

3. The use according to Claim 1, wherein the constituting units expressed by the general formula (I) comprise two or more kinds of the constituting units.

4. The use according to Claim 1, wherein the ratio of the weight-average molecular weight to the number-average molecular weight is in the range of 1.05 to 1.50.

5. A method of production of an ether compound expressed by the general formula (XV): 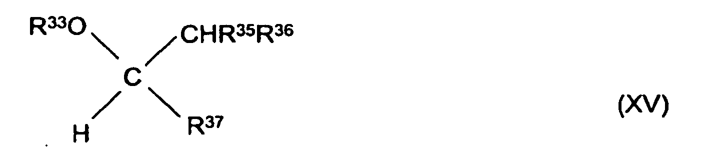 or by the general formula (XVI): 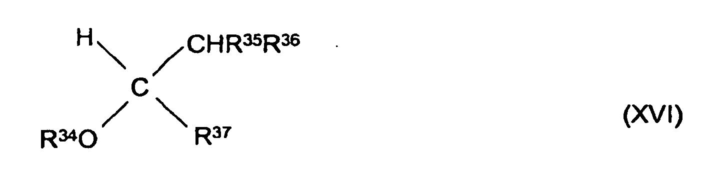 wherein R³³ and R³⁴ are each a hydrocarbon group or a hydrocarbon group containing ether oxygens in the main chain, in the side chain or in both of them, and may be the same or different from each other and R³⁵, R³⁶ and R³⁷ are each a hydrogen atom, a hydrocarbon group or a hydrocarbon group containing ether oxygens in the main chain, in the side chain or in both of them, and may be the same or different from each other,
which comprises bringing an acetal compound or a ketal compound expressed by the general formula (XIV): 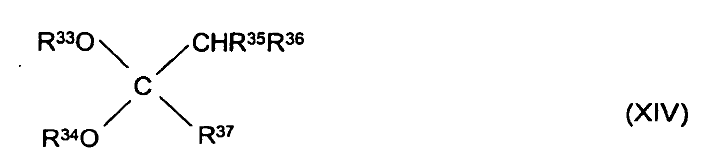 wherein R³³, R³⁴, R³⁵, R³⁶ and R³⁷ are the same as those in the formulae (XV) and (XVI),
into the reaction with hydrogen in the presence of a solid catalyst having acidic property and hydrogenating ability,
wherein the solid catalyst having acidic property and hydrogenating ability is a catalyst comprising a combination of a hydrogenation catalyst and a solid acid catalyst or a solid acid catalyst having hydrogenating ability,
wherein the solid catalyst having acidic property and hydrogenating ability is
(A) a catalyst comprising a combination of a hydrogenation catalyst and a solid acid catalyst,
whereby the hydrogenation catalyst is selected from the group consisting of
(1) nickel, palladium, rhodium and ruthenium alone or as the main component,
(2) catalysts having the metal component of (1) supported on carriers such as activated charcoal, alumina and diatomaceous earth or
(3) Raney catalysts such as Raney nickel and Raney cobalt, and the solid catalyst is selected from the group consisting of activated clay, acidic clay, various kinds of zeolite, ion exchange resins, silica-alumina and heteropolyacids;
or (B) a solid acid catalyst having hydrogenating ability selected from the group consisting of nickel, palladium, platinum, and ruthenium supported on various kinds of zeolite.

6. The method of production of an ether compound as claimed in Claim 5, wherein the acetal compound or the ketal compound expressed by the general formula (XIV) is a compound expressed by the general formula (XVII): 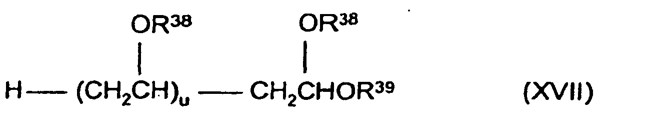 wherein R³⁸ and R³⁹ are each a hydrocarbon group having 1 to 20 carbon
atoms or a hydrocarbon group containing ether oxygens, and may be the
same or different from each other, R³⁸ may be the same or different
among the constituting units and u is an integer of 1 to 500, and the ether compound thus obtained is a compound expressed by the general formula (XVIII): 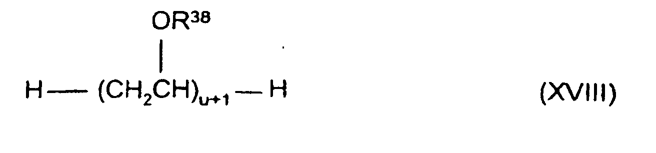 or by the general formula (XIX) : 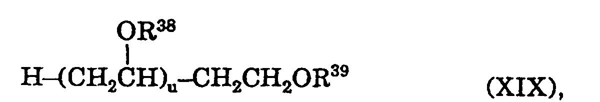 wherein R³⁸, R³⁹ and u are each the same as those in the formule (XVII).

7. The method of production of an ether compound as claimed in Claim 5, wherein the acetal compound or the compound expressed by the general formula (XIV) is a compound expressed by the general formula (XX):
R⁴⁰CH(OR⁴¹)₂ (XX),
wherein R⁴⁰ and R⁴¹ are each a hydrocarbon group having 1 to 20 carbon atoms, and may the same or different from each other, and the ether compound thus obtained is a compound expressed by the general formula (XXI),
R₄₀CH₂OR⁴¹ (XXI),
wherein R⁴⁰ and R⁴¹ are the same as those in the formula (XX).

## Patentansprüche

1. Verwendung einer Polyvinyletherverbindung, welche ein Homopolymer oder ein Copolymer eines Alkylvinylethers umfasst, enthaltend die durch die allgemeinen Formeln (I) oder (I') dargestellten konstituierenden Einheiten: 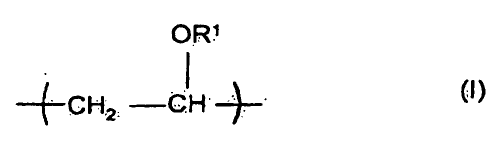 oder 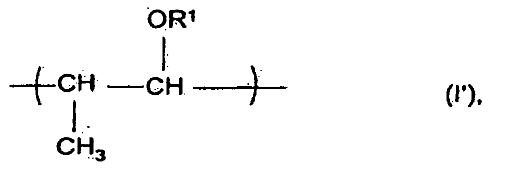 worin R¹ eine Alkylgruppe mit 1 bis 3 Kohlenstoffatomen ist, mit einer massegemittelten Molekülmasse von 400 bis 1200 und einem Ende mit einer Struktur, dargestellt durch die allgemeine Formel (II): 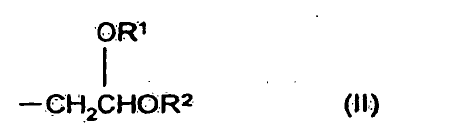 oder die Formel (III):
-CH=CHOR^{a} (III)
worin R¹ wie vorstehend definiert ist, R² eine Kohlenwasserstoffgruppe mit 1 bis 8 Kohlenstoffatomen und R° eine der beiden von R¹ und R² ist, als Schmieröl für Kühlvorrichtungen vom Kompressionstyp.

2. Die Verwendung gemäß Anspruch 1, wobei die durch die allgemeine Formel (I) dargestellten konstituierenden Einheiten eine Art konstituierende Einheit umfassen.

3. Die Verwendung gemäß Anspruch 1, wobei die durch die allgemeine Formel (I) dargestellten konstituierenden Einheiten zwei oder mehr Arten konstituierende Einheiten umfassen.

4. Die Verwendung gemäß Anspruch 1, wobei das Verhältnis von massegemittelter Molekülmasse zum Molekulargewicht-Zahlenmittel im Bereich von 1,05 bis 1,50 ist.

5. Verfahren zur Herstellung einer durch die allgemeine Formel (XV) 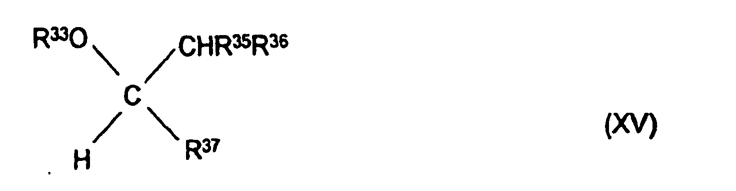 oder durch die allgemeine Formel (XVI) 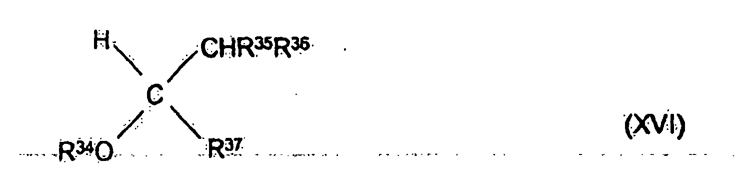 dargestellten Etherverbindung, worin R³³ und R³⁴ jeweils eine Kohlenwasserstoffgruppe oder eine Ethersauerstoffe in der Hauptkette, in der Seitenkette oder in beiden von ihnen enthaltende Kohlenwasserstoffgruppe ist, die gleich oder verschieden sein können und R³⁵, R³⁶ und R³⁷ jeweils ein Wasserstoffatom, eine Kohlenwasserstoffgruppe oder eine Ethersauerstoffe in der Hauptkette, in der Seitenkette oder in beiden von ihnen enthaltende Kohlenwasserstoffgruppe ist, die gleich oder verschieden sein können, welches umfasst, zur Reaktion Bringen einer Acetalverbindung oder einer Ketalverbindung, dargestellt durch die allgemeine Formel (XIV), 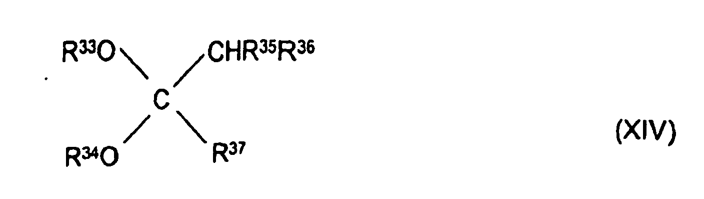 worin R³³, R³⁴, R³⁵, R³⁶ und R³⁷ die gleichen sind wie die in den Formeln (XV) und (XVI), mit Wasserstoff in Gegenwart eines festen Katalysators mit saurer Eigenschaft und Hydrierfähigkeit, wobei der feste Katalysator mit saurer Eigenschaft und Hydrierfähigkeit ein Katalysator ist, umfassend eine Kombination eines Hydrierkatalysators und eines festen Säurekatalysators oder eines festen Säurekatalysators mit Hydrierfähigkeit, wobei der feste Katalysator mit saurer Eigenschaft und Hydrierfählgkeit ist
(A) ein Katalysator umfassend eine Kombination eines Hydrierkatalysators und eines festen Säurekatalysators,
wobei der Hydrierkatalysator ausgewählt ist aus der Gruppe, bestehend aus
(1) Nickel, Palladium, Rhodium und Ruthenium allein oder als Hauptkomponente,
(2) Katalysatoren mit der Metallkomponente von (1), gestützt auf Träger, wie Aktivkohle, Aluminiumoxid und Diatomeenerde oder
(3) Raneykatalysatoren wie Raneynickel und Raneykobalt und der feste Katalysator ausgewählt ist aus der Gruppe, bestehend aus aktivierter Tonerde, saurer Tonerde, verschiedenen Arten von Zeolith, Ionenaustauscherharzen, Siliziumdioxid-Aluminiumoxid und Heteropolysäuren;
oder (B) ein fester Säurekatalysator mit Hydriertähigkeit, ausgewählt aus der Gruppe, bestehend aus Nickel, Palladium, Platin und Ruthenium, gestützt auf verschiedene Arten von Zeolith.

6. Das Verfahren zur Herstellung einer Etherverbindung wie in Anpruch 5 beansprucht, worin die durch die allgemeine Formel (XIV) dargestellte Acetalverbindung oder Ketalverbindung eine durch die allgemeine Formel (XVII) dargestellte Verbindung ist, 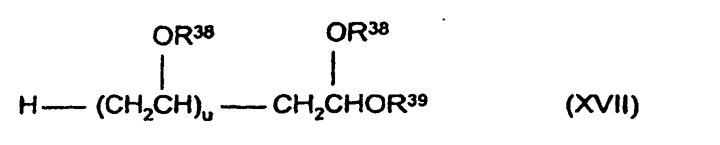 worin R³⁸ und R³⁹ jeweils sind eine Kohlenwasserstoffgruppe mit 1 bis 20 Kohlenstoffatomen oder eine Ethersauerstoffe enthaltende Kohlenwasserstoffgruppe, die gleich oder verschieden sein können, R³⁸ unter den konstitulerenden Einheiten gleich oder verschieden sein kann und u eine ganze Zahl von 1 bis 500 ist und die so erhaltene Etherverbindung eine durch die allgemeine Formel (XVIII) dargestellte Verbindung ist 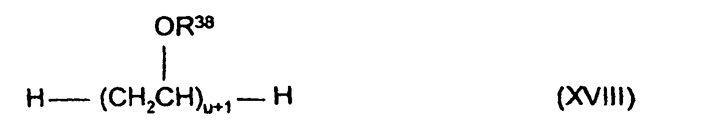 oder eine durch die allgemeine Formel (XIX) 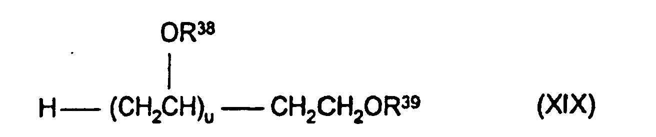 dargestellte Verbindung, worin R³⁸, R³⁹ und u jeweils die gleichen sind wie diejenigen in Formel (XVII).

7. Das Verfahren zur Herstellung einer Etherverbindung wie in Anspruch 5 beansprucht, worin die durch die allgemeine Formel (XIV) dargestellte Acetalverbindung eine durch die allgemeine Formel (XX) dargestellte Verbindung ist
R⁴⁰CH(OR⁴¹)₂ (XX),
worin R⁴⁰ und R⁴¹ jeweils eine Kohlenwasserstoffgruppe mit 1 bis 20 Kohlenstoffatomen sind und gleich oder verschieden sein können und die so erhaltene Etherverbindung eine durch die allgemeine Formel (XXI) dargestellte Verbindung ist
R₄₀CH₂OR⁴¹ (XXI),
worin R⁴⁰ und R⁴¹ die gleichen sind wie diejenigen in Formel (XX).

## Revendications

1. Utilisation d'un composé d'éther polyvinylique qui comprend un homopolymère ou un copolymère d'un éther de vinyle et d'alkyle comprenant les motifs de structure exprimés par la formule (I) ou (I') : 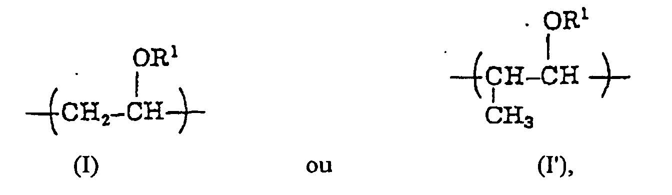 dans lesquelles R¹ est un groupe alkyle ayant 1 à 3 atomes de carbone, et ayant une masse moléculaire moyenne en masse de 400 à 1200 et une extrémité ayant la structure exprimée par la formule générale (II) : 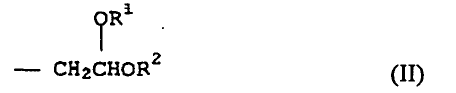 ou la formule (III) :
-CH = CHOR^{a} (III),
dans lesquelles R¹ est tel que précédemment défini, R² est un groupe hydrocarboné ayant 1 à 8 atomes de carbone et R^{a} est l'un de R¹ ou R² comme huile de lubrification pour réfrigérateurs à compression.

2. Utilisation selon la revendication 1, dans laquelle les motifs de structure exprimés par la formule générale (I) comprennent un type de motif de structure.

3. Utilisation selon la revendication 1, dans laquelle les motifs de structure exprimés par la formule générale (I) comprennent deux ou plusieurs types de motifs de structure.

4. Utilisation selon la revendication 1, dans laquelle le rapport de la masse moléculaire moyenne en masse à la masse moléculaire moyenne en nombre est dans la gamme de 1,05 à 1,50.

5. Procédé de production d'un composé d'éther exprimé par la formule générale (XV) : 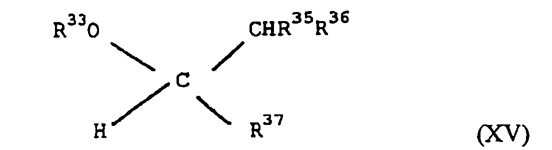 ou par la formule générale (XVI) : 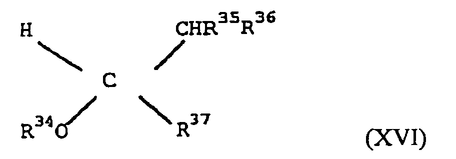 dans lesquelles R³³ et R³⁴ sont chacun un groupe hydrocarboné ou un groupe hydrocarboné contenant des atomes d'oxygène en position éther dans la chaîne principale, dans la chaîne latérale ou dans les deux d'entre elles, et peuvent être identiques ou différents l'un de l'autre et R³⁵, R³⁶ et R³⁷ sont chacun un atome d'hydrogène, un groupe hydrocarboné ou un groupe hydrocarboné contenant des atomes d'oxygène en position éther dans la chaîne principale, dans la chaîne latérale ou dans les deux d'entre elles, et peuvent être identiques ou différents les uns des autres,
qui comprend l'étape consistant à mettre à réagir un composé acétal ou un composé cétal exprimé par la formule générale (XIV) : 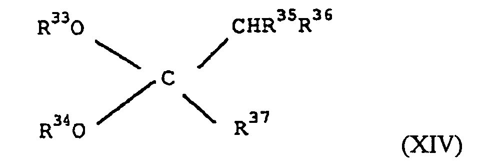 dans laquelle R³³, R³⁴, R³⁵, R³⁶ et R³⁷ sont les mêmes que ceux des formules (XV) et (XVI),
avec l'hydrogène en présence d'un catalyseur solide ayant des caractéristiques acides et une capacité d'hydrogénation, dans lequel le catalyseur solide ayant des caractéristiques acides et une capacité d'hydrogénation est un catalyseur comprenant une combinaison d'un catalyseur d'hydrogénation et d'un catalyseur acide solide ou un catalyseur acide solide ayant une capacité d'hydrogénation,
dans lequel le catalyseur solide ayant des caractéristiques acides et une capacité d'hydrogénation est
(A) un catalyseur comprenant une combinaison d'un catalyseur d'hydrogénation et d'un catalyseur acide solide,
dans lequel le catalyseur d'hydrogénation est choisi dans le groupe consistant en
(1) nickel, palladium, rhodium et ruthénium seul ou comme composant principal,
(2) des catalyseurs ayant le composé métallique catalytique de (1) supporté sur des supports tels que charbon activé, alumine et terre à diatomées ou
(3) des catalyseurs de Raney tels que nickel de Raney et cobalt de Raney,
et le catalyseur solide est choisi dans le groupe consistant en argile activée, argile acide, différents types de zéolithes, résines échangeuses d'ions, silice-alumine et hétéropolyacides ;
ou (B) un catalyseur acide solide ayant une capacité d'hydrogénation choisi dans le groupe consistant en nickel, palladium, platine, et ruthénium supporté sur différents types de zéolithes.

6. Procédé de production d'un composé d'éther tel que revendiqué à la revendication 5, dans lequel le composé acétal ou le composé cétal exprimé par la formule générale (XIV) est un composé exprimé par la formule générale (XVII) : 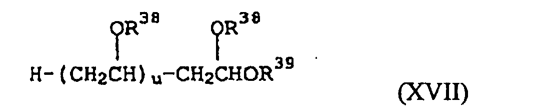 dans laquelle R³⁸ et R³⁹ sont chacun un groupe hydrocarboné ayant 1 à 20 atomes de carbone ou un groupe hydrocarboné contenant des atomes d'oxygène en position éther, et ils peuvent être identiques ou différents l'un de l'autre, les groupes R³⁸ peuvent être identiques ou différents entre les motifs de constitution et u est un entier de 1 à 500, et le composé d'éther ainsi obtenu est un composé exprimé par la formule générale (XVIII) 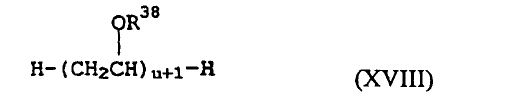 ou par la formule générale (XIX) 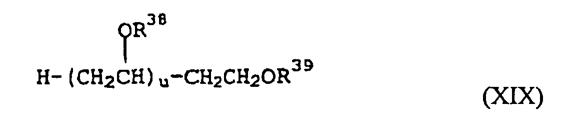 dans lesquelles R³⁸, R³⁹ et u sont chacun les mêmes que ceux de la formule (XVII).

7. Procédé de production d'un composé d'éther tel que revendiqué à la revendication 5, dans lequel le composé acétal ou le composé exprimé par la formule générale (XIV) est un composé exprimé par la formule générale (XX) :
R⁴⁰CH(OR⁴¹)₂ (XX)
dans laquelle R⁴⁰ et R⁴¹ sont chacun un groupe hydrocarboné ayant 1 à 20 atomes de carbone, et peuvent être identiques ou différents l'un de l'autre, et le composé d'éther ainsi obtenu est un composé exprimé par la formule générale (XXI) :
R⁴⁰CH₂OR⁴¹ (XXI)
dans laquelle R⁴⁰ et R⁴¹ sont les mêmes que ceux de la formule (XX).
